# EUROPEAN PATENT SPECIFICATION

(11) **EP 0 611 376 B1**
(45) Date of publication and mention of the grant of the patent: **17.06.1998**
(21) Application number: 92925173.4
(22) Date of filing: 06.11.1992
(51) Int. Cl.: C07K 14/00

(54) **PRODUCTION AND USE OF MULTIMERIC HEMOGLOBINS**
VERFAHREN ZUR HERSTELLUNG UND ANWENDUNG VON MULTIMEREN HÄMOBLOBINEN
PRODUCTION ET UTILISATION D'HEMOGLOBINES MULTIMERES

(30) Priority: 08.11.1991 US 789179
(43) Date of publication of application: 24.08.1994
(60) Divisional application: 97203723.8
(73) Proprietor: Somatogen Inc., Boulder, Colorado 80301 (US)
(72) Inventor: ANDERSON, David, C., Lafayette, CO 80026 (US); MATHEWS, Anthony, James, Louisville, CO 80027 (US); STETLER, Gary, L., Boulder, CO 80302 (US)
(74) Representative: Plougmann, Vingtoft & Partners A/S
(86) International application number: PCT/US92/09752
(87) International publication number: WO 93/09143

(56) References cited:
- EP-A- 0 290 252
- EP-A- 0 402 300
- WO-A-88/09179
- WO-A-91/16349
- WO-A-92/11283
- CHEMICAL ABSTRACTS, vol. 116, no. 9, 1991, Columbus, Ohio, US; abstract no. 78773n,
- CHEMICAL ABSTRACTS, vol. 108, no. 9, 1987, Columbus, Ohio, US; abstract no. 70799q,
- CHEMICAL ABSTRACTS, vol. 79, no. 21 Columbus, Ohio, US; abstract no. 122753z,

## Description

### Cross-Reference to Related Applications

Hoffman and Nagai, U.S. Patent No. 5,028,588, presently owned by Somatogen, Inc., relates to the use of low oxygen affinity and other mutant hemoglobins as blood substitutes, and to the expression of alpha and beta globin in nonerythroid cells. Hoffman and Nagai, U.S. Patent No. 5,449,759 discloses certain additional dicysteine hemoglobin mutants; it is a continuation-in-part of the application leading to U.S. Patent No. 5,028,588.

### BACKGROUND OF THE INVENTION

### Field of the Invention

The present invention is directed to multimeric hemoglobin-like proteins composed of two or more pseudotetramers linked together either by genetic fusion or by chemical crosslinking.

### Description of the Background Art

### A. Structure and Function of Hemoglobin

Hemoglobin (Hgb) is the oxygen-carrying component of blood. Hemoglobin circulates through the bloodstream inside small enucleate cells called erythrocytes (red blood cells). Hemoglobin is a protein constructed from four associated polypeptide chains, and bearing prosthetic groups known as hemes. The erythrocyte helps maintain hemoglobin in its reduced, functional form. The heme iron atom is susceptible to oxidation, but may be reduced again by one of two enzyme systems within the erythrocyte, the cytochrome b₅ and glutathione reduction systems.

The structure of hemoglobin is well known. We herewith incorporate by reference the entire text of Bunn and Forget, eds., Hemoglobin: Molecular, Genetic and Clinical Aspects (W.B. Saunders Co., Philadelphia, PA: 1986) and of Fermi and Perutz "Hemoglobin and Myoglobin," in Phillips and Richards, Atlas of Molecular Structures in Biology (Clarendon Press: 1981).

About 92% of the normal adult human hemolysate is Hgb A (designated alpha2 beta2, because it comprises two alpha and two beta chains). Other recognized hemoglobin species are Hgb A₂ (α₂ δ₂), Hgb A₁ₐ, Hgb A_{1b}, and Hgb A_{1c}, as well as the rare species Hgb F (α₂ gamma₂), Hgb Gower-1 (Zeta₂ epsilon₂), Hgb Gower-2 (alpha₂ epsilon₂), Hgb Portland (Zeta₂ gamma₂), and Hgb H (beta₄) and Hgb Bart (gamma₄). They are distinguished from Hgb A by a different selection of polypeptide chains.

The primary structure of a polypeptide is defined by its amino acid sequence and by identification of any modifications of the side chains of the individual amino acids. The amino acid sequences of both the alpha and beta globin polypeptide chains of "normal" human hemoglobin is given in Table 1. Many mutant forms are also known; several mutants are identified in Table 400. The wild-type alpha chain consists of 141 amino acids. The iron atom of the heme (ferroprotoporphyrin IX) group is bound covalently to the imidazole of His 87 (the "proximal histidine"). The wild-type beta chain is 146 residues long and heme is bound to it at His 92. Apohemoglobin is the heme-free analogue of hemoglobin; it exists predominantly as the αβ-globin dimer.

Segments of polypeptide chains may be stabilized by folding into one of two common conformations, the alpha helix and the beta pleated sheet. In its native state, about 75% of the hemoglobin molecule is alpha-helical. Alpha-helical segments are separated by segments wherein the chain is less constrained. It is conventional to identify the alpha-helical segments of each chain by letters, e.g., the proximal histidine of the alpha chain is F8 (residue 8 of helix F). The non- helical segments are identified by letter pairs, indicating which helical segments they connect. Thus, nonhelical segment BC lies between helix B and helix C. In comparing two variants of a particular hemoglobin chain, it may be enlightening to attempt to align the helical segments when seeking to find structural homologies. For the amino acid sequence and helical residue notation for normal human hemoglobin Aₒ alpha and beta chains, see Bunn and Forget, supra, and Table 1 herein.

The tertiary structure of the hemoglobin molecule refers to the steric relationships of amino acid residues that are far apart in the linear sequence, while quaternary structure refers to the way in which the subunits (chains) are packed together. The tertiary and quaternary structure of the hemoglobin molecule have been discerned by X-ray diffraction analysis of hemoglobin crystals, which allows one to calculate the three-dimensional positions of the very atoms of the molecule.

In its unoxygenated ("deoxy", or "T" for "tense") form, the subunits of hemoglobin A (alpha1, alpha2, beta1, and beta2) form a tetrahedron having a twofold axis of symmetry. The axis runs down a water-filled "central cavity". The subunits interact with one another by means of Van der Waals forces, hydrogen bonds and by ionic interactions (or "salt bridges"). The alphalbetal and alpha2beta2 interfaces remain relatively fixed during oxygenation. In contrast, there is considerable flux at the alphalbeta2 (and alpha2beta1) interface. In its oxygenated ("oxy", or "R" for "relaxed" form), the intersubunit distances are increased.

The tertiary and quaternary structures of native oxyhemoglobin and deoxyhemoglobin are sufficiently well known that almost all of the nonhydrogen atoms can be positioned with an accuracy of 0.5 Å or better. For human deoxyhemoglobin, see Fermi, et al., J. Mol. Biol., 175: 159 (1984), and for human oxyhemoglobin, see Shaanan, J. Mol. Biol., 171: 31 (1983).

Normal hemoglobin has cysteines at beta 93 (F9), beta 112 (G14), and alpha 104 (G11). The latter two positions are deeply buried in both the oxy and deoxy states; they lie near the α₁β₁ interface. Beta 93, however, in the oxy form is reactive with sulfhydryl reagents.

Native human hemoglobin has been fully reconstituted from separated heme-free alpha and beta globin and from hemin. Preferably, heme is first added to the alpha-globin subunit. The heme-bound alpha globin is then complexed to the heme-free beta subunit. Finally, heme is added to the half-filled globin dimer, and tetrameric hemoglobin is obtained. Yip, et al., PNAS (USA), 74: 64-68 (1977).

The human alpha and beta globin genes reside on chromosomes 16 and 11, respectively. Bunn and Forget, infra at 172. Both genes have been cloned and sequenced, Liebhaber, et al., PNAS 77: 7054-58 (1980) (alpha-globin genomic DNA); Marotta, et al., J. Biol. Chem., 252: 5040-53 (1977) (beta globin cDNA); Lawn, et al., Cell, 21:647 (1980) (beta globin genomic DNA).

Hemoglobin exhibits cooperative binding of oxygen by the four subunits of the hemoglobin molecule (two alpha-globins and two beta-globins in the case of Hgb A), and this cooperativity greatly facilitates efficient oxygen transport. Cooperativity, achieved by the so-called hemeheme interaction, allows hemoglobin to vary its affinity for oxygen. Hemoglobin reversibly binds up to four moles of oxygen per mole of Hgb.

Oxygen-carrying compounds are frequently compared by means of a device known as an oxygen dissociation curve. This curve is obtained when, for a given oxygen carrier, oxygen saturation or content is graphed against the partial pressure of oxygen. For Hgb, the percentage of saturation increases with partial pressure according to a sigmoid relationship. The P₅₀ is the partial pressure at which the oxygen-carrying solution is half saturated with oxygen. It is thus a measure of oxygen-binding affinity; the higher the P₅₀, the more loosely the oxygen is held.

When the oxygen dissociation curve of an oxygen-carrying solution is such that the P₅₀ is less than that for whole blood, it is said to be "left-shifted."

The oxygen affinity of hemoglobin is lowered by the presence of 2,3-diphosphoglycerate (2,3-DPG), chloride ions and hydrogen ions. Respiring tissue releases carbon dioxide into the blood and lowers its pH (i.e. increases the hydrogen ion concentration), thereby causing oxygen to dissociate from hemoglobin and allowing it to diffuse into individual cells.

The ability of hemoglobin to alter its oxygen affinity, increasing the efficiency of oxygen transport around the body, is dependent on the presence of the metabolite 2,3- DPG. Inside the erythrocyte 2,3-DPG is present at a concentration nearly as great as that of hemoglobin itself. In the absence of 2,3-DPG "conventional" hemoglobin binds oxygen very tightly and would release little oxygen to respiring tissue.

Aging erythrocytes release small amounts of free hemoglobin into the blood plasma where it is rapidly bound by the scavenging protein haptoglobin. The hemoglobinhaptoglobin complex is removed from the blood and degraded by the spleen and liver.

Isolated alpha globin chains are monomers; exhibit high oxygen affinity but of course lack subunit cooperativity. Isolated beta globin chains aggregate to form a β₄ tetramer (HbH). The β₄ tetramer has a high but noncooperative oxygen affinity.

### B. Blood Substitutes, Generally

It is not always practical to transfuse a patient with donated blood. In these situations, use of a red blood cell substitute is desirable. The product must effectively transport O₂, just as do red blood cells. ("Plasma expanders", such as dextran and albumin, do not transport oxygen.) The two types of substitutes that have been studied most extensively are hemoglobin solutions and fluorocarbon emulsions.

It is clear from the above considerations that free native hemoglobin A, injected directly into the bloodstream, would not support efficient oxygen transport about the body. The essential allosteric regulator 2,3-DPG is not present in sufficient concentration in the plasma to allow hemoglobin to release much oxygen at venous oxygen tension.

Nonetheless, solutions of conventional hemoglobin have been used as RBC substitutes. The classic method of preparing hemoglobin solutions employs outdated blood. The red cells are lysed and cellular debris is removed, leaving what is hopefully "stromal-free hemoglobin" (SFH).

Several basic problems have been observed with this approach. The solution must be freed of any toxic components of the red cell membrane without resorting to cumbersome and tedious procedures which would discourage large-scale production. DeVenuto, "Appraisal of Hemoglobin Solution as a Blood Substitute", Surgery, Gynecology and Obstetrics, 149: 417-436 (1979).

Second, as expected, such solutions are "left-shifted" (lower P₅₀) as compared to whole blood. Gould, et al., "The Development of Polymerized Pyridoxylated Hemoglobin Solution as a Red Cell Substitute", Ann. Emerg. Med. 15: 1416- 1419 (Dec. 3, 1986). As a result, the oxygen affinity is too high to unload enough oxygen into the tissues. Benesch and Benesch, Biochem. Biophys. Res. Comm., 26:162-167 (1967).

Third, SFH has only a limited half-life in the circulatory system. This is because oxy Hgb partially dissociates into a dimer (αβ) that is rapidly cleared from the blood by glomerular filtration and binding to circulating haptoglobulin. If large amounts of soluble hemoglobin are introduced into the circulation, glomerular filtration of the dimers may lead to a protein and iron load on the kidney capable of causing renal damage. Bunn, H.F., et al. (1969) The renal handling of hemoglobin I. Glomerular filtration. J. Exp. Med. 129:909-923; Bunn, H.F., and J.H. Jandl; (1969) The renal handling of hemoglobin II. Catabolism. J. Exp. Med. 129:925-934; Lee, R.L., et al. (1989) Ultrapure, stroma-free, polymerized bovine hemoglobin solution: Evaluation of renal toxicity (blood substitutes) J. Surgical Res. 47:407-411; Feola, M., et al. (1990) Nephrotoxicity of hemoglobin solutions. Biomat. Art. Cell Art. Org., 18(2):233-249; Tam, S.C. and J. T.F. Wong (1988) Impairment of renal function by stroma-free hemoglobin in rats. J. Lab. Clin. Med. 111:189-193.

Finally, SFH has a high colloid osmotic pressure (COD). Thus, administration of SFH in a dose that would have the same oxygen-carrying capacity as a unit of packed red blood cells is inadvisable, since the high osmotic pressure (60mm Hg) would cause a massive influx of water from the cells into the bloodstream, thus dehydrating the patient's tissues. This consideration limits the dose of SFH to that which provide a final concentration of about 6-8 gm Hgb/dl.

In an effort to restore the desired P₅₀, researchers added 2,3-DPG to the hemoglobin solution. Unfortunately, 2,3- DPG was rapidly eliminated from the circulation. Scientists then turned to other organic phosphates, particularly pyridoxal phosphate. Like 2,3-DPG, these compounds stabilized the "T state" of the Hgb by forming a salt bridge between the N- termini of the two beta chains. The pyridoxylated hemoglobin had a P₅₀ of 20-22 torr, as compared to 10 torr for SFH and 28 torr for whole blood. While this is an improvement over SFH, the pyridoxylated Hgb remains "high affinity" relative to whole blood.

### C. Naturally Occurring Cysteine Substitution Mutants of Hemoglobin (Non-Polymerizing)

There are a few known naturally occurring mutants of human hemoglobin in which a cysteine residue is substituted for another residue of normal hemoglobin Ao.

In hemoglobin Nigeria, the mutation is α 81 Ser → Cys; no disulfide is formed. Haris, et al., Blood, 55(1):131-137 (1980). In Hemoglobin Rainier, an intrasubunit disulfide is formed between the wild type F9(93)β Cysteine and the cysteine introduced by replacement of the Tyr at HC2(145)β. Greer, et al., Nature [New Biology], 230:261-264 (1971). Hemoglobin Nunobiki (β 141 Drg → Cys) also features a non-polymerizing cysteine substitution. In both Hb Rainier and Hb Nunobiki, the new cysteine residues are on the surface of the tetramer.

### D. Naturally Occurring Polymerizing or Polymeric Hemoglobins

Three other human mutants are known which polymerize as a result of formation of intermolecular (first tetramer to second tetramer) disulfide bridges. In Hemoglobin Porto Alegre, the Ser at A6(9)β is replaced by Cysteine, and since this cysteinyl residue is externally oriented, spontaneous polymerization occurs, and results in formation of a dodecamer with three Porto Alegre tetramers linked by disulfide bonds. An octamer has also been made by a 1:1 mixture of Porto Alegre hemoglobin and normal hemoglobin. Tondo, Biochem. Biophys. Acta, 342:15-20 (1974); Tondo, An. Acad. Bras. Cr., 59:243-251 (1987).

Hb Mississippi is characterized by a cysteine substitution in place of Ser CD3(44)β. Hemolysates of a patient were subjected to gel filtration column chromatography, and 48.8% eluted in the void volume. Since the molecular weight exclusion was about 600kD, this suggested that Hb MS polymers are composed of ten or more hemoglobin tetramers. Adams, et al., Hemoglobin, 11(5):435-452 (1987).

A β83(EF7)Gly→Cys mutation characterizes Hemoglobin Ta Li. This mutant showed slow mobility in starch gel electrophoresis, indicating that it was a polymer.

Polymeric mouse hemoglobins have been reported. In BALB/cJ mice, there is a reactive cysteinyl residue near the NH₂-terminal of the beta chain (β-13 in the mouse). This mouse mutant has been compared to Hemoglobin Porto Alegre, which likewise has a cysteinyl residue in the A-helix of the beta chain. Octamer formation is most common. However, each tetramer has two extra cysteinyl residues, one on each β-chain, that may react with different tetramers; "this explains why aggregates larger than octamers occur". Benaventura and Riggs, Science, 149:800-802 (1967); Riggs, Science, 147:621-623 (1965).

Macaques also exhibit a polymerizing hemoglobin variant. Takenaka, et al., Biopchem. Biophys. Acta, 492:433-444 (1977); Ishimoto, et al., J. Anthrop. Soc. Nippon, 83(3):233-243 (1975). This mutant has been compared to the Ta Li variant in humans.

Both amphibians and reptiles possess polymerizing hemoglobins. For example, in the bullfrog, hemoglobin "Component C" polymerizes by disulfide bond formation between tetramers. This is said to be primarily dependent on cysteinyl residues of the alpha chain. Tam, et al., J. Biol. Chem., 261:8290-94 (1986).

The extracellular hemoglobin of the earthworm (Lumbricus terrestris) has a complex structure. There are twelve subunits, each being a dimer of structure (abcd)₂ where "a", "b", "c", and "d" denote the major heme containing chains. The "a", "b", and "c" chains form a disulfide-linked trimer. The whole molecule is composed of 192 heme-containing chains and 12 non-heme chains, and has a molecular weight of 3800 kDa. Other invertebrate hemoglobins are also large multi-subunit proteins.

The brine shrimp Artemia produces three polymeric hemoglobins with nine genetically fused globin subunits. Manning, et al., Nature, 348:653 (1990). These are formed by variable association of two different subunit types, a and bβ. Of the eight intersubunit linkers, six are 12 residues long, one is 11 residues and one is 14 residues.

### E. Artificially Crosslinked Hemoglobins (Non-Polymerizing)

The properties of hemoglobin have been altered by specifically chemically crosslinking the alpha chains between the Lys99 of alphal and the Lys99 of alpha2. Walder, U.S. 4,600,531 and 4,598,064; Snyder, et al., PNAS (USA) 84: 7280-84 (1987); Chaterjee, et al., J. Biol. Chem., 261: 9927-37 (1986). The beta chains have also been chemically crosslinked. Kavanaugh, et al., Biochemistry, 27: 1804-8( 1988). Kavanaugh notes that the beta N-termini are 16 Å apart in the T state and 20 Å apart in the R state. Not surprisingly, the introduction of a DIDS bridge between the N- termini of T state hemoglobin hindered the shift to the R state, thereby decreasing the O₂ affinity of the molecule. While the Kavanaugh analogue has desirable oxygen binding and renal clearance characteristics, it too is obtained in low yield.

Hoffman and Nagai, USP 5,028,588 suggest that the T state of hemoglobin may be stabilized by intersubunit (but intratetrameric) disulfide crosslinks resulting from substitution of cysteine residues for other residues. A particularly preferred crosslink was one connecting beta Gly Cys with either alpha G17 (Ala→Cys) or G18 (Ala→Cys).

### F. Artificially Crosslinked Hemoglobin (Polymerizing)

Bonsen, USP 4,001,401, USP 4,001,200, and USP 4,053,590 all relate to polymerization of red blood cell-derived hemoglobin by chemical crosslinking. The crosslinking is achieved with the aid of bifunctional or polyfunctional crosslinking agents, especially those reactive with exposed amino groups of the globin chains. The result of the crosslinking reaction is a polydisperse composition of covalently cross-linked aggregates.

Bonhard, USP 4,336,248 discloses chemical crosslinking of hemoglobin molecules to each other, or to serum proteins such as albumin.

Bonhard, USP 4,777,244 sought to stabilize the dialdehyde-cross-linked hemoglobins of the prior art, which tended to polymerized further while in storage, by adding a reducing agent to stabilize the azomethine bond.

Bucci, USP 4,584,130, at col. 2, comments that "the polyhemoglobin reaction products are a heterogeneous mixture of various molecular species which differ in size and shape. The molecular weights thereof range from 64,500 to 600,000 Daltons. The separation of individual molecular species from the heterogeneous mixture is virtually impossible. In addition, although longer retention times in vivo are obtained using polyhemoglobins, the oxygen affinity thereof is higher than that of stroma-free hemoglobin."

According to Tye, USP 4,529,179, "most workers have chosen to form the random intermolecular crosslinked polymers of hemoglobin because they believed that the 65,000 Dalton tetramer was filtered by the glomerulus.... Usually the amino groups of lysine on the surface of the hemoglobin molecule are coupled with a bifunctional reactant such as gluteraldehyde or suberimidate. There are 42 lysines available for reaction per hemoglobin tetramer so that one can get an infinite number of different inter [or] intra molecular crosslinks making various polymers of hemoglobin.... The random polymerization is difficult to control and gives a range between two and ten tetramers per polymer.... No one has yet standardized an analytical scheme to establish lot to lot variability of structure and function.... [Polymerized pyridoxylated hemoglobin has] a profound chemical heterogeneity making it difficult to study as a pharmaceutical agent."

### G. Fused Genes and Proteins, Generally

Genes may be fused together by removing the stop codon of the first gene, and joining it in phase to the second gene. Parts of genes may also be fused, and spacer DNAs which maintain phase may be interposed between the fused sequences. The product of a fused gene is a single polypeptide, not a plurality of polypeptides as is expressed by a polycistronic operon. Different genes have been fused together for a variety of purposes. Thus, Gilbert, U.S. 4,338,397 inserted a rat preproinsulin gene behind a fragment of the E. coli penicillinase gene. His purpose was to direct E. coli transformants to secrete the expression product of the fused gene. Fused genes have also been prepared so that a non- antigenic polypeptide may be expressed already conjugated to an immunogenic carrier protein.

The use of linker DNA sequences to join two different DNA sequences is known. These linkers are used to provide restriction sites for DNA cleavage, or to encode peptides having a unique character that facilitates purification of the encoded fusion protein or a fragment thereof. See, e.g., Rutter, U.S. 4,769,326.

Hallewell, et al., J. Biol. Chem., 264: 5260-68 (1989) prepared an analogue of CuZn superoxide dismutase. Each dismutase molecule is a dimer of two identical subunits; a copper ion and a zinc ion are liganded to the subunit. The dimer interaction in CuZn superoxide dismutase is so strong that the subunits have not been separated without inactivating the enzyme. The enzyme has considerable conformational similarity to immunoglobulins; Hallewell, et al., joined two human superoxide dismutase genes, either directly or with DNA encoding a 19-residue human immunologlobulin IgA1 hinge region and expressed the fused genes in a transformed host. In attempting to express the directly joined genes, recombination occurred to eliminate one of the tandem genes in some plasmid molecules. Hallewell, et al., postulated that the direct connection distorted the dimer, causing the exposure of hydrophobic areas which then had a toxic effect. This would have provided selection pressure favoring gene deletion. No recombination was detected with the IgA1 linker construction.

Hoffman, et al., WO88/09179 describe the production, in bacteria and yeast, of hemoglobin and analogues thereof. The disclosed analogues including hemoglobin proteins in which one of the component polypeptide chains consists of two alpha or two beta globin amino acid sequences covalently connected by peptide bonds, preferably through an intermediate linker of one or more amino acids, without branching. In normal hemoglobin, the alpha and beta globin subunits are non-covalently bound.

### SUMMARY OF THE INVENTION

The present invention relates to multimeric hemoglobin-like proteins wherein two or more tetramers or pseudotetramers are covalently bonded. Between any pair of covalently linked tetramers, the covalent linkage may take the form of a crosslink between two cysteine residues of different polypeptide chains, or of a peptide linker connecting the "carboxy most" residue of a globin-like domain of one tetramer with the "amino most" residue of a similar domain of a second tetramer.

Preferably, the multimeric hemoglobin-like protein-containing composition is at least 50% monodisperse, more preferably, at least 95% monodisperse.

Although free hemoglobin purified from natural sources may be polymerized by chemical crosslinking to increase halflife via increased molecular weight, and to reduce oncotic pressure, all such preparations are heterogeneous. Monodispersability can be achieved only by laborious purification.

The present invention provides means of exerting strict control over the degree of polymerization of hemoglobin tetramers. The ability to strictly control formation of multimers will greatly facilitate purification and characterization of the final product and will reduce the chance of adverse reaction to minor components. It is also believed that a more monodisperse composition will have greater consistency of clinical effect.

Hemoglobin also may be made by expression of alpha and beta globin genes in the same or different host cells, and subsequent assembly of the expressed alpha and beta globins, with heme, to form hemoglobin. While the introduction of suitable Cys codon mutations into the globin genes facilitates the production of a crosslinked multimeric hemoglobin, the expression product in general, will not be essentially monodisperse. Hemoglobin is composed of two alpha and two beta globin subunits. Both alpha globin subunits are natively expressed from a single alpha globin gene, and both beta globin subunits, from a single beta globin gene. Thus, if an alpha globin gene is expressed which contains a single Cys codon substitution, the assembled tetramer will contain two alpha globin subunits, each with a crosslinkable Cys. One Cys could crosslink to a second tetramer, and the other to a third, thus resulting in formation of a higher order oligomer.

In one embodiment, the multimeric protein is an octamer consisting essentially of two tetramers which are covalently crosslinked. To avoid unwanted polymerization, each tetramer has only a single participating cysteinyl residue, whose thiol groups are reacted either with each other (under oxidizing conditions, forming a disulfide bond) or with a thiol-reactive crosslinking agent, to form the crosslink.

A fused gene which encodes a single polypeptide comprising two globin-like domains may be mutated so as to provide an externally crosslinkable Cys in only one of the two otherwise substantially identical domains of the resulting pseudodimeric polypeptides. This pseudodimer may then be assembled with the complementary subunits to form a tetramer with only the single cysteine. Two such tetramers, finally, may be crosslinked to obtain the octamer, preferably in essentially monodisperse form.

If the formation of a higher order multimer, such as a dodecamer, is desired, the component pseudotetramers, each having a single externally crosslinkable cysteine, are each covalently attached to a reactive site of a polyfunctional crosslinker having a suitable half-life in the bloodstream.

The multimeric proteins of the present invention, particularly at higher levels of polymerization, may prolong the half-life of recombinant hemoglobin by reducing extravasation and glomerular filtration of dissociated subunits in vivo compared to native human hemoglobin. Studies of halflife as a function of macromolecular size indicate a correlation between increased size and increased circulatory halflife for chemically crosslinked Hb as well as other macromolecules. Preferably, in humans, the half-life exceeds 9 hours at a dose of at least 1 gm/kgm body weight. This would be expected to correspond to a half-life of about 3 hours in rats given a comparable dose.

Intravascular retention may also be enhanced by engineering the tetramer crosslinking sites so that the haptoglobin binding sites of the tetramers are wholly or partially occluded. Independent mutations may also be made to sterically hinder haptoglobin binding, or to electrostatically repel or sterically hinder the approach of agents which otherwise might degrade the crosslink.

The multimeric proteins of the present invention may also increase oncotic pressure because the number of oxygen binding heme groups per polytetramer of order "n" is "n" times the number per tetramer. Independent of size, the oncotic pressure for a given concentration of heme groups in a solution of polytetrameric Hb is expected to be (1/n) times that of an equimolar solution of heme contained in tetrameric Hb. Because of oncotic pressure effects, the maximum concentration of free tetrameric Hb that may be introduced into the blood stream is less on a per volume basis than the concentration of Hb normally carried in intact red blood cells. Reduction of oncotic pressure is therefore useful in increasing the per volume oxygen carrying capacity of a blood substitute.

In a preferred embodiment, one or more globin-like domains contain mutations which reduce the oxygen-binding affinity of the hemoglobin analogue in solution so as to approach the oxygen-binding characteristics of whole blood.

### BRIEF DESCRIPTION OF THE DRAWINGS

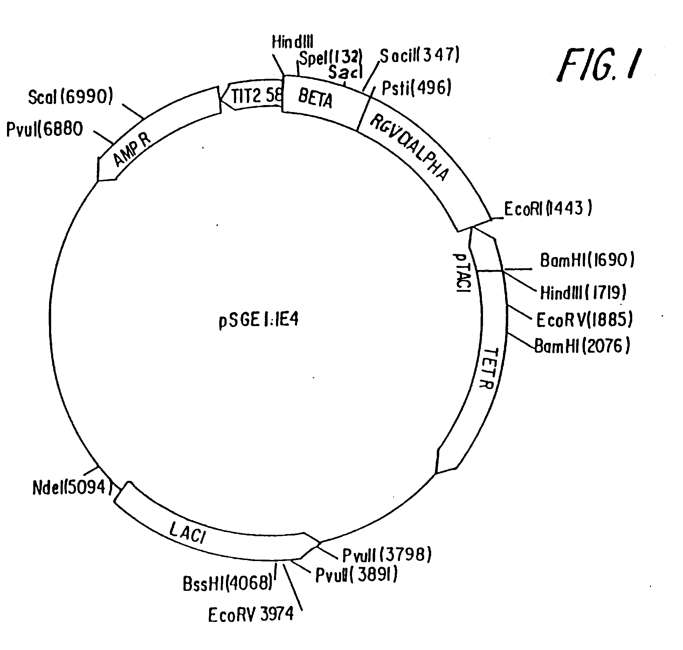

Figure 1 Plasmid pSGE1.1E4. This plasmid bears a polycistronic operon which comprises the pTAC promoter and genes encoding a di-alpha globin and a beta globin. It also carries tetracycline and ampicillin resistance genes, and the lacI gene.

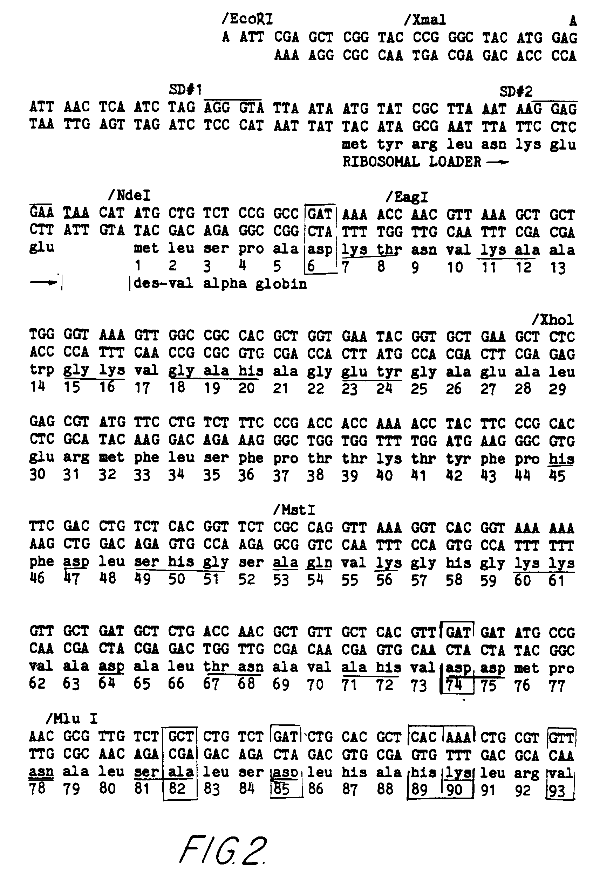

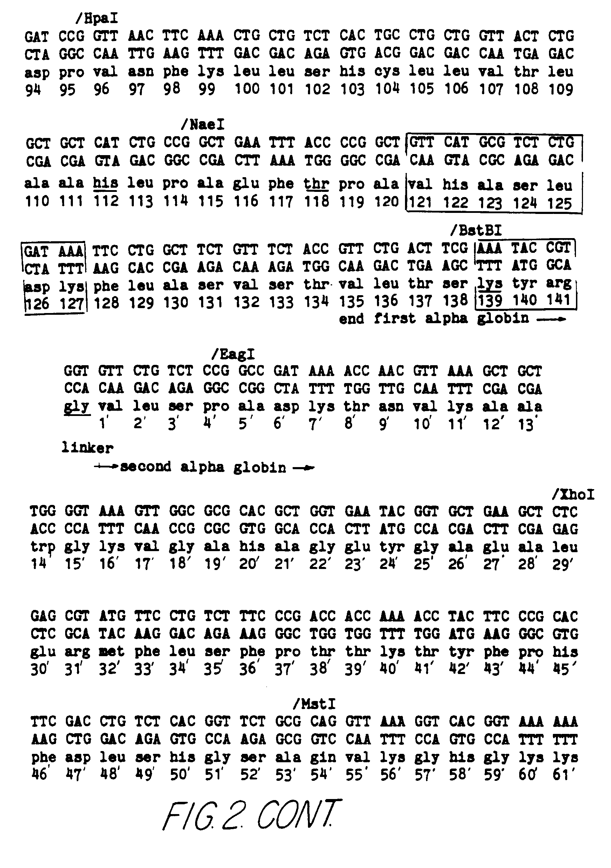

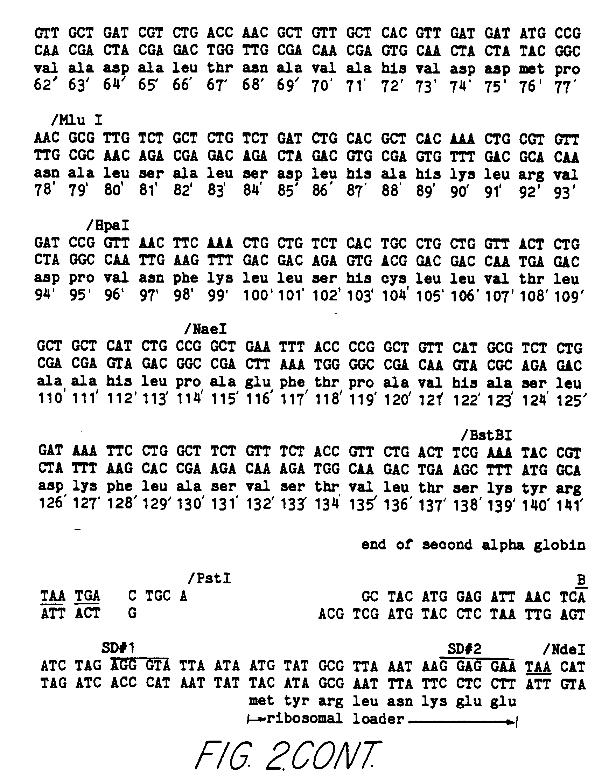

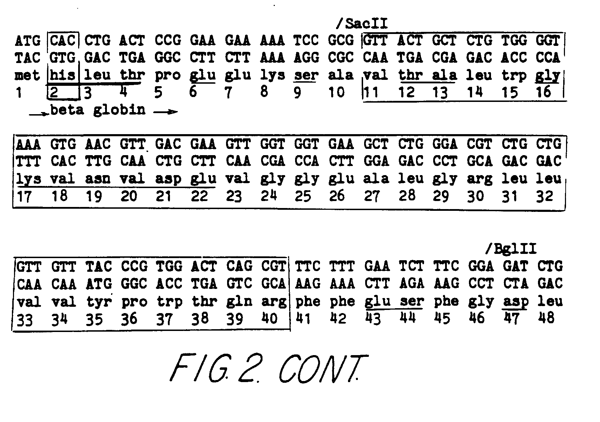

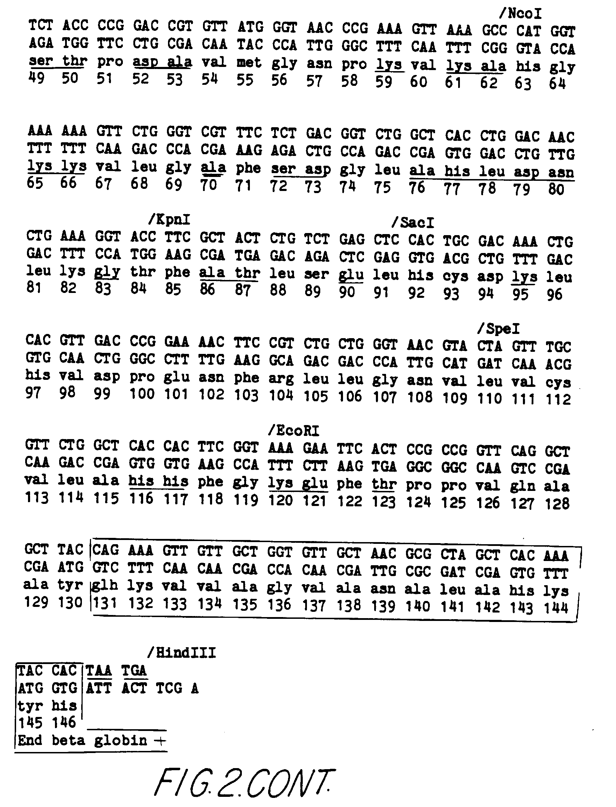

Figure 2 Shows the sequence **[SEQ ID NO:1]** of a preferred synthetic gene for expression of (des-Val)-alpha-(Gly)- alpha and des-Val beta globin. This gene is carried by pSGE1.1E4. A shows the region (EcoRI to PstI) containing Shine-Delgarno ribosomal binding sites (SD#1 and SD#2), the sequence expressing the octapeptide (Met...Glu) (SEQ ID NO:25) which serves as a cotranslational coupler, and the sequence encoding the two nearly identical alpha globin-like polypeptides and the interposed Gly-Gly linker. The first alpha globin sequence begins "Met-Leu", that is, it contains an artifactual methionine, omits the valine which is the normal first residue of mature alpha globin, and continues with the second residue, leucine. The residues are numbered 1 to 141 (SEQ ID NO:26). The second alpha globin sequence begins "Val-Leu", immediately after the underlined "Gly-Gly" linker. The residues are numbered 1' to 141' (SEQ ID NO:27). Start and stop codons are underlined. B shows the analogous region (PstI to HindIII) containing the coding sequence for des-Val beta globin. The beta residues are numbered 1 to 146 (SEQ ID NO:28). A and B are connected at the PstI site to form a single polycistronic operon.

When a three letter amino acid code is singly underlined, this indicates that the residue is a potential site for an Xaa → Cys mutation to provide a crosslinkable site. The mutations should be made asymmetrically, i.e., only one region of a di-alpha or di-beta gene, so only one crosslink is added per tetramer. While, in Figure 2, the sites are marked only on the first copy of the alpha gene, they could instead be in the second copy. For convenience, the appropriate beta globin mutation sites are also marked. However, these mutations should be made in only one beta-globin of a di-beta globin gene.

Doubly underlined amino acid codes identify sites where formation of two disulfide bonds (or per subunit) would be sterically hindered, so use of a di-alpha or di-beta construction is unnecessary.

Residues which are candidate sites for mutations to block haptoglobin binding are boxed.
Figure 3 is a stylized representation of one form of pseudooctameric Hgb, in which the octameric hemoglobin is formed by linking or crosslinking two molecules of an asymmetric di-alpha Hgb.
Figure 4 depicts coiled coil crosslinkers suitable for joining (a) four or (b) six Hgb tetramers. Fig. 4(c) is a top view of a 4-helical bundle, with attachment sites marked.
Figure 5 Proposed alpha₁-beta₂ globin pseudodimer.

### DETAILED DESCRIPTION OF THE PREFERRED EMBODIMENTS

### Definitions

A hemoglobin is a protein which contains heme (ferroprotoporphyrin IX) and that binds oxygen at a respiratory surface (skin, gills, trachea, lung, etc.) and transports the oxygen to inner tissues, where it is released and used for metabolism. In nature, low molecular weight hemoglobins (16-120 kilodaltons) tend to be enclosed in circulating red blood cells while the larger polymeric hemoglobins circulate freely in the blood of hemolymph.

For the purpose of the appended claims, a hemoglobin-like protein is a protein with the following characteristics:
(a) it is sufficiently soluble in blood to be clinically useful as a blood substitute;
(b) it reversibly binds oxygen, under physiological conditions;
(c) each polypeptide chain comprises at least one globin-like domain (as defined below); and
(d) each globin-like domain bears (or is capable of incorporating) a heme prosthetic group;

A multimeric hemoglobin-like protein is further characterized as follows:
(e) it is composed of two or more polypeptide chains;
(f) it is composed of two or more tetramers, each tetramer comprising four globin-like domains, and
(g) each component tetramer is covalently attached to at least one other component tetramer.

Preferably, the hemoglobin-like proteins of the present invention have a P₅₀ of 2 to 45 torr, more preferably 24 to 32 torr, at 37°C, in blood. Preferably, they also exhibit some degree of cooperativity. Also, they desirably have an intravascular retention at least comparable to that of normal human hemoglobin administered as a blood substitute.

Tetrameric hemoglobin-like proteins have four globin-like domains, octameric hemoglobin-like proteins have eight globin-like domains, and so forth. The term "multimeric" covers any hemoglobin-like protein comprising (4 x n) globin-like domains, where n>1.

A pseudomeric hemoglobin-like protein is one for which the number of globin-like domains is greater than the number of component polypeptide chains, i.e., at least one chain comprises at least two globin-like domains. The pseudoheterotetrameric hemoglobin-like proteins, for example, may be composed of (a) one di-alpha globin-like and two beta globin-like polypeptides, (b) two alpha globin-like and one di-beta globin-like polypeptides, (c) one di-alpha globin-like and one di-beta globin-like polypeptides, (d) one fused alpha/beta globin-like polypeptide and separate alpha and beta globin-like polypeptides, or (e) two fused alpha/beta globin-like polypeptides. The term "tetramer" includes "pseudotetramers."

A "genetically fused hemoglobin" is a hemoglobin-like protein comprising at least one "genetically fused globin-like polypeptide", (globin pseudooligomer), the latter comprising two or more globin-like domains which may be the same or different and which are connected directly, or through an amino acid or peptide linker. A di-alpha globin-like polypeptide is one which consists essentially of two alpha-globin-like polypeptide sequences (domains) connected by peptide bonds between the normal C- terminus of the first alpha-globin-like polypeptide (domain) and the normal N-terminus of the second alpha-globin-like polypeptide (domain). These two sequences may be directly connected, or connected through a peptide linker of one or more amino acids; the term "peptide bonds" is intended to embrace both possibilities. Alpha globin chains crosslinked at the N- and C-terminals other than by peptide bonds (e.g., by DIDS) are not di-alpha globins. The di-alpha globin-like polypeptide must be capable of folding together with beta globin and incorporating heme to form functional hemoglobin-like protein. The di-beta globin-like polypeptide is analogously defined. A di-alpha or di-beta globin-like polypeptide with a mutation in only one of the component domains is called "asymmetric".

It is also possible to provide an "alpha/beta-globin- like pseudodimer" in which an alpha globin-like sequence is connected by peptide bonds to a beta globin-like sequence. This "alpha/beta globin-like polypeptide", and the di-alpha and di-beta globin-like polypeptides, may collectively be referred to as "pseudodimeric globin-like polypeptides" or as "diglobins". By extension, a hemoglobin-like protein comprising a di-alpha, a di-beta, or a alpha/beta globin-like polypeptide is a "pseudotetramer".

Pseuddotetramers which bear only a single externally crosslinkable cysteine may be referred, by way of shorthand, as "mono-cys" molecules. However, the use of this term should not be taken as implying that the tetramer may not comprise other cysteines. A "mono-cys" pseudotetramer is merely one which has only a single cysteine which can participate to a significant degree in crosslinking reactions with a cysteine residue of a second pseudotetramer.

A hemoglobin-like protein is said to be heteromeric if at least two of its globin-like domains are different. Since conventional human hemoglobin is composed of two alpha globins and two beta globins, it is a heterotetramer. A multimeric human hemoglobin-like protein is a heteromer wherein each tetramer or pseudotetramer has two human alpha globin-like domains and two human beta globin-like domains.

### The Globin-Like Domain

The globin-like domains may be, but need not be, one per polypeptide chain, and they need not correspond exactly in sequence to the alpha and beta globins of normal human hemoglobin. Rather, mutations may be introduced to alter the oxygen affinity (or its cooperativity, or its dependence on pH, salt, temperature, or other environmental parameters) or stability (to heat, acid, alkali, or other denaturing agents) of the hemoglobin, to facilitate genetic fusion or crosslinking, or to increase the ease of expression and assembly of the individual chains. Guidance as to certain types of mutations is provided, e.g., by Hoffman and Nagai, U.S. Patent 5,028,588, and U.S. Patent No. 5,449,759. The present invention further includes molecules which depart from those taught herein by gratuitous mutations which do not substantially affect biological activity.

A "globin-like domain" is a polypeptide domain which is substantially homologous with a globin subunit of a naturally occurring hemoglobin. A "vertebrate," "mammalian" or "human" globin-like domain is one which is substantially homologous with a globin subunit of, respectively, a naturally occurring vertebrate, mammalian or human hemoglobin.

A human alpha globin-like domain or polypeptide is native human alpha globin or a mutant thereof differing from the native sequence by one or more substitutions, deletions or insertions, while remaining substantially homologous (as hereafter defined) with human alpha globin, and still capable of incorporating heme and associating with beta globin. The term "human alpha globin-like domain" is intended to include but not be limited to naturally occurring human alpha globins, including normal human alpha globin. A beta globin-like domain or polypeptide is analogously defined. Subunits of animal hemoglobins or mutants thereof which are sufficiently homologous with human alpha or beta globin are embraced by the term "human alpha or beta globin-like domain or polypeptide." For example, the subunits of bovine hemoglobin are within the scope of these terms.

In determining whether a polypeptide is substantially homologous to alpha (or beta) globin, sequence similarity is an important but not exclusive criterion. Sequence similarity may be determined by conventional algorithms, which typically allow introduction of a small number of gaps in order to achieve the best fit. A human alpha-globin-like domain will typically have at least about 75% sequence identity with wild-type human alpha globin, and greater homology with human alpha globin than with human beta globin. However, a polypeptide of lesser sequence identity may still be considered "substantially homologous" with alpha globin if it has a greater sequence identity than would be expected from chance and also has the characteristic higher structure (e.g., the "myoglobin fold") of alpha globin, the ability to incorporate heme, and oxygen-binding activity. (Note that, as elsewhere explained, an alteration in oxygen affinity (P50), intravascular retention, or cooperativity may be desired, and does not render the mutant nonhomologous if it can still contribute to reversible oxygen-binding activity.) By way of comparison, Artemia's heme-binding domains are considered homologous with myoglobin even though the primary sequence similarity is no more than 27%, as alignment of the heme- binding domains around their conserved residues and the residues conserved in other hemoglobins (i.e., involved in heme contacts or in determining the relationship of the helical segments to each other) suggested that the Artemia domains possessed the classical globin helices A to H with their corresponding turns, as well as various conserved globin family residues. Also, among the serine protease inhibitors, there are families of proteins recognized to be homologous in which there are pairs of members with as little as 30% sequence homology.

Over a hundred mutants of human hemoglobin are known, affecting both the alpha and beta chains, and the effect of many of these mutations on oxygen-binding and other characteristics of hemoglobin are known. The human alpha and beta globins themselves differ at 84 positions. In addition, interspecies variations in globin sequence have been extensively studied. Dickerson, Hemoglobin: Structure. Function. Evolution and Pathology, ch. 3 (1983) reported that in 1982, the 60 known vertebrate alpha globins had identical residues at 23 of their 141 positions, while for the 66 vertebrate beta globins considered, 20 of the 146 amino acids are identical. The 60 vertebrate myoglobins, which also belong to the globin family, had 27 invariant amino acids out of 153 positions. If only mammals are considered, then the invariant amino acids are 50/141 for the alpha globins, 51/146 for the beta globins, and 71/153 for the myoglobins. Invariant positions cluster around the centers of activity of the molecule: the heme crevice and the intersubunit contacts. Of the variable amino acids, some diverge from the consensus sequence for only a small fraction of the species considered.

The number of total differences between human alpha globin and selected other vertebrate alpha globins is as follows: rhesus monkey (4), cow (17), platypus (39), chicken (35), human zeta (embryonic) (61), carp (71), and shark (88). For invertebrate globins the divergences are sea lamprey (113), mollusc (124), Glycera (marine bloodworm) (124) and Chironomus (midge) (131). Turning to the beta globin family, the differences of human beta globin from other vertebrate beta globins are rhesus monkey (8), human delta globin (10), cow beta globin (25), cow gamma globin (33), human gamma globin (39), human epsilon (embryonic) globin (36), platypus (34), chicken (45), shark (96), sea lamprey (123), mollusc (127), Glycera (125) and Chironomus (128).

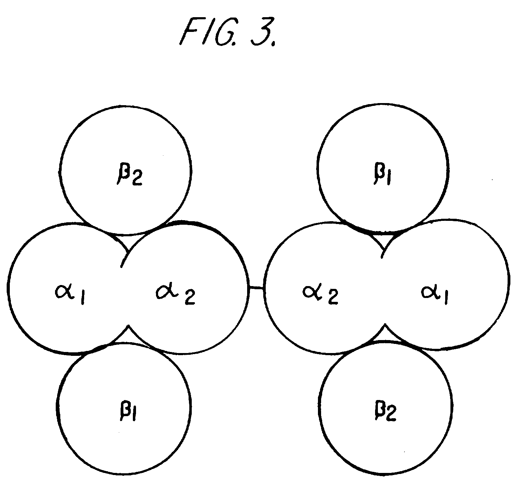

Many of these differences may be misleading -- variable amino acids may exhibit only "conservative substitutions" of one amino acid for another, functionally equivalent one. A "conservative substitution" is a substitution which does not abolish the ability of a globin- like polypeptide (or domain) to incorporate heme and to associate with alpha and beta globin subunits to form a tetrameric (or pseudotetrameric) hemoglobin-like protein which, in keeping with the definition thereof, will reversibly bind oxygen. The following resources may be used to identify conservative substitutions (and deletions or insertions):
(a) data on functional hemoglobin mutants (over a hundred such mutants exist);
(b) data on sequence variations among vertebrate, especially mammalian, alpha globins and beta globins;
(c) data on sequence variations among vertebrate, especially mammalian, myoglobins;
(d) data on sequence variations between vertebrate and invertebrate globins, or among the invertebrate globins;
(e) data on the three-dimensional structures of human hemoglobin and other oxygen-binding proteins, and molecular modelling software for predicting the effect of sequence changes on such structures; and
(f) data on the frequencies of amino acid changes between members of families of homologous proteins (not limited to the globin family). See, e.g., Table 1-2 of Schulz and Schirmer, Principles of Protein Structure (Springer- Verlag: 1979) and Figure 3-9 of Creighton, Proteins: Structure and Molecular Properties (W.H. Freeman: 1983).

While the data from (a) - (d) is most useful in determining tolerable mutations at the site of variation in the cognate proteins, it may also be helpful in identifying tolerable mutations at analogous sites elsewhere in the molecule. Based on the data in category (f), the following exchange groups may be identified, within which substitutions of amino acids are frequently conservative:
I small aliphatic, nonpolar or slightly polar residues -
   Ala, Ser, Thr (Pro, Gly)
II negatively charged residues and their amides - Asn, Asp, Glu, Gln
III positively charged residues - His, Arg, Lys
IV large aliphatic nonpolar residues - Met, Leu, Ile, Val (Cys)
V large aromatic residues - Phe, Tyr, Trp

Three residues are parenthesized because of their special roles in protein architecture. Gly is the only residue without a side chain and therefore imparts flexibility to the chain. Pro has an unusual geometry which tightly constrains the chain. Cys can participate in disulfide bonds which hold proteins into a particular folding. Note that Schulz and Schimer would merge I and II above. Note also that Tyr, because of its hydrogen bonding potential, has some kinship with Ser, Thr, etc.

In general, functionality is less likely to be affected by mutations at surface residues, at least those not involved in either the heme crevice or the subunit contacts. In addition, "loops" connecting alpha helices, as well as free amino or carboxy termini, are more tolerant of deletions and insertions.

A "Met FX alpha globin" is an alpha globin-like polypeptide comprising an N-terminal methionine, a oligopeptide which acts as a recognition site for Factor Xa (e.g., Ile-Glu- Gly-Arg) (SEQ ID NO:2), and an alpha globin-like sequence (e.g., Val-His-Leu- Thr-Pro...) (SEQ ID NO:3) which may correspond to wild-type alpha globin or to a mutant thereof as taught herein. The term "Met FX alpha globin" is some-times abbreviated as "FX alpha globin". "FX beta globin" is an analogously defined beta globin-like polypeptide.

"Met-alpha globin" is an alpha globin-like polypeptide with an extra N-terminal methionine. The second amino acid is valine, which is the first amino acid of mature wild-type alpha globin. Met-beta globin is analogously defined. A "Des-FX alpha globin" gene (or "dFX alpha globin") is a Met-alpha globin gene obtained by excising the FX codons from a Met-FX alpha globin gene. Note that "Met-Hgb" is used to refer to methionyl Hgb formed from methionyl-alpha globin and methionyl-beta globin.

"Des-Val-alpha globin" (or "dVal alpha globin") is an alpha globin-like polypeptide wherein methionine is substituted for the valine which begins the sequence of mature wild-type alpha globin. Des-Val-beta globin is analogously defined. Des-Val-alpha/alpha globin (di-Des-Val-alpha globin) is a "di- alpha globin" in which a "Des-Val-alpha" sequence is linked via an appropriate peptidyl linker to an alpha globin-like sequence which begins with Val.

### Low Affinity Mutants

The term "low affinity hemoglobin-like protein" refers to a hemoglobin-like protein having a P₅₀ which is at least 10% greater than the P₅₀ of cell free normal hemoglobin Aₒ under the same conditions. Preferably, the protein, if used as a blood substitute, qualifies as a low affinity protein, and more preferably, its P₅₀ is closer to the P₅₀ of whole blood cells than to that of cell free hemoglobin.

Low affinity mutant hemoglobins, i.e., those with "right shifted" oxygen equilibrium binding curves relative to cell-free normal hemoglobin, have many potential uses. Most notably, mutant hemoglobins that have an oxygen affinity similar to whole red blood cells may be used as an oxygen- carrying transfusion substitute in place of donated red blood cells, eliminating the risk of infection and alleviating problems with supply. Cell-free native human hemoglobin cannot function as a transfusion substitute, among other reasons because oxygen is bound too tightly. In addition, because cell-free hemoglobin solutions do not need to be cross-matched and are expected to have a longer shelf life than whole blood, low affinity hemoglobin solutions may be widely used in situations where whole blood transfusion is not feasible, for example in an ambulance or on a battlefield. Mutant hemoglobins that have an even lower oxygen affinity than red blood cells may in fact delivery oxygen more effectively in many situations. Mutant hemoglobins that have a somewhat higher oxygen affinity than whole blood (but a lower affinity than cell-free native human hemoglobin) will still function as an adequate transfusion substitute and may in fact deliver oxygen more effectively than red blood cells in some situations. This is because oxygen is released directly to plasma from hemoglobin-based solutions, without the need to diffuse through the red cell membrane, and because cell-free hemoglobin may penetrate into regions not accessible to red blood cells. As an example, low affinity mutant hemoglobin is expected to deliver oxygen effectively during coronary artery balloon angioplasty procedures, whereas circulation of red blood cells is obstructed during such procedures. Low affinity mutant hemoglobin may also be useful as a perfusion component in organ preservation prior to transplantation or as a mammalian cell culture additive.

Possible low affinity mutants are discussed in detail, by way of example and not of limitation, in Table 1 (natural low affinity hemoglobin mutants) and Table 2 (candidate non-naturally occurring low affinity hemoglobin mutants) of Hoffman, et al., U.S. Patent 5,028,588. Low affinity mutants of particular interest are the Presbyterian (beta Lys¹⁰⁸) beta Phe⁶³, beta Ile⁶⁷, and Kansas (beta Thr¹⁰²) mutants.

An unexpected and surprising change in oxygen binding characteristics of hemoglobin was observed upon replacement of the N-terminal valine with methionine. Hemoglobin Aₒ purified from blood has a P₅₀ value of 4.03 with N=2.8 when measured at 25°C. DesFX-hgb produced in E. coli, a hemoglobin identical to Aₒ except for the addition of a methionine at the N-termini of the alpha and beta chains, has essentially the same P₅₀ and N values. Thus, the addition of a methionine, without altering the adjacent valine residue, has little or no effect on oxygen binding. On the other hand, a higher P₅₀ value, 6.6, was observed for desVal-hgb produced in E. coli, a hemoglobin in which the normal N-terminal valine of each chain was replaced with methionine. Cooperativity, as measured by N, was virtually the same, however, for all three molecules.

A similar comparison was made for two hemoglobins each containing the Presbyterian mutation, one produced in E. coli and one in yeast. The E.coli hemoglobin was constructed with a Des-Val alpha chain, i.e., the N-terminus had the normal valine replaced with methionine. Oxygen binding was characterized by P₅₀=19.8, N=2.5 at 25°C. and by P₅₀=34.5 and N=2.5 at 37°C. The corresponding yeast coding region begins with an additional methionine codon in front of the normal valine codon. Because this initial methionine is removed post translationally in vivo, the purified hemoglobin has a normal N-terminal valine. For this molecule, P₅₀=23 to 25 and N=2.5 when measured at 37°C. Thus, in the above instances, the replacement of an N-terminal valine with an N- terminal methionine increased the P₅₀ value. Under physiological conditions, it is expected that the genetically fused Presbyterian hemoglobin produced in E. coli will deliver 20-30% more oxygen than the similar hemoglobin, with its altered N-terminus, produced in yeast.

### High Affinity Mutants

The term "high affinity hemoglobin-like protein" refers to a hemoglobin-like protein having a P₅₀ which is at least 10% less than the P₅₀ of cell free hemoglobin Aₒ under the same conditions.

High affinity mutant hemoglobin may have utility in certain situations. For example, perfluorocarbon-based blood substitute preparations are under clinical study for enhancement of radiation therapy and certain chemotherapy treatments of solid tumors (Dowling, S., Fischer, J.J., and Rockwell, S. (1991) Biomat. Art. Cells Immobil. Biotech, 19, 277; Herman, T.S. and Teicher, B.A. (1991) Biomat. Art. Cells and Immobil. Biotech, 19, 395; Holden, S.A., Teicher, B.A. and Herman, T.S. (1991) Biomat. Art. Cells and Immobil. Biotech, 19, 399.) The basis of these investigations is the fact that oxygen is a required component of the cell toxicity action of radiation and certain chemotherapy reagents. Solid tumors frequently exhibit extremely low partial oxygen pressure in the interior of the tumor, rendering therapy inefficient. Perfluorocarbon-based oxygen-carrying solutions appear to dramatically enhance certain tumor therapies, and hemoglobin- based blood substitutes are expected to have a similar utility. It is likely that cell-free hemoglobin unlike whole red blood cells, will be able to penetrate the interior region of tumors for delivery of oxygen. Actual percent of oxygen released by a cell-free hemoglobin preparation is not a direct function of P₅₀ but rather depends on the shape of the oxygen equilibrium binding curve between the two pressures representing the partial oxygen pressure of the lungs (where oxygen is loaded onto hemoglobin) and the partial pressure of the tissue where oxygen is unloaded. Therefore, it is possible that a high affinity mutant hemoglobin would be preferred as a tumor therapy adjuvant. A high affinity hemoglobin would retain its bound oxygen throughout the normal circulatory system, where partial oxygen pressure remains relatively high, but release its oxygen in the extremely oxygen-depleted tumor interior. Normal or low affinity hemoglobin might have less hemoglobin available for release by the time it reaches the interior of the tumor.

Naturally occurring high affinity hemoglobin mutants are also known, see Bunn and Forget, Table 14-1, and candidate non-naturally occurring high affinity hemoglobin mutants may be proposed in view of the known mutants and hemoglobin structure. Particularly preferred high affinity mutants are set forth in Table 400.

It should be noted that genetic fusion and crosslinking can affect oxygen binding affinity.

### Cysteine Mutations and Disulfide Bridge Formation

Cysteine mutations are of value for increasing the stability of the tetramer (See USP 5,028,588 and U.S P 5,449,759. They also facilitate constructing poly(tetrameric) (n>=2) hemoglobins. This is because the cysteines on adjacent tetramers (including pseudotetramers) can be oxidized to form a disulfide bridge, covalently coupling the tetramers. In addition, the thiol groups of cysteines may be reacted with a variety of crosslinking agents.

A variety of sites are available for introduction of cysteines into a hemoglobin-like protein.

The criteria governing site selection are: (1) the mutation does not affect functionality; (2) the side chain is accessible to water in oxy or deoxy structure; (3) the site should lie on the surface of the folded protein; (4) the sulfhydryl of the side chain should extend away from the surface rather than toward the interior of the molecule; (5) the site should be in a portion of the molecule that is not directly invovled in the R->T transition; (6) the change should be in a portion of the molecule that does not have a tightly fixed position (such regions generally give indistinct X-ray diffraction patterns); (7) the mutations will not destroy the local secondary structure, i.e., avoid pro->cys mutations, which might result in a refolding problem; and (8) if possible, a conservative change should be made such as ser->cys or ala>cys. A mutation does not necessarily have to meet all of the above requirements to be useful. For example, one might envision a site that is involved in the R->T transition (cf. 5 above) but confers a beneficial change in P₅₀ (cf. 1 above) because of that involvement. The most important considerations are that the mutation does not abolish O₂ binding, before or after crosslink formations, and that the cysteine is accessible for participation in the desired crosslinking reaction.

Candidate sites on the alpha surface include: his72, asn 78, asn68, ala71, thr67, lys7, lys11, thr8, ala12, thrll8, lys16, ala45, glu116, glyl5, his112, thr24, glu23, lys60, lys56, his50, gly51, glu53, ser49, asp47, glns4, his45, lys90, ala82, lys61, ala19, his20, asp85, ser81, asp75, asp74, lys139, asp64, and gly18 (total 40 amino acids).

Candidate sites on the beta surfaces includes: asp79, his2, leu3, thr4, glu6, ser9, thr12, ala13, gly16, lys17, va118, asn19, va120, asp21, glu22, lys65, ser72, ala76, his77, asp79, asn80, gly83, ala86, thr87, glu90, lys95, lys59, glu43, ser44, asp47, ser49, thr50, ala53, asp52, lys61, glu121, lys120, thr123, lys66, asp73, ala62, his116, his117 (total 45 amino acids).

There are a number of naturally occurring mutants which already show mutations at these sites. These are listed below:

| Residues | Region | Mutation |
|---|---|---|
| 19 | AB1 | ALA->GLU |
| | | ALA->ASP |
| | | |
| 54 | E3 | GLN->ARG |
| | | GLN->GLU |
| | | |
| 71 | E20 | ALA->GLU |
| | | |
| 75 | EF4 | ASP->GLY |
| | | ASP->HIS |
| | | ASP->TYR |
| | | ASP->ASN |
| | | |
| 81 | F2 | SER->CYS |
| | | |
| 47 | CE5 | ASP->GLY |
| | | ASP->HIS |
| | | ASP->ASN |

If the pseudo-octamer (n=2) is formed by directly linking two pseudo-tetramers via a disulfide bond, the halflife in serum may be influenced by the rate at which endogenous serum small molecule thiols (such as glutathione) reduce the disulfide bond. The mechanism of these reactions involves the thiolate anion as the actual reducing species (Creighton, T.E. (1978) Prog. Biophys. Molec. Biol., 33:259-260; Creighton, T.E. (1975) J. Mol. Biol., 96:767; Creighton, T.E. (1977) J. Mol. Biol., 113:313). Thus the rate of reduction will be a function of the molecular electrostatic environment in the vicinity of the disulfide bond. A slower rate of reduction would be predicted if the disulfide was located in an electrostatically negative environment,, due to the repulsion of the thiolate anion. In the case of glutathione, even the unreactive transient protonated species has a net negative charge and would be repulsed, thus further reducing the rate of disulfide reduction.

A surface or near-surface amino acid residue of di- alpha or di-beta hemoglobin that is located in close proximity to a negatively charged surface residue might therefore be a good choice for location of a single cysteine mutation in the di-alpha or di-beta polypeptide. Although formation of the initial disulfide bond between two such cysteines might also be slower because of repulsion between the negative charges on the two hemoglobin molecules in the vicinity of the cysteines, the reaction could be facilitated by use of high salt or high pH during the in vitro bond formation reaction. If carried out under deoxy conditions in a redox buffer, the reaction might also be facilitated by temperature elevation.

### Preferred sites for cys mutations proximal to negative charged residues

- alpha ser49: near asp47; naturally occurring ser49 to arg has normal O₂ affinity
- alpha his20: near glu23; naturally occurring his20 to tyr, gln, arg have no known undesirable properties
- alpha lys16: near glu116; naturally occurring lys to glu has normal O₂ affinity
- alpha his50: near glu30; naturally occurring his50 to asp has no known undesirable properties
- beta thr50: near asp52; naturally occurring thr50 to lys has no known undesirable properties
- beta lys65: near asp21
- beta asn19: near asp21

Surface or near-surface cysteine mutations in general are not expected to have major effects on the functionality of the hemoglobin pseudotetramer. Cysteine mutations would not be expected to significantly destabilize alpha helices, and surface residues are not directly involved in the oxygen binding properties of hemoglobin. Most surface residues undergo considerable motion and are not tightly constrained. It should also be noted that because of protein breathing motions, the cysteine side chain would not necessarily have to point directly into solution to be accessible for disulfide bond formation.

In addition to the use in construction of a pseudo- octamer, there may be additional uses of surface cysteine mutations. These include: (1) construction of multimeric hemoglobins (n>2) by use of synthetic sulfhydryl reactive peptides with more than two reactive sites; (2) surface cysteine residues could be used to attach chelates that bind radioisotopes for imaging; and (3) surface cysteines could be used to attach bio-active peptides or other therapeutic agents to increase their circulating half-life, or target their delivery. If the attachment of the drug were via a disulfide, the rate of release of the peptide from its carrier could be controlled by neighboring residues. For uses (2) and (3), restriction to one cysteine per di-alpha or di-beta is unnecessary.

It may be desirable to eliminate the cysteine at beta 93 of normal human hemoglobin so that it cannot participate in polymerization reactions. This cysteine may be replaced by serine, alanine or threonine, for example. Other wild-type cysteines may also be replaced, if desired, but it is unlikely that they participate in crosslinking reactions after the tetramer is formed.

### Mutations to Reduce Haptoglobin Binding

It is presently believed that haptoglobin binding plays a role in the catabolism of hemoglobin. If so, intravascular retention of hemoglobin might be enhanced by mutations which inhibit haptoglobin binding. Oxyhemoglobin dissociates into alpha-beta dimers, which are then bound by haptoglobin. While much of the binding energy is associated with binding to residues which are buried in the tetramer but exposed in the dimer, it appears that there are also secondary binding sites on the surface of the tetramer. Though the mechanism is not clear, the haptoglobin-bound dimers are transported to Kupffer cells in the liver, where they are catabolized.

It would be most desirable to mutate sites which both are involved in haptoglobin binding and which are suitable for attachment of another tetramer. Candidate mutation sites are in the alpha chain of normal human alpha globin, residues 1, 6, 74, 82, 85, 89, 90, 93, 118, 120-127 and 139-141, and in the beta chain, residues 2, 11-40 and 131-146. It is unlikely that haptoglobin binding can be blocked merely by single substitution mutation of one genetically encoded amino acid to another. However, if the above residues are replaced by a cysteine, and the cysteine is crosslinked to another molecule which is significantly larger than the usual amino acid side chain, the steric effect is magnified considerably and haptoglobin binding may be inhibited. Of course, to retain polymerization control, these mutations should be made asymmetrically in a pseudooligomeric polypeptide so that there is only one crosslink per tetramer.

It is known that even covalently crosslinked hemoglobins can be processed by haptoglobin; this is thought to be the result of the "breathing" of the tetramer in its oxy form sufficiently to allow the haptoglobin access to the normally buried residues of the subunit interfaces in question. This may be prevented by tightly crosslinking the globin subunits so dissociation will not occur within the time span of interest. Unlike the mutations discussed above, these mutations should be made in all of the indicated subunits for maximum efficiency.
beta37->Cys and alpha92->Cys
beta40->Cys and alpha92->Cys
beta97->Cys and alpha41->Cys

The above mutations all lie at the alpha₁beta₂ and beta₁alpha₂ interfaces and lock these interfaces shut so that "breathing" does not allow haptoglobin access.

"Breathing" may also be inhibited by low oxygen affinity mutations; the tetramer then spends more time in the deoxy state, which is not susceptible to haptoglobin attack.

### Pseudomeric Globin-Like Polypeptides and Pseudotetrameric Hemoglobin-Like Proteins Useful as Intermediates in Preparation of Multimeric Hemoglobin-Like Proteins

In the liganded form, haemoglobin readily dissociates into αβ dimers which are small enough to pass through the renal glomeruli, and Hb is thereby rapidly removed from the circulatory system. Intravenous administration of haemoglobin in amounts far less than that needed to support oxygen transport can result in long term kidney damage or failure. Ackers, G.K. and Halvorson, H.R., Proc. Nat. Acad. Sci. (USA) 71, 4312-16 (1974); Bunn, H.F., Jandl, J., J. Exp. Med. 129, 925-34 (1969). If dissociation into dimers is prevented, there is an increase in intravascular half life and a substantial reduction off renal toxicity. Lee, R., Atsumi, N., Jackbs, E., Austen, W., Vlahakes, G., J. Surg. Res. 47, 407-11 (1989). The hemoglobin-like proteins of the present invention cannot dissociate into αβ-dimers without the breakage of a peptide bond and should have the advantages of a longer intravascular half life and reduced renal toxicity.

In the crystal structures of both deoxyhaemoglobin and oxyhaemoglobin the N-terminal Val residue for one α subunit and the C-terminal Arg residue of the other « subunit are only between 2 and 6 Å apart, and are bound to one another through a salt bridge in deoxyhaemoglobin. Fermi, G., Perutz, M., Shaanan, B., Fourme, R., J. Mol. Biol., 175, 159-74 (1984); Shaanan, B., J. Mol. Biol. 171, 31-59 (1983). This distance could be spanned by one or two amino acids. One extra amino acid can be added to the C-terminal Arg residue of the α subunits by trypsin catalyzed reverse hydrolysis without significantly altering the oxygen binding properties. Nagai, K., Enoki, Y., Tomita, S. and Teshima, T., J. Biol. Chem., 257, 1622-25 (1982) Preferably the di-alpha linker (if one is used) consists of 1 to 3 amino acids which may be the same or different. A Mono-Gly linker is especially preferred. In designing such a linker, it is important to recognize that it is desirable to use one or more amino acids that will flexibly connect the two subunits, transforming them into domains of a single di-alpha globin polypeptide.

The preparation of "di-beta" mutants is also contemplated. The distance between the N-terminus of one beta subunit and the C-terminus of the other is 18.4 Å in the deoxy configuration and 5.2 Å in the oxy form. Preferably, the di- beta linker consists of 2 to 9, amino acids which may be the same or different. Glycine amino acids are particularly preferred.

The length of the (-gly-)ₙ genetically fused link between the N-terminus of one beta chain (at beta₁, 1 Val) and the C terminus of the second beta chain (beta₂, 146 His) in di- beta hemoglobin may range between 1 and approximately 9 glycines. In the oxy and deoxy crystal structures of human hemoglobin Aₒ, the distance between these termini is 5.22 Å and 17.93 Å respectively (from the N-terminal nitrogen to the C terminal carbon of the carboxylate). A single glycine linker, which is a little less than 4 Å in length, may come close to linking the two termini in the oxy structure, however, it is expected that this linker will fall ^{~} 14Å short in the deoxy structure. Significantly more perturbation of the deoxy structure vs the oxy structure might be anticipated with this linker. Some alterations in the oxygen binding properties may be caused by deletion of the positive and negative charges at the two termini and their inclusion in the amide bond. In addition, the linker molecule itself may destabilize the oxy structure less than the deoxy structure, and thus lead to a relative increase in oxygen affinity. Likewise, two glycines inserted as linkers may also differentially stabilize the oxy structure and hence relatively increase the oxygen affinity by the same mechanism described above.

When the number of linking glycines is increased to 5, the linker should just span the cleft between the beta chain termini in the deoxy structure, and, moreover, insert added steric bulk between the termini in the oxy structure, thus leading to a relative stabilization of deoxy (or destabilization of oxy) and perhaps resulting in a concomitant decrease in oxygen affinity. Due to the large space between the beta termini in the deoxy (but not the oxy structure), addition of glycine linkers in the range of 6-9 may further stabilize the oxy structure and, in the same manner, further decrease oxygen affinity.

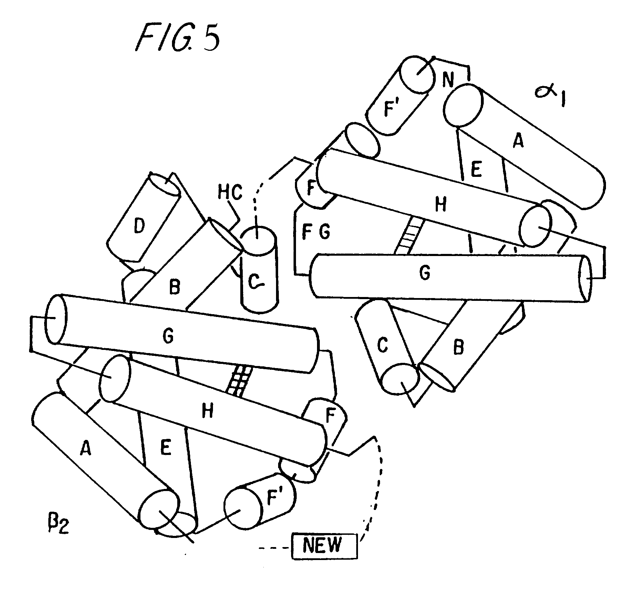

A third form of globin pseudodimer is one comprising both alpha and beta globin domains. A possible route to fusing alphal to beta2 and so stabilizing hemoglobin against α₁β₁/α₂β₂ dimer formation, is to fuse the alphal C-terminal residue to the N-terminal residue of beta2 C helix, creating a new C- terminus at the end of the beta2 B helix. The original beta N terminus, Vall, would be fused to the original beta subunit C- terminal residue, His146, by means of an intervening new section of protein, thus creating a continuous polypeptide chain comprising the alpha and beta subunits of different dimers. This chain may be described as follows: α(1-14)-Gly₃- β(35-146)-Gly₁₋₃-Ala₁₃-Gly₁₋₃-β(1-34); See Figure 5.

Inspection of the structure of human deoxyhemoglobin using a molecular graphics computer indicates the following relevant distances. The distance between the Alphal Arg141 carboxyl carbon and Beta2 Tyr35 N atoms is approximately 8.6 Angstroms. A fully extended linear triglycine peptide measured approximately 10.1 Angstroms from the N to C terminal residues. This suggests that three glycine residues could be employed to span the distance between the Arg141 and Tyr35 residues with a minimum of unfavorable steric interactions and maximum conformational freedom. The distance requirements could be different in oxyhemoglobin, and if so, the sequence of the fusion peptide could be altered to best accommodate the requirements of both structures.

In human deoxyhemoglobin, the distance between the Beta2 His146 carboxyl carbon and the Beta2 Vall nitrogen atoms is approximately 25 Angstroms. A right handed 3.6 Alpha helix constructed from a linear sequence of 13 Alanine residues was found to measure 22 Angstroms from N to C terminus. With the addition of one to three glycine residues at each end of this helix (to give Glyₙ(Ala)₁₃Glyₙ where n=1 to 3), it could span the required distance and have sufficient conformational flexibility to avoid serious tertiary packing conflicts. Additionally, the amino acid sequence of the helix could be altered to introduce favorable hydrogen bonds and salt bridges between the new helix and the Beta2 helix against which it would pack in the folded protein. Such interactions could aid stabilization of the engineered protein.

Glycine is the preferred amino acid in the linkers, since it is known to be quite flexible, Cantor and Schimmel, Biophysical Chemistry, part 1, pp. 266-9 (1980), and also allows chains into which it is incorporated to assume a more compact structure. However, the residues comprising the linker are not limited to glycines; other residues may be included instead of or in addition to glycine, such as alanine, serine, or threonine. Since these amino acids have a more restricted conformational space in a protein, they will likely result in more rigid linking chains, and hence have a more pronounced effect on the relative stabilization/destabilization of the oxy/deoxy structures.

It should be understood that the minimum and maximum number of amino acids in the linker is a function of the distance to be spanned in both the oxy or deoxy forms, the amino acids chosen, and the propensity of the particular amino acid sequence to form a secondary structure. While a random coil is usually preferred, it is not required, and a linker with a large number of amino acids in a secondary structure may have the same span as a random coil linker with fewer amino acids. A linker may comprise, e.g., 1-3 glycines, followed by a sequence having a secondary structure, followed by 1-3 more glycines. The translation per residue, in angstroms is 1.9 for polyproline I, 3.12 for polyproline II, 3.1 for polyglycine II, 3.4 for an antiparallel β sheet, 3.2 for a parallel β-sheet, 1.5 for a right handed α-helix, 2.0 for a 310 helix, and 1.15 for a π helix. In a fully extended chain, the maximum translation per residue is 3.63 Å if the repeating units are staggered and 3.8Å if the peptide bond is trans.

The number of amino acids in the linker may be such that a formation of a secondary structure, such as an alpha helix or a beta-sheet, is undesirable, as the span is reduced. Certain amino acids have a greater tendency to participate in such structures. See Chou and Fasman, Biochemistry, 13:222-245 (1974), incorporated by reference. The amino acids are ranked in order of decreasing participation below. The preferred linker amino acids are boldfaced. Glycine is the most suitable amino acid for this purpose. The most preferred di-alpha linkers are Gly or Gly-Gly.

| Alpha Helix Formers | Beta Sheet Formers |
|---|---|
| Glu (1.53) | Met (1.67) |
| Ala (1.45) | Val (1.65) |
| Leu (1.34) Hα | Ile (1.60) Hβ |
| His (1.24) | Cys (1.30) |
| Met (1.20) | Tyr (1.29) |
| Gln (1.17) | Phe (1.28) |
| Val (1.14) | Gln (1.23) |
| Trp (1.14) | Leu (1.22) |
| Phe (1.12) hα | Thr (1.20) |
| **Lys** (1.07) | Trp (1.19) hβ |
| Ile (1.00) | Ala (0.97) Iβ |
| **Asp** (0.98) | **Arg** (0.90) |
| Thr (0.82) | **Gly** (0.81) |
| **Arg** (0.79) | **Asp** (0.80) iβ |
| **Ser** (0.79) | **Lys** (0.74) |
| Cys (0.77) iα | **Ser** (0.72) |
| **Asn** (0.73) | His (0.71) |
| Tyr (0.61) bα | **Asn** (0.65) |
| **Pro** (0.59) | **Pro** (0.62) bβ |
| **Gly** (0.53) Bα | Glu (0.26) Bβ |

(The letter symbols are Hα, strong α former; hα, α former; Iα; weak α former; iα, α indifferent; bα, α breaker; and Bα strong « breaker. The β symbols are analogous. Trp is bβ if near the C-terminal of a β-sheet region.)

The alpha helix of a polypeptide chain comprises an average of 3.6 residues per turn. In globular proteins, the average length is about 17Å, corresponding to 11 residues or 3 helix turns. In alpha and beta globin, the helices range in length from 7 to 21 amino acids (A.A.). The beta pleated sheet comprises 2.3 residues per turn; the average length is about 20Å or 6 residues.

Chou and Fasman define an alpha helix nucleus as a hexapeptide containing four helix forming residues and not more than one helix breaker, and a beta sheet nucleus as a pentapeptide containing three beta sheet forming residues and not more than one sheet breaker.

The amino acid sequence in the vicinity of the di-alpha linker is as follows:

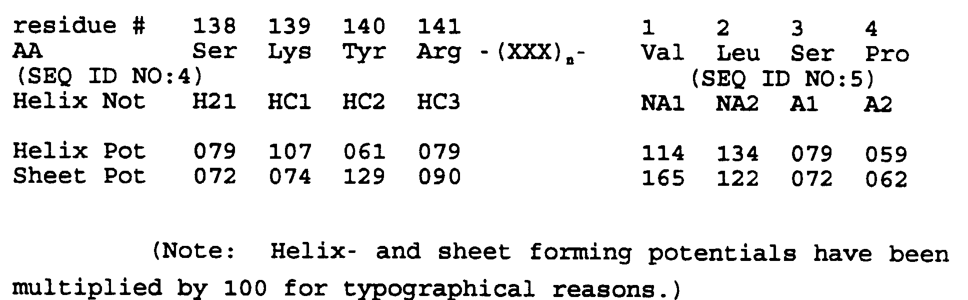

The di-alpha linker is preferably only 1-3 amino acids. Thus, it can form an alpha helix only in conjunction with the linker "termini". A one or two residue linker, even if composed of amino acids with strong secondary structure propensities, would be unlikely to assume an alpha helix or beta sheet configuration in view of the disruptive effect of, e.g., Arg 141 or Ser 3. If the linker is 3 residues long, it would be preferable that no more than one residue be a strong alpha helix former, unless the linker also included a strong alpha helix breaker.

The amino acid sequence in the vicinity of the di- beta linker may impose more stringent constraints.

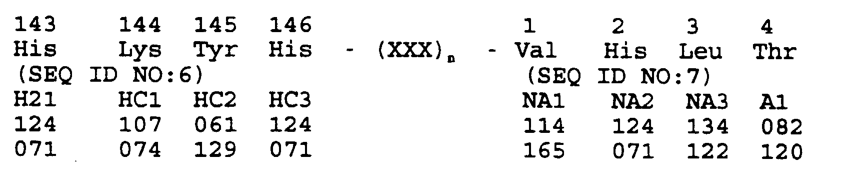

The di-beta linker is likely to be longer (preferably 1-9 A.A.) and therefore more susceptible to secondary structure formation. If secondary structure formation is not desired, it is desirable that the amino acid adjacent to Val-1 be an alpha helix breaker (e.g., Glycine) in view of alpha- helix propensities of Val-His-Leu. More generally, it is desirable that the linker not contain (or cooperate with the proximately linked amino acids to form) an alpha helix nucleus or beta sheet nucleus.

When secondary structure is not desired, amino acids with a high propensity toward alpha helix formation may be used in the linker if accompanied by "helix breaking" amino acids. Similarly, Beta sheet formation may be prevented by "sheet disrupting" amino acids.

Of course, prediction of secondary structure using Chou and Fasman's approach has its limitations and the ultimate test of the acceptability of a linker is whether or not the di- alpha or di-beta hemoglobin has the desired affinity for oxygen. In particular, a poly-alanine linker, despite its supposed propensity to alpha-helix formation, may well be of value since the alanine group is compact and therefore the linker should be quite flexible if secondary structure does not form.

In an especially preferred embodiment, di-alpha and beta globin genes are combined into a single polycistronic operon. The use of a polycistronic operon is not, however, necessary to practice the present invention, and the alpha (or di-alpha) and beta (or di-beta) globin genes may be expressed from separate promoters which may be the same or different.

While the preferred "genetically fused hemoglobin" of the present invention is one comprising a di-alpha and/or di- beta globin, other globin chains may be genetically fused and used in the production of multimers of hemoglobins of species other than Hgb Al (α₂β₂).

### Pseudo-Octameric (Ditetrameric) Hemoglobin-like Proteins With Disulfide Bridges

The ability to produce pseudotetrameric recombinant hemoglobins consisting of a single dialpha polypeptide and two beta chains (or a dibeta polypeptide and two alpha chains) provides a unique opportunity to create an asymmetric pseudotetramer from the normally symmetric pseudotetramer. Because the two alpha globin domains are expressed as a single polypeptide, it is possible to alter one of the alpha globin domains without altering the other. The result is a protein that, in its final folded state, contains two different alpha globin domains in a strict 1:1 ratio. This type of asymmetric hemoglobin molecule, with its unique chemical properties, cannot be easily constructed by any other method. A preferred embodiment of this invention would involve use of site-directed mutagenesis to substitute a cysteine residue in one of the two alpha globin domains of a di-alpha hemoglobin such as SGE1.1 (a di-alpha hemoglobin with a beta chain Presbyterian mutation) such that the cysteine would be on the surface of the folded recombinant hemoglobin molecule. A homogeneous preparation of pseudo-octameric hemoglobin could then be formed through interhemoglobin linkage of two pseudotetramers either directly by simple oxidation of purified pseudotetramers or by reaction with a bridging molecule (Figure 3).

Although direct formation of a disulfide bond between two "mono cys" tetramers is desirable in order to avoid the need for chemical crosslinking, naturally occurring reducing agents may reduce the disulfide bond in vivo at a significant rate. Preliminary experiments suggest that the rate of reduction of the bond may be influenced by the location of the cysteine mutation on the surface of the hemoglobin.

The surface cysteine mutants (MW = 64 kDa) can be oxidized to the disulfide-linked dimer under oxidative conditions. This can be accomplished by stirring a concentrated solution of the expressed protein at pH 8 under pure oxygen at 4°C or room temperature in the dark. Trace levels of transition metal ions such as Cu⁺² may be added to level below 1 uM to catalyze the oxidation (1). Formation of the 128 kDa octamer can be monitored by gel filtration. Saturation of the solution with oxygen at elevated pH should minimize autooxidation of recombinant hemoglobin.

An alternative procedure, which may be the preferred method of catalyzing this reaction, involves the use of redox buffers such as reduced and oxidized glutathione, or reduced and oxidized dithiothreitol (2). This catalysis of the reaction through disulfide interchange may be necessary to control trace transition metal catalysis (3). An second, similar approach involves conversion of the surface cysteines in the 65 kDa species to sulfonates before purification (to avoid 128 kDa species formation during purification), followed by conversion to the disulfide-linked 128kDa species with reduced glutathione (2).
(1) Freedman, R.B. and Hillson, D.A. (1980) "Formation of Disulfide Bonds"IN: The Enzymology of Post Translational Modification of Proteins, Vol. 1, p. 157 ff. (Academic Press).
(2) DiMarchi, R., et al. (1988) Chemical synthesis of human epidermal growth factor (EGF) and human type a transforming growth factor (TGFa) IN: Peptides: Chemistry and Biology (G.R. Marshall, ed.) pp. 202-203 (Leiden:ESCOM).
(3) Creighton, TE (1978) Experimental studies of protein folding and unfolding. Prog. Biophys. Molec. Biol. 33:231-297

### Multimeric Hemoglobin-Like Protiens With Other Intercysteine Linkages

It is also possible, of course, to couple two mono cys molecules with a homobifunctional crosslinking reagent resulting in linkage via nonreduceable bonds. The degree of polymerization is still controlled by the use of the mono cys di-alpha or di-beta Hgb starting material.

By using bi-, tri-, tetra-, hexa-, or octa-functional crosslinkers several properties of multimeric hemoglobin which may contribute to longer serum half life can be controlled. The crosslinkers can be designed to give a nonreducible bond between two tetramers, to yield high molecular weight multimers of n>2 psuedo-tetramers (e.g. dodecamers, etc.) and/or to drop the overall isoelectric point of a hemoglobin octamer to further increase its half life.

Correlations of molecular weight with serum half life for proteins such as IL-2, demonstrate that a significantly longer half life may be expected as the molecular weight of a protein increases, particularly above the renal filtration limit of 50-70 kDa. However, a factor potentially limiting the half life of multimeric hemoglobin formed by a disulfide link between tetramers is reduction of the cys-cys disulfide bond by endogenous thiol-reducing agents found in the serum. Estimates of small molecule thiol levels in plasma vary from 17 µM to 5 µM. The major species is reduced glutathione. Other thiol compounds in plasma include cysteine, homocysteine, and gamma-glutamyl cysteine. Thus, small molecule plasma thiols are available for reduction of disulfide bonds. This may be reflected in the diminished half life seen with antibody-ricin A chains conjugates linked by regular disulfides (6.7 hrs) relative to conjugates linked with sterically hindered, and thus less reducible, alpha-methyl disulfides (42.5 hours).

Thus, in one embodiment, the octameric hemoglobin features a nonreducible sulfur-containing crosslink such as a thioether bond or thiol-maleiimide adduct. These may substantially extend the multimer half life. Simple homobifunctional crosslinkers or polyethylene glycol (peg) derivatives would likely be useful for this purpose (see below). The reaction of a bifunctional cysteine-specific crosslinker with a mono-cys di-alpha or di- beta Hgb should limit the products of the reaction to a dumbbell-like octameric hemoglobin and unreacted hemoglobin. The reaction should be stoichiometric when the Hgb and crosslinker are present at high concentrations and the Hgb is present in a slight excess over the crosslinker maleiimides at pH 6.5-7.0. Further, there should not be substantial interference by reaction with globin lysines. The preferential reactivity of the thiols to lysines can be roughly calculated as the product of their molar ratios and the ratio of the intrinsic reactivity of a maleiimide to thiols versus amines. This product is ca. [1 cys/40 lys]x[1000]=25 at pH 7. The side products would still be octamers, with one attachment site being a secondary amine and thus might well be functionally equivalent to the S-crosslinked octamers. Hydrolysis of the maleiimide adduct at pH 7 would be slow, and the ring opening would leave the crosslink intact.

The reaction of the thioether RC(=O)CH₂I with the sulfhydryl-bearing protein (R'SH) results in the crosslink RC(=O)CH₂-S-R'. The reaction of the maleiimide with the protein results in the addition of the R'SH across the double bond of the five-membered maleiimide ring, yielding a thiomaleiimide adduct.

The following are examples of homobifunctional crosslinkers that may form metabolically stable crosslinks between monocysteine pseudo tetramers:
1) 1,2-bis-(2-iodoethoxy)ethane
2) 4,4'-dimaleiimidylbenzene or N,N'-p-phenylenedimaleiimide
3) N,N'-bis-(3-maleiimido-propionyl)-2-hydroxy-1,3-propane diamine.

Longer half lives may also be obtained by increasing the apparent solution molecular weight by simply lengthening the distance between the two linked tetramers using a long crosslinking agent. The use of some potentially novel polyethylene glycol derivatives as homobifunctional crosslinkers, reacting with SGE1.1 monocys, may provide one mechanism for significantly increasing the molecular weight of octameric hemoglobin by virtue of the length of the crosslinker alone.

A suitable crosslinker for this purpose is

maleiimido-CH₂CH₂C(=O)(OCH₂CH₂)ₙOC(=O)CH₂CH₂-maleiimido.

The length may be adjusted by variation of n. A few examples are given below.

| Structure | Max Length | Source |
|---|---|---|
| n = 22 | ^{~} 49Å | peg -1000 |
| n = 76 | ^{~}166Å | peg -3350 |
| n = 227 | ^{~}499Å | peg -10000 |

Homobifunctional N-hydroxysuccinimide-activated peg has been used previously to derivatize hemoglobin. Yabuki, et al., Transfusion, 30:516 (1990). This reaction resulted in a polydisperse mixture of monomeric, dimeric, and trimeric species with an average stiochiometry of peg/hemoglobin of 6.2. However, 83% of the hemoglobin derivatized by peg was not crosslinked to another hemoglobin molecule. Control of the peg-derivitization of wild-type hemoglobin was not possible because there is no site-directed labeling of the hemoglobin starting material.

In contrast, the combination of SGE 1.1 mono-cys starting material and a peg crosslinker should yield a substantially monodisperse dumbbell (pseudo-octameric) product. The site-direction of the crosslinker attachment site should result in precise control of the apparent molecular weight, which will depend on the size of the crosslinker. Moreover, careful control of the site of the cys mutation on the surface of the recombinant hemoglobin should ensure that the functionality of the derivatized hemoglobin is maintained.

### Higher Multimeric Hemoglobins

The above crosslinkers all involve the attachment of one tetramer at each end of a crosslinker. It may be advantageous to attach more than two tetramers to a single crosslinker to yield more oxygen-carrying capacity and to further increase the molecular weight.

A multimeric hemoglobin may be assembled with the aid of one or more linker peptides, each having a controlled number of reactive sites to which a cysteine residue of a hemoglobin tetramer or pseudotetramer may be attached, directly or indirectly.

With a peptide linker of considerable length, there is the concern that it will be degraded by serum proteases, thus degrading the multimeric hemoglobin into its component tetramers. This problem, if significant, may be remedied by use of a peptide linker which is less susceptible to proteolysis. A non-exhaustive list of such linkers would include peptides composed of D-amino acids, peptides with stable, extended, secondary structures, and branched peptides.

In the case of peptides composed of D-amino acids, use of D-Glu or D-Asp is particularly preferred.

A number of stable, extended, secondary structures are known. The simplest is possibly polyproline. Another example is the 2-stranded coiled coil, in which two peptide chains intertwine. A 4-helical or a 4-stranded coiled coil are also possiblities.

Branched structures, such as those obtained by derivation of the secondary amino group of lysine, are typically resistant to protease.

If desired, several of these approaches may be combined. For example, several coiled coils may lead off a branched structure, or D-amino acids may be incorporated into a coiled coil.

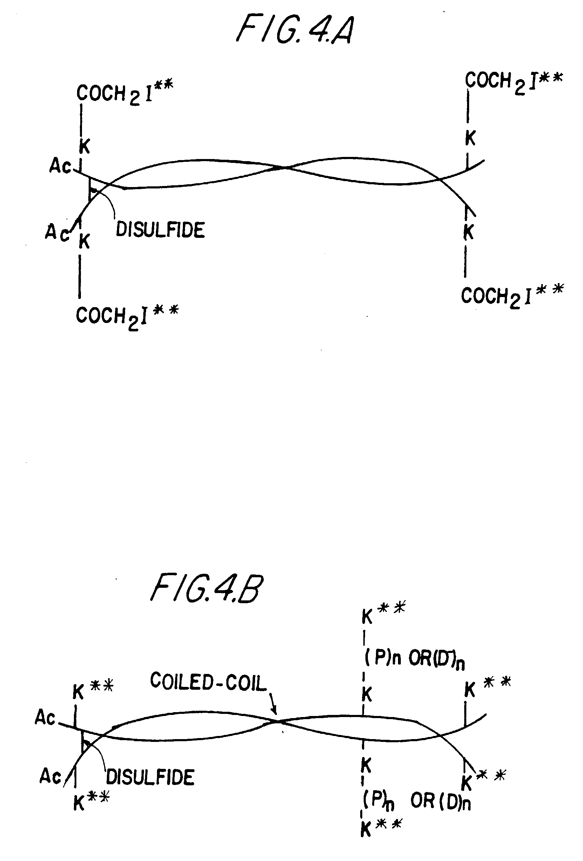

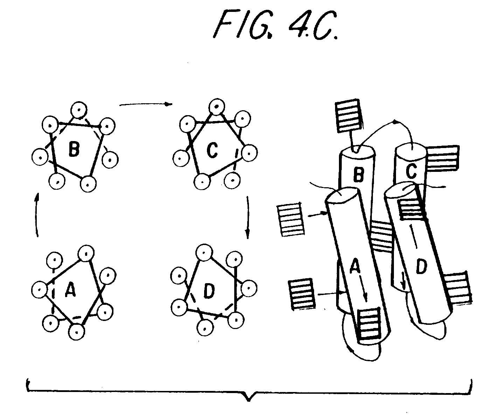

A hypothetical 4- tetramer coiled-coil linker complex is shown in Figure 4(a). Design and synthesis of these coiled coil peptides has already been explored (for an example see Cohen and Perry, Proteins, 7:1-15 (1990)). The rationale for a coiled coil is that two intertwined alpha helices will be less sensitive to proteolytic cleavage than a single naked secondary structure like an extended peptide (rapidly cleaved by proteases), an alpha helix or a beta sheet.

Using molecular modeling, an internal disulfide may be designed in the center of a bi-functional coiled coil linker such that the strands are covalently attached. This should stabilize formation of the correct coiled coil crosslinker before mono-cys di-alpha or di-beta Hgb (e.g., sge1.1 cys) is attached. Additionally, a tri-functional crosslinker can be stabilized by use of a orthogonallyprotected lysine (lys-FMOC) rather than a disulfide in the center of a proteolytically inert secondary structure. A polyproline helix can be used as the linker, and can be stabilized by branching the synthesis at the lys-FMOC after removal of the side chain. The three remaining lysines in the branched peptide would then be iodoacetylated to site-specifically attach a thiol-reactive group using either iodoacetic anhydride or N-succinimidyliodo- acetate and subsequently reacted with sge1.1-cys. An analogous tetra-functional crosslinker could be synthesized by inserting 1-2 prolines between two internal branching lysines to rotate them such that the two internal branching chains growing off the orthogonally protected lysines head in (nearly) opposite directions. Analogous structures could be made using D- glutamate(E) or D-aspartate(D) to provide protease resistance, and these would form an extended polyanionic chain at pH 7.

The sequence of a hypothetical alpha-helical coiled coil is modified from that given in Semchuck, et al., in Peptides: Chemistry, Structure and Biology; 566 (Rivier and Marshall, eds:1990), to leave only two lysines (K) at each end:

This coiled coil should have about 10 turns of a helix and thus will be ca. 54 Å long, allowing two tetramers to attach on each side without steric interference. The exact sequence and length to allow appropriate placement of 4 tetramers would depend on the results of molecular modeling.

Suggested trifunctional and tetrafunctional crosslinkers are diagrammed below.

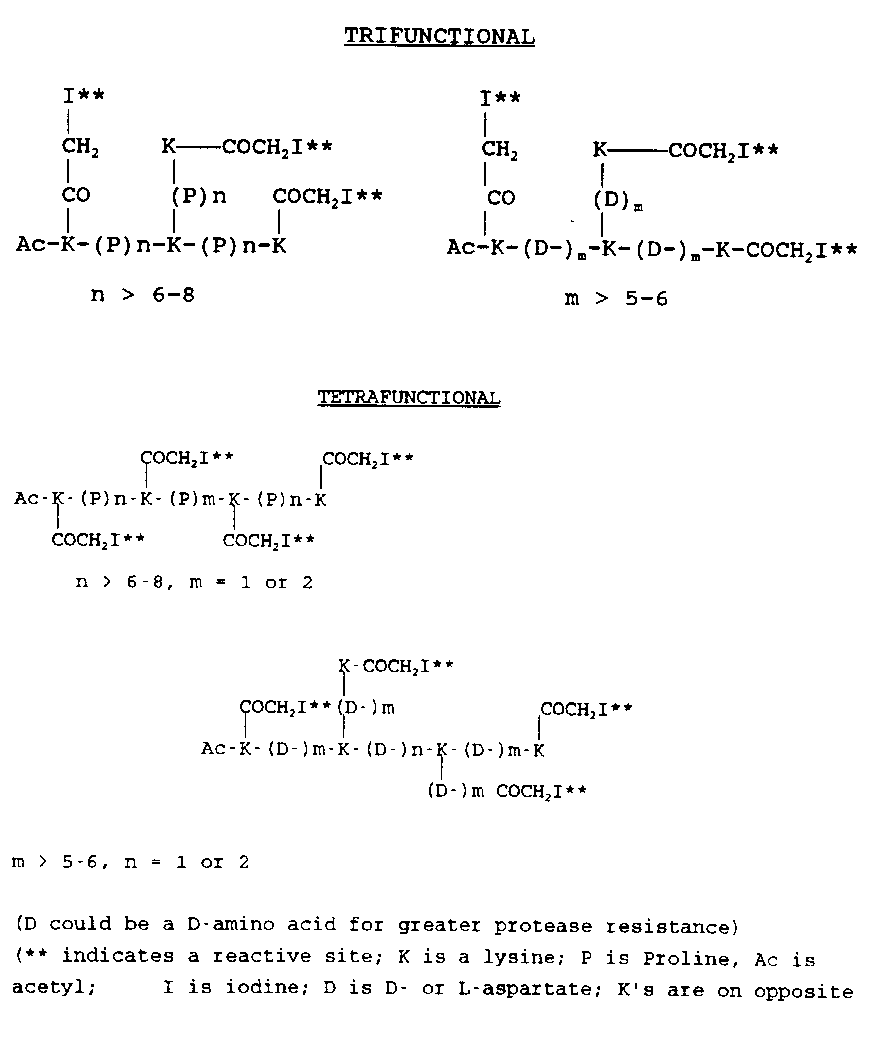

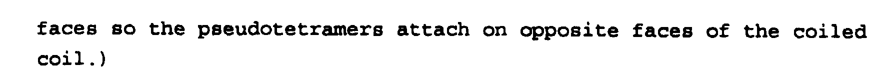

Another possibility is an 8-hemoglobin complex (Figure 4(b)). The rationale for considering this sort of complex is that it may be the way to obtain a very long half-life, due to the extreme stability of the "crosslinker" and the substantially higher molecular weight of the complex. The crosslinker might take the form of a doubly branched coiled coil, with a Lys(FMOC) replacing an Arg in the middle of the chain to allow the branching, and with a polyproline helix or other protease resistant secondary structure comprising the branching moiety. This structure could allow attachment of 6 SGE1.1's per crosslinker. Alternatively, a 4-helical bundle protein (See Figure 4(c)) or 4-stranded coiled-coil such as those synthesized by DeGrado, Science, 243:622 (1989), with each helix in the 4-helical bundle containing the consensus sequence GELEELLKKLKELLKG, (SEQ ID NO:9) the helices being linked by three PRR or RPR loops, could be utilized as a suitable core for the linker. This is one of the most stable proteins known, with a G = -22 kcal/mole separating the folded from the unfolded state. Each helix would be 4+ turns or ca. 22 Å long. Since this may not be enough room to fit two hemoglobins with one anchored at each end of the helix, they might have to be attached to different faces of the same helix, to lysines placed at each end of the polar face of each helix. Each helix is amphipathic; this should allow relative freedom to have a total of 8 lysines (and no more) and to change the remaining lysines to arginines. At least two of the i, i+4 salt bridges per helix would be retained for stability of the protein. Attachment of an externally crosslinkable cysteine-bearing tetramer could be via iodoacetylation of the lysine epsilon amino groups and then reaction with the thiol group of the cysteine.

An example of a modification that might allow more room between tetramers would be addition of one turn of the helix to the N-terminus of the A and C helices and the C- terminus of the B and D helices. This and similar modifications would be subject to modeling and experimental constraints.

Analogous core proteins could be made as mutants of known 4-helical bundle proteins such as myohemerythrin or apoferritin, with the surface residues changed so that 8 (or more if topologically possible) lysines (2 per helix) exist on the surface for subsequent modification and attachment of the tetramer.

### Poly(tetrameric) Hemoglobins with Reduced Isoelectric Points

If the isoelectric point of the whole crosslinked conjugate also affects the serum half life, via electrostatic exclusion from the renal filter's "pore", additional negative charges could be included in the crosslink itself (rather than in the hemoglobin, which could change the function of the molecule) to drop the isoelectric point of the overall crosslinked particle. An additional benefit of this might be reduced uptake by the reticuloendothelial system, this uptake being a function of pI for cationized albumin.

We have preliminary evidence from succinylation of SGE1.1 which correlates the number of modified lysines with isoelectric point. This gives a rough estimate of the number of lys to glu and/or lys to asp mutations which may be necessary to reach a pI of 5 or less, the pI range which we expect we need to significantly extend half life. We believe that as many as 8 lysines may have to be modified (a total shift in charge of 16 units) to drop the pI roughly 2 units. It should be less disruptive of the functional properties of hemoglobin to do this via a eptide crosslinker rather than by mutation of the alpha and beta globin subunits proper. However, some mutations could be made in the crosslinker and the remainder in the subunits. As before, the SGE1.1-cys would be attached to iodoacetylated lysine epsilon amino groups by reaction at pH 6.5-7.0.

For human serum albumin in the rat, the half life varied roughly linearly with the pI of the protein, from ca. 4.6 hours for native albumin (pI=4) to 0.8 hrs at a pI above 9.5. Clearance was probably by multiple mechanisms, including potentially increased uptake into the reticuloendothelial system with increased pI. For rat trypsinogens, the difference in serum half life between versions with a pI of 5.-0 (t_{1/2} of 4 min) was even larger. Thus a lower pI clearly appears to be an important variable in the serum half life of these proteins.

The following table gives examples of crosslinkers between mono-cys tetramers which should diminish the isoelectric point of the overall complex. 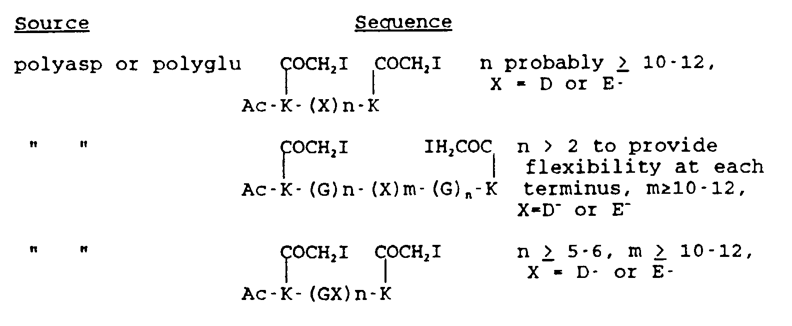 A number of the proposed crosslinkers could combine at least two, or possibly three of these attributes for potential additive effects.

It is possible that the unique amine groups in the peptide crosslinkers could be directly iodoacetylated during the peptide synthesis by treating iodoacetic acid as the last amino acid to be added, after deprotecting the lysine amine groups on the resin. In this case, the lysines would be orthogonally protected with N-FMOC or N-nitropyridinesulfenyl groups, or with BNPEOC. This could greatly simplify their synthesis.

Alternate methodologies to iodoacetylation as part of the synthesis could include the reaction of either sge1.1-SH or the peptide crosslinker with a heterobifunctional crosslinker specific for sulfhydryls and amines, such as sulfo-SMCC or similar reagents available from Pierce Chemical Co. (Rockford, IL).

### Gene Construction and Expression

The DNA sequences encoding the individual polypeptide chains may be of genomic, cDNA and synthetic origin, or a combination thereof. Since the genomic globin genes contains introns, genomic DNA must either be expressed in a host which can properly splice the premessenger RNA or modified by excising the introns. Use of an at least partially synthetic gene is preferable for several reasons. First, the codons encoding the desired amino acids may be selected with a view to providing unique or nearly unique restriction sites at convenient points in the sequence, thus facilitating rapid alteration of the sequence by cassette mutagenesis. Second, the codon selection may be made to optimize expression in a selected host. For codon preferences in E. coli, see Konigsberg, et al., PNAS, 80:687-91 (1983). Finally, secondary structures formed by the messenger RNA transcript may interfere with transcription or translation. If so, these secondary structures may be eliminated by altering the codon selections.

Of course, if a linker is used to genetically crosslink subunits, the linker will normally be encoded by a synthetic DNA.

The present invention is not limited to the use of any particular host cell, vector, or promoter. The host cell may be prokaryotic or eukaryotic, and, in the latter case, may be a plant, insect or mammalian (including human) cell. The cell may also be of any suitable tissue type, including, inter alia, an erythrocyte. However, the preferred host cells are bacterial (especially, E. coli) and yeast (especially S. cerevisiae) cells. The promoter selected must be functional in the desired host cells. It preferably is an inducible promoter which, upon induction, provides a high rate of transcription. A preferred bacterial promoter is the Tac promoter, a trp/lac hybrid described fully in DeBoer, U.S. 4,551,433 and commercially available from Pharmacia-LKB. Other promoters which might be used include the temperature sensitive lambda P_{L} and P_{R} promoters, as well as the lac, trp, trc, pIN (lipoprotein promoter and lac operator hybrid), gal and heat shock promoters. The promoter used need not be identical to any naturally-occurring promoter. Guidance for the design of promoters is provided by studies of promoter structure such as that of Harley and Reynolds, Nucleic Acids Res., 15:2343-61 (1987) and papers cited therein. The location of the promoter relative to the first structural gene may be optimized. See Roberts, et al., PNAS (USA), 76:760-4 (1979). The use of a single promoter is favored. Suitable yeast expression systems are described in detail elsewhere in this specification.

The vector used must be one having an origin of replication which is functional in the host cell. It desirably also has unique restriction sites for insertion of the globin genes and the desired regulatory elements and a conventional selectable marker. A vector may be modified to introduce or eliminate restriction sites to make it more suitable for futher manipulations.

The component polypeptide chains of the multimeric hemoglobin may be expressed either directly or as part of fusion proteins. When expressed as fusion proteins, the latter may include a site at which they may be cleaved to release the globin-related moiety free of extraneous polypeptide. If so, a site sensitive to the enzyme Factor Xa may be provided, as taught in Nagai and Thorgerson, EP B 161,937, incorporated by references herein. Alternatively, the fusion proteins may be synthesized, folded and heme incorporated to yield a hemoglobin analogue. The direct expression of the component polypeptides is desirable.

In bacterial mRNA, the site at which the ribosome binds to the messenger is a polypurine stretch which lies 4-7 bases upstream of the start (AUG) codon. The consensus sequence of this stretch is 5'...AGGAGG...3', and is frequently referred to as the Shine-Dalgarno sequence. Shine and Dalgarno, Nature, 254: 34 (1975). The exact distance between the SD sequence and the translational start codon, and the base sequence of this "spacer" region, affect the efficiency of translation and may be optimized empirically. Shepard, et al., DNA 1: 125 (1985); DeBoer, et al., DNA 2: 231 (1983); Hui, et al., EMBO J., 3: 623 (1984).

In addition, the SD sequence may itself be modified to alter expression. Hui and DeBoer, PNAS (USA), 84:4762-66 (1987). Comparative studies of ribosomal binding sites, such as the study of Scherer, et al., Nucleic Acids Res., 8:3895- 3907 (1907), may provide guidance as to suitable base changes. If the hemoglobin is to be expressed in a host other than E. coli, a ribosomal-binding site preferred by that host should be provided. Zaghbil and Doi, J. Bacteriol., 168:1033-35 (1986).

Any host may be used which recognizes the selected promoter and ribosomal binding site and which has the capability of synthesizing and incorporating heme. Bacterial and yeast hosts are preferred.

The intracellularly assembled hemoglobin may be recovered from the producing cells and purified by any art- recognized technique.

### Polycistronic Expression in Bacteria

While not required, it is desirable that the subunits, when expressed in bacteria, be co-expressed in the same cell, and it is still more preferable that they be co-expressed polycistronically. A polycistronic operon encodes a single messenger RNA transcript having one promoter sequence, but two or more pairs of start and stop codons that define distinctly translatable sequences. Each such sequence is known as a "cistron," and the polypeptides corresponding to the cistrons are thus co-expressed under the control of the single promoter.

The majority of bacterial operons are polycistronic, that is, several different genes are transcribed as a single message from their operons. Examples include the lactose operon with three linked genes (lacZ, lacY and lacA) and the tryptophan operon with five associated genes (trpE, trpD, trpC, trpB, and trpA). In these operons, the synthesis of messenger RNA is initiated at the promoter and, within the transcript, coding regions are separated by intercistronic regions of various lengths. (An operon is a cluster of genes that is controlled as a single transcriptional genetic unit). Translational efficiency varies from cistron to cistron. Kastelein, et al., Gene, 23: 245-54 (1983).

When intercistronic regions are longer than the span of the ribosome (about 35 bases), dissociation at the stop codon of one cistron is followed by independent initiation at the next cistron. With shorter intercistronic regions, or with overlapping cistrons, the 30S subunit of a terminating ribosome may fail to dissociate from the polycistronic mRNA, being instantly attracted to the next translational initiation site. Lewin, Gene Expression, 143-148 (John Wiley & Sons: 1977).

Unlike bacterial mRNAs, eukaroyotic mRNAs are generally monocistronic in nature. Lewin, Gene Expression, 157.

In one embodiment, expression of the genes encoding two or more component polypeptides are driven by a single promoter, and the genes are arranged so that a polycistronic messenger RNA transcript is transcribed, from which the separate globin-like polypeptides are subsequently translated. However, the present invention includes the co-expression of the different polypeptides from separate promoters, i.e., the host transcribes separate alpha and beta globin mRNAs.

Ideally, alpha and beta globin-like domains are expressed in stoichiometrically equal amounts. While use of a single promoter does not guarantee equality, it eliminates one unbalancing influence --differences in transcription owing to differences in promoter strength and accessibility. If differences in promoter strength were minimized by use of two identical promoters on the same plasmid, plasmid stability would be reduced as there would be a propensity toward recombination of the homologous regions.

Preferably, the alpha and beta globin-like domain-encoding genes are arranged so that the ribosome will translate the alpha globin cistrons first. The rationale is that there is some basis for believing that alpha globin affects the folding of beta globin. Nonetheless, the position of the genes may be switched so that a beta globin-like domain is synthesized first.

The stability of the polycistronic mRNA transcript, the efficacy of its translation into alpha and beta globin-like polypeptides, and the folding of the globin chains into tetrameric hemoglobin may be modified by varying the length and base sequence of the intercistronic regions (the region lying between the stop codon of one cistron and the start codon of the next cistron), the phasing of a second cistron relative to a first cistron, and the position and sequence of the ribosomal binding site for the one cistron relative to the preceding cistron.

In a preferred embodiment, the alpha and beta globin-like polypeptides genes are each preceded by a short "introductory" cistron or "ribosomal loader" which facilities the subsequent translation of the globin cistron. In Figure 2, region A contains two cistrons and a Shine-Delgarno sequence preceeding each cistron. The first Shine-Delgarno sequence (SD#1) is bound by the ribosome, which then translates the first cistron, a short cistron encoding an octapeptide. (This cistron is referred to as an "introductory cistron or ribosomal loader.) The second cistron is a globin gene, in this case, an FX alpha-globin gene. The Shine-Delgarno sequence (SD#2) for facilitating translation of the second cistron actually lies within the first cistron. For this reason, the two are said to be "translationally coupled". Region B is identical in structure, except that the second cistron encodes FX-beta globin. Between regions A and B is a 43-base intercistronic region. The introductory cistrons of regions A and B correspond to the first cistron of the two-cistron expression system denoted pCZ144 in Schoner, et al., Meth. Enzymol., 153: 401-16 (1987). The present invention is not, however, limited to the particular "starter" cistron taught by Schoner, et al.; other introductory cistrons that allow for restart of high level translation of a following cistron may be used.

Guidance as to the design of intercistronic sequences and as to the location of SD sequences may be obtained by comparing the translational efficiency of spontaneous or controlled mutants of the same polycistronic operon, as exemplified by Schoner, et al., PNAS, 83: 8506-10 (1980). It is also possible to look for consensus features in the intercistronic regions of different operons. McCarthy, et al., EMBO J., 4: 519-26 (1985) have identified a translation- enhancing intercistronic sequence in the E. coli atp operon.

The present invention is intended to reduce or avoid the localization of the hemoglobin or its component polypeptides into inclusion bodies. Consequently, a further feature of the invention is that the functional hemoglobin is substantially found (preferably over 80%) in the soluble fraction of the cell. It appears that with this invention, over 90% of the functional hemoglobin can be so directed when alpha₂ beta₂ hemoglobin is assembled from alpha- and beta- globin chains co-expressed from a tetracistronic operon as described herein. With di-alpha, beta₂ hemoglobin, nearly 100% is soluble when expression is induced at 25°C and less at higher induction temperatures. These percentages reflect the percent of all di-alpha and beta chains found in the soluble fraction of the cell and not actual recovery of protein from the cell.

### Expression in Yeast

In another embodiment the present invention relates to the production of hemoglobin-like molecules in yeast. Our preferred host for expression in yeast is Saccharomyces cerevisiae. However, other fungi or yeast may be used for the purpose, such as strains of Aspergillus or Pichia. For yeast to be a suitable host it must be capable of being transformed with recombinant vectors, either replicating or integrating types. This allows the insertion of the desired DNA sequence for the gene of interest. It must also be capable of high density cell growth, in appropriate volume to provide sufficient cell mass to isolate the desired gene product from the desired reaction vessels, where ideally the growth would be easily controlled by several parameters including nutrient formulation, agitation and oxygen transfer and temperature. It is also desirable to be able to induce the expression of protein synthesis with the manipulation of the media, temperature, or by the addition or consumption of certain chemicals. Finally, to be a suitable host, the yeast must be capable of producing recombinant proteins, preferably in excess of 1% of the total cell protein. This allows more facile isolation of the desired recombinant protein.

Either a haploid or a diploid strain of S. cerevisiae may be used. For example, the following diploid strains are preferred:
BJY3505 x RSY330
BJY3505 x BJY 1991

Other matings may likewise be used in practicing the present invention.

The use of protease-deficient strains may also be advantageous.

Yeast expression systems can be divided into two main categories: (1) Systems designed to secrete protein and (2) system designed for the cytoplasmic expression of proteins. At present, cytoplasmic expression is preferred since the yeast cells fold together the globin chains and incorporate heme to produce hemoglobin in vivo. However, it is possible to separately express and secrete the alpha and beta globin chains and assemble hemoglobin in vitro.

The globin genes must be placed under the control of a suitable promoter. The commonly used yeast promoters generally fall into two broad categories: regulated and constitutive. Constitutive promoters that are in wide use include GAP, PGK (phosphoglycerate kinase) and the α-factor promoter. Regulated promoters have also been used and these include the yeast metallothionein promoter (regulated by copper), the Gall-10 promoter, GAL7 promoter (regulated by galactose and glucose) the ADHII promoter (regulated by ethanol and glucose) the PH05 promoter (phosphate regulation) and several hybrid promoters such as PH05-GAP, GAL-PGK, ADHII-GAP, and GAL-CYC1.

The use of a GAL-GAP hybrid promoter is preferred. Both elements (the GAL_{UAS} and the GAP transcriptional initiation site) are well understood. Studies on the mechanisms of transcriptional regulation of the GAL regulon have been fairly extensive. The galactose regulon includes five genes that encode enzymes required for the utilization of galactose. Four of these genes (GAL1, GAL7, GAL10, and GAL2) are expressed only in the presence of galactose. Galactose induction does not occur in the presence of glucose unless the yeast strain bears a mutation in the REG1 gene. The GAL1, 7, 10 and 2 genes are regulated by at least two other genes, GAL80 and GAL4. The GAL4 gene is a transcriptional activator protein that activates mRNA synthesis from the GAL1, 7, 10 and 2 upstream activator sequences (UAS_{GAL}). Although GAL4 is constitutively expressed, it is functionally silent in the absence of galactose. Repression of GAL4 activity, in the absence of galactose is maintained by the product of the GAL80 gene. The GAL80 protein apparently interacts physically with GAL4 to prevent transcriptional activation. Presumably galactose or a galactose derivative prevents this interaction to allow GAL4 mediated induction.

Haploid strains of S.cerevisiae have three different genes encoding the enzyme glyceraldehyde-3-phosphate dehydrogenase (GAP). These genes have been designated TDH1, TDH2 and TDH3 and each is present as a single copy per haploid genome. The TDH3 gene produces approximately 60% of the cell's GAP enzyme and TDH1 and 2 produce about 12% and 28%, respectively (McAllister, L and M.J. Holland, 1985. J. Biol Chem, 260: 15019-15027). Holland's group (Holland et al. 1981. J. Biol Chem, 256:1385-1395; and Holland et al. 1983. J Biol Chem 258:5291-5299) has cloned and characterized the three GAP genes of S.cerevisiae. The clones have been designated pGAP11, pGAP63, and pGAP491. pGAP491 corresponds to the TDH3 gene and is therefore, the most highly expressed.

This promoter is commonly used as a 600-850bp fragment and is essentially un-regulated. In its long form this is a very powerful promoter. The form we are using consists of only ^{~}200bp 5' of the translational initiation site. This form, with no added enhancer sequences is substantially less active than the longer form of the promoter (Edens, L. et al. Cell, 37:629 (1984)). Our addition of the GAL enhancer region confers both regulation and high levels of expression. With only the GAP491 promoter, alpha and beta globin were produced at a level of less than 0.2% total cell protein; with the GAL-GAP491 hybrid promoter, expression jumped to 7-10% total cell protein.

Several other hybrid promoters are of particular interest: GAL-SIGMA; SIGMA-GAP; GAL-EF III; SIGMA-EF III.

One could easily conceive of other promoter systems that would also work. This would include, but not be limited to, a variety of constitutive promoters. For example, the yeast mating factorα (MFα) promoter or the mating factor a promoter MF(a), the phosphoglycerate kinase promoter (PGK), hexokinasel, hexokinase2, glucokinase, pyruvate kinase, triose phosphate isomerase, phosphoglycerate isomerase, phosphoglycerate mutase, phosphofructose kinase or aldolase promoters may all be used. In short, any well expressed yeast promoter may work for expression of hemoglobin in yeast. A wide variety of naturally occurring, regulated promoters could also be used, for example: GAL1-10, GAL7, PHO5, ADHII have all been used to produce heterologous proteins in yeast. A variety of synthetic or semi-synthetic yeast promoters could also be employed such as GAL-PGK, GAL-MFα-1, GAL-MFal, GAL-SIGMA. ADHII regulatory sequences could also be coupled to strong transcriptional initiation sites derived from a variety of promoters. The PHO5 regulatory sequence or the sigma element regulatory sequences could also be used to construct powerful hybrid promoters. In addition to yeast promoters, it is conceivable that one could use a powerful prokaryotic promoter like the T7 promoter. In this case, one could place the T7 polymerase under the control of a tightly regulated yeast promoter. Induction of the phage polymerase in yeast cells bearing hemoglobin genes under T7 promoter regulation would allow transcription of the genes by this very efficient phage polymerase.

Because most of the yeast regulatory sequences described above serve as targets for proteins that are positive regulators of transcription, it is conceivable that these proteins may limit transcription in situations where the target sequence is present in many copies. Such a situation may obtain with vectors such as pC1B, pCIT, pClU or pC1N which may be present in excess of 200 copies per cell. Over-expression of the positive regulator (for example GAL4) may result in enhanced expression. It is possible to construct a strain in which the GAL4 gene is altered to remove its promoter and the promoter replaced with the GAL7 or GAL1-10 promoters, both of which are transcribed more efficiently than the GAL4 promoter. In this situation, the positive transcriptional activator protein GAL4 would be expressed at elevated level at the time hemoglobin expression was induced.

The consensus sequence for higher eukaryotic ribosome binding sites has been defined by Kozack (Cell, 44:283-292 (1986)) to be: G^{AA}_{G}CCAUGG (SEQ ID NO:10). Deviations from this sequences, particularly at the -3 position (A or G), have a large effect on translation of a particular mRNA. Virtually all highly expressed mammalian genes use this sequence. Highly expressed yeast mRNAs, on the other hand, differ from this sequence and instead use the sequence AAAAAUGU (Cigan and Donahue, Gene, 59:1-18 (1987)). The ribosome binding site that we use for expression of the α and β-globins corresponds to the higher eukaryotic ribosome binding site. It is within the contemplation of this invention to systematically alter this RBS to test the effects of changes that make it more closely resemble the RBS of yeast. It should be pointed out, however, that alterations at the -2, -1 and +3 positions, in general, have been found to only slightly affect translational efficiency in yeast and in mammals.

Intracellular expression of genes in S. cerevisiae is primarily affected by the strength of the promoter associated with the gene, the plasmid copy number (for plasmid-borne genes), the transcription terminator, the host strain, and the codon preference pattern of the gene. When secretion of the gene product is desired, the secretion leader sequence becomes significant. It should be noted that with multicopy plasmids, secretion efficiency may be reduced by strong promoter constructions. Ernst, DNA 5:483-491 (1986).

A variety of extrachromosomally replicating vectors (plasmids) are available for transforming yeast cells. The most useful multicopy extrachromosomal yeast vectors are shuttle vectors that use a full length 2µ-circle combined with an E. coli plasmid. These vectors carry genes that allows one to maintain the plasmid in appropriate yeast mutants and antibiotic resistance markers that allow selection in E. coli. Use of the full-length 2µ-circle, in contrast to vectors containing only a partial 2µ sequence, generally results in much higher plasmid stability, particularly in yeast strains that have been cured of endogenous 2µ plasmid. The pC series of vectors described herein are vectors of this type.

Strains could also be constructed in such a way that the GALGAP hemoglobin expression cassettes were integrated into chromosomes by using yeast integrating vectors. Although the copy number of the hemoglobin genes would be lower than for plasmid vectors, they would be quite stable and perhaps not require selection to be maintained in the host cell. Yeast integrating vectors include Yip5 (Struhl, et al, PNAS, 76:1035- 39, 1989), Yipl (Id.), and pGT6 (Tchumper and Carbon, Gene, 10:157-166, 1980). For information on these and other yeast vectors, see Pouwels, et al., Cloning Vector, VI-I, et seg. (Elsevier, 1985).

The genes encoding the desired globin-like domains may be introduced by separate plasmids, or both upon the same plasmid.

Highly expressed yeast genes show a very high codon bias. The genes encoding glyceraldehyde-3-phosphate dehydrogenase and ADH-I, for example, show a 90% bias for a set of 25 codons. Highly expressed yeast genes (>1% of the total mRNA) have yeast codon bias indices of >.90. Moderately expressed genes (0.1-.05% of the total mRNA) have bias indices of 0.6-0.8, and genes expressed at low levels (>0.05% of the total cell protein) have a codon bias of 0.10-0.50 (Bennetzen and Hall, J. Biol. Chem., 257:3026-3031 (1982)). The calculated value for the codons of the human α-globin cDNA is 0.23. A similar value can be calculated for the β-globin cDNA. Because there is a very high correlation between the most commonly used codons, it is possible that hemoglobin expression from the human cDNA in yeast may be limited by the availability of the appropriate tRNA molecules. If this is so, a complete synthesis of the gene using the most highly favored yeast codons could improve the expression levels. It is quite possible that the greatest negative effect of adverse codon use would be if there was an abundance of codons used in the cDNA that are represented by low abundance tRNAs. In such a case, high level expression of hemoglobin could completely drain that pool of tRNA molecules, reducing translation not only of hemoglobin but of yeast proteins that happen to use that codon as well. In the case of the α-globin human cDNA, the most commonly used leucine codon is CTG (14 of 21), this codon is never used in highly expressed yeast genes (Guthrie and Abelson, The Molecular Biology of the Yeast Saccharomyces, Eds. Stratern, Jones and Broach, 1982. Cold Spring Harbor, NY). The low codon bias index and the presence of rare yeast codons in the globin cDNAs have been sufficient incentive for us to synthesize a modified form of the alpha- and beta-globin genes using the preferred yeast codons.

### Miscellaneous

The appended claims are incorporated as a further enumeration of the preferred embodiments.

Preparation of expression vectors suitable for use in production of the claimed multimeric hemoglobins may be facilitated by the Budapest Treaty deposit of the following vectors, all made with American Type Culture Collection, Rockville, Maryland USA on May 10, 1990:

### ATCC 68323 pDL III-14c

This is a derivative of pKK223-3 (Parmacia LKB, Piscataway, New Jersey, UDA) and pGEM1 (PromeguCorp., Madison, Wisconsin, USA) which carries synthetic genes for des-Val alpha globin and des-Val beta globin as part of a polycistronic operon driven by a single Tac promoter.

### ATCC 68324 pDL IV-8a

This is a derivative of PDL III-14c which contains a fused gene encoding an alpha globin moiety, a glycine, and a second alpha globin moiety, as well as a second gene encoding des-Val beta globin.

### ATCC 20992 pGS 389

This is a yeast vector which expresses alpha and beta globin under control of GALGAP promoters.

The deposit of these vectors should not be construed as a license to make, use or sell them.

### Example 1:

### Construction of di-α globin mono-cysteine (A71C, D75C, or S81C) mutant expression vector

The following plasmids, whose preparation is fully described in Hoffman, et al., W088/09179, were manipulated in this Example.

### Plasmid pDL II-83a

A gene encoding a Met initiation codon, a Factor X site (Ile-Glu-Gly-Arg)(SEQ ID NO:11), and human alpha globin, collectively referred to as FX-A, was synthesized and cloned into the XmaI/PstI sites of M13mp19. The EcoRI-PstI fragment bearing the FX-A gene was excised and recloned into pKK 223-3, placing it under control of the Tac promoter. This derivative was called pDLII-62m. The FX-A gene was removed from pDLII-62m and ligated with EcoRI/PstI linearized pGEM1, forming pGEM-FX-A. This was digested with NdeI and EaqI, removing the Factor Xa coding sequence (and part of the α globin coding sequence). The excised fragment was replaced by a synthetic oligonucleotide which restored the missing α globin codons; the resulting plasmid was named pDLII-83a. The protein expressed was "Met-Val-Leu-...."

*Plasmid pDLII-91f,* in which the gene encodes "Met Leu..." instead of "Met-Val-Leu", was likewise constructed from pGEM-FX-A, but with a different synthetic replacement oligonucleotide, missing the Val codon.

### Plasmid pSGE 1.1 E4

*This plasmid* (also known as SGE1.1) is depicted in Figure 1. Plasmid pDLIV-8A may be converted to SGE1.1E4 by the following protocol.

The expression plasmid pDLIV-8a contains the dialpha coding sequences, in which the alpha globins are linked by a single glycine codon, and the des-val beta globin genes, under the control of a single Ptac promoter. The plasmid encodes ampicillin resistance, does not have a functional tetracycline resistance gene, and is Rop+ and Lac. Oligonucleotide directed site specific mutagenesis using a commercially available kit such as DoubleTake™ (Stratagene, Inc.) can be used to insert the Presbyterian mutation into the beta globin sequence.

The final expression plasmid, SGE 1.1E4 (amp R, tet R, Rop-, Lac+) is then constructed by insertion of both a functional tetracycline resistance gene and the lacl gene which encodes the lac repressor protein that inhibits the Ptac promoter until induction with an inducing agent. These modifications are described below.

The initial modification to the plasmid is the insertion of the lacI gene. This gene can be synthesized by polymerase chain reaction (PCR) amplification, i according to the manufacturer's protocol (Perkin Elmer Cetus, Norwalk, CT) using the F episome of E. coli strain JM109 (FtraD36, proAB, lacI¶ΔM15) as a substrate. The following oligonucleotides can be used as primers:

The primers contain, at their 5' ends, sequences which encode for NotI restriction enzyme sites. The product of the PCR amplification reaction can be blunt ended and cloned into the PuvII site of the expression plasmid. The PuvII site is not reconstructed during this cloning step, so digestion with PuvII following ligation will linearize plasmids which do not incorporate the lacI sequence. Linearized plasmids will not transform E. coli. Because the primers are complementary only to the translated portion of the lacI gene, this fragment does not contain its own promoter. Note that inducibility or expression of hemoglobin is dependent on the orientation of the lacI gene, thus orientation should be checked after insertion of the lacI gene. The correct orientation has a smaller EcoR5 fragment than the incorrect orientation. Moreover, insertion of the lacI repressor gene into the PuvII restriction site inactivates the rop gene product and results in increased plasmid copy number.

The final modification to the plasmid is restoration of a functional tetracycline resistance gene. This can be accomplished by digestion of commercially available pBR322 with EcoRI followed by insertion via ligation of a synthetic DNA linker containing the 5' and 3' ends complementary to the EcoRI overhangs and na internal BamHI site. The BamHI fragment from this modified pBR322 vector containing the 5' coding sequence of the tetracycline resistance gene is purified by agarose gel electrophoresis, then inserted into the BamHI site of the modified pDL IV-8a plasmid. Only one orientation of the tet^{R} fragment results in tetracycline resistance; strains can be screened for the proper orientation by growth on the appropriate medium. Insertion of the ter^{R} fragment into the modified vector restores tetracycline resistance and produces SGE1.1E4.

### Plasmid pGEM di-alpha.

The di-alpha gene-bearing SmaI/PstI fragment of SGE 1.1 E4 was ligated with SmaI/PstI-cut pGEM 1 to form pGEM di-alpha.

### 1.1 Subcloning of the α gene into phagescript

The desfxα pGem (pDLII-83a) vector was cut with EcoR1 and Pstl endonucleases and ligated into EcoR1/Pstl digested phagescript (obtained from Stratagene). E. coli strain DH5α was transformed with the ligation mixture and cells were plated on 2xYT plates overlaid with 3 ml top agar containing 10 µ1 100 mM IPTG, 25 µ1 2% X-Gal in DMSO and 150 µ1 XL-1 cells (Stratagene). Clear plaques were picked and grown at 37°C in 2xYT containing XL-1 cells. Double stranded DNA was isolated from the cultures and checked for the presence of the 500 bp α gene by restriction analysis and agarose gel electrophoresis. Single stranded DNA was isolated from one of the desfxα phagescript transformants (named f191). The single stranded DNA was sequenced to confirm the presence of the desfxα gene in the phagescript.

### 1.2 Mutagenic Oligonucleotides

Three mutagenic oligonucleotides were used in three separate mutagenic reactions. The sequences of the oligonucleotides were as follows (mutant codon is underlined):

### 1.3 Kinase reaction conditions for mutagenic oligonucleotides A71C, D75C and S81C.

1 µl oligonucleotide (approx. 300 pmol)
2 µl 10x kinase buffer containing 10 mM ATP
0.5 µl T4 polynucleotide kinase (10 U/µl, New England Biolabs)
15.5 µl H₂O
1 µ1 10 mM spermidine
Reactions were incubated for 1 hr. at 37°C, then 80 µl H₂O was added and the reaction was terminated by heat inactivation at 65°C for 10 min.

### 1.4 Mutagenesis Reaction

1 µl fw 191 ss DNA (0.5 pmol)
3 µl kinased oligonucleotide (either A71C, D75C or S81C approx. 45 pmol)
2 µl 10x annealing buffer (Promega)
14 µl H₂O

The no primer control contained:
1 µl fw 191 ss DNA
2 µl 10x annealing buffer
17 µl H₂O
Reactions were heated to 65°C, cooled slowly to 35°C (approx. 70 min), and put on ice for 5 min. The following reagents were added and the reactions were incubated at 37°C for 90 min.
3 µl 10x synthesis buffer (Promega)
1 µl T4 gene 32 protein (0.5 Mg/µl, Biorad)
1 µl T4 DNA polymerase (3 U/µl, NEB)
0.5 µl T4 DNA ligase (10U/µl, NEB)
5 µl H₂O
200 µl 71-18 mut S competent cells (made according to Promega Altered Sites procedure) were transformed with 10 µl of each mutagenesis reaction, put on ice for 30 min and heat shocked for 2 min at 42°C. The transformation mixture was added to 3 ml 2xYT media and grown at 37°C (with shaking) for 5.5 hr. After incubation, 1 ml of each of the three cultures was removed, centrifuged and 800 µl was stored in a fresh tube at 4°C as the stock solution of mutant phage.

### 1.5 Screening for mutants of D75C

100 µl of a 10⁻⁵ dilution of the D75C mutant phage stock was plated on 153 mm 2xYT plates overlain with top agar containing 0.5 ml XL-1 cells. Plates were incubated at 37°C for approx. 5 hrs. Duplicate nitrocellulose filters were lifted off each plate and the plaques were lysed in 6 ml 0.5 M NaOH/1.5 M NaCl, neutralized in 10 ml 1 M Tris-HCl pH 8.0/1.5 M NaCl and washed in 500 ml 6xSSC. The filters were air dried and baked at 75°C for 45 min. The filters were then boiled briefly in 1% SDS prior to prehybridization. Filters were prehybridized in 20 ml solution for 4 hr at 68°C. The prehybridization solution was as follows:
5xSSC (20x SSC prepared according to recipe in Maniatis).
0.1% (w/v) N-lauroylsarcosine
0.02% (w/v) SDS
0.5% blocking reagent (Genius Kit, Boehringer Mannheim)
The D75C oligonucleotide was labelled with π^{[32]} ATP as follows:
1 µl oligonucleotide (80 pmol)
10 µl 10x kinase buffer
1 µl π^{[32]}P ATP (10 µC/µl. Specific activity>3000 Ci/mmol).
87 µl H₂O
1 µl kinase (10U/µl, NEB)
The reaction was incubated for 5 hrs. at 37°C. Unincorporated ATP was removed by centrifugation through a Biospin 30 column (Biorad). The entire probe (17,000 cpm/µl) was added to the prehybridization mixture and the filters were hybridized overnight at 46°C along with a no primer control filter. The following day, filters were washed for 10 min. at room temperature (RT) in 6xSSC and exposed overnight at -70°C on Kodak X-Omat film. Filters were washed in 6xSSC at 57°C for 10 min, dried and exposed overnight, then washed in 6xSSC/0.1% SDS at 67°C for 10 min, and dried and re-exposed overnight. The final was was in 6xSSC/0.1% SDS at 70°C for 10 min and the filters were again dried and exposed overnight.

Ten plaques were picked which hybridized differentially to the mutant oligonucleotide (compared to the no primer control plaques). The plaques were placed in 5 ml 2xYT media containing 0.25 ml XL-1 cells. The cultures were incubated with shaking at 37°C for 7.5 hr. 1 ml of each culture was removed, centrifuged 5 min, placed in a fresh tube and stored at 4°C for subsequent sequencing and plaque purification.

### 1.6 Screening for mutants of A71C and 881C

1 µl of a 10⁻³ dilution of the A71C stock phage mutagenesis reaction and 20 µl of a 10⁻⁵ dilution of the S81C mutagenesis reaction were plated on four separate 82 mm 2xYT/tet(10mg/ml) plates overlaid with 3 ml top agar and 100 µl XL-1 cells. A no primer control was also plated as above. The plates were incubated at 37°C for 5 hr; plaques were lifted from each plate onto nitrocellulose filters and the filters dried overnight at room temperature. The following day, the plaques were lysed with 0.5 M NaOH/1.5 M NaCl for 3 min, neutralized in 1 M Tris- HCl pH 7.0/1.5 M NaCl for 3 min and washed in 6xSSC for 5 min. Filters were air dried then baked at 75°C for 1 hr. The filters were boiled briefly in 1.5% SDS prior to prehybridization at 60°C for 6 hr. in 10 ml prehybridization solution as described above.

### 1.7 Labelling of A71C and S81C oligonucleotides using digoxigenin

(All reagents supplied by Boehringer Mannheim)
2 µl A71C (100 pmol) or 1 µl S81C (110 pmol)
10 µl terminal transferase buffer
5 µl 25 mM CoCl₂
1 µl 1 mM dUTP-digoxigenin
30 µl 04 31 µl H₂O (A71C and S81C reactions respectively)
1 µl terminal transferase (25U/µl)
Reactions were incubated at 37°C for 3 hr. followed by 6 hr. at RT. Digoxigenin-labelled A71C and S81C probes (20 µl) were added to the appropriate filters in 10 ml prehybridization solution along with a no primer control filter. The filters were hybridized overnight at 47°C.

### 1.8 Filter Washes and Development

All filters were initially washed in 6xSSC/0.1% SDS for 15 min at 30°C, then for 15 min at 42°C.
Each of the four filters which had been probed with either the labelled A71C or S81C oligonucleotides were then separated and washed at increasingly higher temperatures along with a no primer control filter. One each of the A71C and S81C filters were placed in plastic bags containing 10 ml of 6xSSC/0.1% SDS and washed for 10 min at one of four temperatures, i.e., 50°C, 60°C or 65°C. After the high temperature washes, each set of filters were developed according to the Genius Kit protocol.

Initially, bags containing the filters were filled with 10 ml of 100 mM Tris-HCl, pH 7.5/150 mM NaCl (buffer A) and incubated for 15 min. The buffer was removed and replaced with 10 ml buffer A containing 0.5% blocking reagent and incubated a further 15 min at RT without shaking. Anti-digoxigenin antibody (2 µl) was added directly to each bag and incubated with for 30 min at RT. The filters were then removed from their respective bags and washed altogether in 100 ml buffer A/0.05% blocking reagent for 15 min at RT, followed by a 15 min wash in buffer A alone at RT. The final wash was 100 ml 100 mM Tris-HCl, pH 9.5/100 mM NaCl/50 mM MgCl₂ (buffer B) for 5 min at RT. Each set of filters from a given temperature was placed in a separate bag along with 5 ml of color development solution (5 ml buffer B containing 22.5 µl 75 mg/ml NBT/15 µl 50 mg/ml X-phosphate). The filters were incubated for 30 min in the dark at RT. After 30 min, the filters were removed from the development solution, washed for 5 min in 100 ml 10xTE and 5 min in 100 ml 1x TE, both at RT. Filters were dried at RT.
Using the results from the Genius Kit screening procedure, 10 plaques which differentially hybridized to the labelled oligonucleotides A71C or S81C were picked and placed in ml 2xYT media containing 0.25 ml XL-1 cells and incubated for 7.5 hr. at 37°C with shaking. 1 ml of each culture was removed, centrifuged 5 min, placed in a fresh tube and stored at 4°C for subsequent sequencing and plaque purification.

### 1.9 Confirmation of mutations by sequencing

Single stranded DNA was isolated from 800 µl mutant phage stock supernatant and sequenced using the Sequenase kit (USB) with the α 179 oligonucleotide as the primer. The α 179 aligonucleotide is an 18-mer hemolog cys to a region about 100 bps upstream of the mutation site.) Sequencing confirmed the presence of the αA71C, αD75C and α S81C mutations.
Phage stock was plaque purified by plating 10 µl of 10⁻⁸ and 10⁻¹⁰ dilutions on 2xYT/tet (10 mg/ml) plates overlaid with 3 ml top agar containing 200 µl XL-1 cells. After 7 hrs incubation at 37°C, a single isolated plaque from each mutant plate was picked and used to inoculate 90 ml 2xYT/tet (10 mg/ml) media containing 10 ml XL-1 cells. Cultures were grown overnight at 37°C with shaking. 1 ml of each mutant phage culture was removed, centrifuged and the supernatant was frozen at -80°C as the respective purified mutant phage stock. Double stranded DNA was prepared from the remaining culture for use in the subsequent subcloning steps into the final expression vector 1.1E4.

### 1.10 Subcloning of the α cys mutants into 1.1E4

Construction of the di-α gene with each of the three cysteine mutations in either the N-terminal or C-terminal domain of the di-α protein required three subcloning steps:
1) Transfer of the cys mutant α gene from phagescript as an Eagl-Pstl fragment into the Eagl-Pstl digested desval α pGem vector (pDL II-91f). This step provided the mutant α gene with the correct 5' terminus.
2) A mutant di-α gene with each of the cys mutations in the 3' α gene was constructed by inserting the Eagl DNA fragment from di-α pGem (pGem di-alpha) into the Eagl site of the relevant cys mutant desval α pGem plasmid. The mutant diα gene with the cys mutation in the 5' α gene was constructed by inserting the BstB1 DNA fragment from diα pGem into the BstB1 site of the cys mutant desval α pGem plasmid.
3) Finally each of the mutant diα genes were cloned into the 1.1E4 expression vector as a Sma1-Pst1 fragment.

Transformations into DH5α at each step in the subcloning procedure were carried out as described in the methods of subcloning of the β G83C mutation into 1.1E4 (see below). The presence of the relevant cys mutation in the correct α gene was confirmed by sequencing at each stage in the subcloning procedure. Each of the diα cys mutants in 1.1E4 were transformed into E. coli strain 127, grown in TB complete media and induced with IPTG. Expression of the diα and β proteins was confirmed by SDS-PAGE and Western blot analysis.

### Example 2: Protocol for the oxidation of two SGE1.1 monocys's to form a pseudo-octamer

The hemoglobin mutants of interest were expressed in E. coli grown in standard fermentation broth, after induction with IPTG. The cells were pelleted, resuspended in 3 mols of 40 mM Tris-base, 2mM benzamidine/gm cell paste, lysed by two passes through a Microfluidizer™ (Microfluidics, Inc.). The lysate was centrifuged to remove cell debris, and the supernatant was collected. The tetrameric hemoglobin was purified from the supernatant using the methodology described in Mathews, et al. (Methods in Enzymology, in press). Briefly, the supernatant is diluted with 5 mM Tris, pH 7.0 until the conductivity reaches 200 µmhos. The solution is then passed through a flow-through bed of Q Sepharose equilibrated with 20mM Tris, pH 7.0. The flow through is collected, the pH of the solution is brought up to 8.0 using concentrated NaOH, and then loaded onto a second Q Sepharose column equilibrated in 20mM Tris, pH 8.0. The hemoglobin captured on the column is washed with 2 column volumes of 20 mM Tris, pH 8.0, and eluted with 20mM Tris, pH 7.0. Fractions are collected and pooled on the basis of absorbance ratios (A₅₇₅/A₅₄₀>1.03). The solution is then buffer exchanged using a MinitanTM (Millipore Corp.) into 14 mM sodium phosphate, pH 8-8.5/150 mM approximately 100 mg/ml. Note that at this point in the purification, some of the mutant hemoglobin tetramers have already formed significant levels of octamer (>20%) by simple air oxidation of the cysteines engineered into the molecules. However, to drive oxidation of the remaining cysteine thiols to disulfides, the solution is incubated under air or oxygen for approximately 48 hours at 4°C.

After incubation, the octamers are separated from any unreacted monomer and any higher order polymers using Sepharcryl S-200 equilibrated in 14 mM sodium phosphate, pH 8-8.5/150 mM NaCl at a linear flow rate of 60 cm/hr. Fractions are collected and poolings are verified using an analytical grade Superose 12 column.

For those hemoglobin mutants where a cysteine replacement is located in close proximity to a negative charge, or for which disulfide formation is otherwise incomplete, the above procedure is preferably modified to enhance disulfide formation. After concentration to 50 mg/ml using the procedure described above, the hemoglobin solution is converted to carbon monoxy hemoglobin by gentle bubbling with CO. Five Cu++/heme are added to the solution using 100 mM CuCl₂, and the hemoglobin is incubated for 5 minutes on ice, under CO. The reaction is quenched by the addition of a five-fold molar excess (with respect to copper) of Na-EDTA. The resulting octamers are then purified as above.

### Example 3: Hypothetical protocol for the construction of hemoglobin molecules stabilized against dimer formation by fusion across the alpha 1- beta 2 or alpha 2- beta 1 dimer interface region

The currently employed inter-dimer di-alpha fusion between the C terminus of one alpha subunit and the N terminus of the adjacent alpha subunit, represents a successful protein engineering approach to stabilizing hemoglobin against dimer formation. In this case, use was made of the fortunate juxtaposition of the two termini which originate from different dimers. One might also make a di-beta polypeptide, as has been described, or a hemoglobin with both di-alpha and di-beta polypeptide, as has been described, or a hemoglobin with both di-alpha and di-beta linked subunits. Alternatively, one can envision other types of fusion in which the alpha subunit of one alpha/beta dimer is fused to the beta subunit of the other dimer (Figure 5). In this, two individual, linked polypeptides would dimerize to form the psuedo-tetrameric hemoglobin. This approach is based on the fact that dimerization involves specific, identical pairs of subunits, generally referred to as α1β1 and α2β2.

As an example of this alternative fusion approach, the alpha subunit C terminal residue (Arg 141) of dimer 1 could be fused, either directly or with an intervening fusion sequence, to the N-terminal amino acid of the beta subunit C helix (Tyr 35) of dimer 2. This would create a new C terminal residue at the end of the beta B helix (Val 34) and would leave a "free" piece of polypeptide comprised of the beta A and B helices (residues 1 to 34 inclusive). These alterations would give rise to a protein comprised of alpha subunit helices A through H fused to beta subunit helices C through H. The polypeptide composed of the beta subunit A and B helices would be covalently attached to the protein by introducing a new helix into the molecule. The helix would be designed to span the distance between the beta C terminus (His 146) and the original beta N terminus of helix A (Val 1). Following these changes, the sequence of helices from the N to C terminus of the new protein would be (alpha) A-B-C-E-F'-F-G-H-(beta)-C-D-E- F'-F-G-H-NEW-A-B. The actual arrangement of the fusion regions would require careful design so that new regions of structure did not extend into the dimer-dimer interface region which is critical to cooperativity. Introduction of amino acids containing basic or acidic residues into the molecule at certain positions cold allow some restoration of functionally important salt bridges and hydrogen bonds which could be lost as a result of manipulating the normal N and C termini. The above approach could also extend to the production of the entire hemoglobin molecule or individual dimers as single polypeptide chains, although in the latter case this would not be expected to offer stabilization against dimer formation.

For the purpose of providing the potential for disulfide bond formation, a cysteine may be introduced into either the α or β globin domain of the α₁β₂ pseudodimer.

### Reference Example A: Reconstitution of Recombinant Alpha-Globin and Recombinant Beta-Globin with Heme and chemical Reduction to Yield Artificial Hemoglobin

Conventional methods of preparing artificial hemoglobin are exemplified by the following procedure.

The lyophilized recombinant alpha and beta-globins (100 mg each) were individually dissolved in 8M urea/50mM Tris- C1, pH 8.01/lmM EDTA/ 1mM DTT, diluted to a concentration of 5 mg/ml and incubated at room temperature for 3-4 hours. The alpha-globin was then diluted to 0.3mg/ml with chilled 20mM K₂HPO₄, pH 5.7/1mM EDTA/1mM DTT. Hemin (25 mg) was dissolved in 2.4mg 0.1M KOH, diluted with an equal volume of 1M KCN; this solution was then made 0.lmg/ml in hemin and 20mM K₂HPO₄, pH 6.7 with stock phosphate buffer. Hemin from this solution was added at a 2.8 molar excess to the chilled alpha-globin; and equal molar amount of beta-globin was added and the solution was dialyzed at 4°C overnight against 0.1M K₂HPO₄, pH 7.6/1mM EDTA/ 1mM KCN. The artificial Hb solution was concentrated by ultra-filtration using a PM-10 membrane (Amicon) and transferred into a 200ml screw-top test tube with a rubber septum. The hemoglobin solution was deoxygenated by evacuation and flushing with N₂, and then the solution was saturated with CO. 100mM sodium dithionite solution was prepared anaerobically in a 20ml screw-top test tube with rubber septum. 4.5 equivalents of dithionite were added to the Hb solution with a syringe, and the mixture incubated on ice for 15 min. The Hb solution was gel-filtered against 10mM Na phosphate buffer pH 6.0 on a 4x40 cm Sephadex G-25 (fine) column. The colored solution was then applied to a 2x10 cm-52 (Whatman) column equilibrated with the same buffer and the chromatography was developed with a linear gradient of 500ml 10mM Na phosphate buffer pH 6.0 and 500ml of 70mM sodium phosphate buffer pH 6.9. CO was removed from Hb by photolysis under a stream of oxygen. Artificial Hgb prepared this way is isolated in only about 25% yield from the fusion peptides but shows native oxygen binding properties.

### Reference Example B: P₅₀ Determination

Our preferred method of measuring P50 of purified hemoglobin solutions for the purpose of the appended claims is as follows:

Hemoglobin-oxygen equilibrium curves are measured using a Hemox Analyzer™ (TCS Medical Products, Southampton, PA) at either 25°C or 37°C+0.1°C in 50 mM HEPES buffer/0.1 M NaCl, pH 7.4. Oxygen equilibrium curves are measured by N2 deoxygenation of an oxyhemoglobin solution that has been previously equilibrated with water-saturated O₂ (for samples iwth a P50>10 torr) or with water-saturated compressed air. Absorbance readings at 568 and 558 nm are measured throughout the run for determination of percent oxyhemoglobin in the sample. Precent oxyhemoglobin is directly proportional to log A 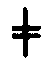 558/log A 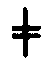 568 and is independent of path length. Both the absorbances and the oxygen pressure are sampled by a programmable-gain 12-bit analog-to-digital converter (Labmaster PGH, Scientific Solutions, Solon, OH) under computer control. The oxygen equilibrium curve is subjected to a low-apss digital filter. P₅₀ values (partial pressure of O₂ required for 50% saturation of oxygen binding sites) and Hill coefficients (ⁿmax) are calculated from the digitally filtered data by using software developed in our laboratory. The Hill coefficients are determined as the maximum slope of the functions dlog[y/(1-y)]/ dlog p, where y is % O₂ saturation and p is partial pressure of O₂.

P₅₀ may also be measured under other conditions, but it should be noted that many environmental factors affect hemoglobin's oxygen affinity. The effect of Ph, CO₂ inorganic unions, organic phosphates and temperature on P₅₀ are discussed in Bunn and Forget, HEMOGLOBIN: MOLECULAR, GENETIC AND CLINICAL ASPECTS 37-47, 95-98 (W.B. Saunders Co., 1986).

Since many determinations of whole blood oxygen binding curves are made under standard physiologic conditions (37°C, pH, 7.4, PCO₂ 40mm Hg), it may be necessary to adjust literature figures. In this context, it should be noted that a 10°C increase results in nearly a two-fold increase in P₅₀, while the dependence of P₅₀, while the dependence of P₅₀ on pH is approximately given as delta log P₅₀/delta pH= -0.5.

Comparing P₅₀ values of purified Hb preparations to P₅₀ values of whole blood can be problematic. Whole blood, or isolated RBC's contain many components that naturally modulate the shape of the hemoglobin-oxygen binding curve. The RBC encapsulates Hgb in the presence of a high concentration of the effector molecule 2.3-DPG; a molecule that causes Hgb to have a markedly lower affinity for O₂. Other intra-erythrocyte components also affect the shape of the binding curve: ATP, Cl- CO₂, H+, orthophosphate, methemoglobin and carboxyhemoglobin. The levels of these substances may vary with age, sex and condition. These substances are not normally present in purified HgB solutions and thus, the P₅₀ value of purified Hgb is lower than that found in whole blood. One very important modulator of Hgb-oxygen affinity is C1- ion. C1 ion is found outside the erythrocyte in the blood serum at a physiologic concentration of approximately 0.15M. Since C1- causes a lower O₂ affinity, a Hgb solution with a P₅₀ measured in vitro may well have much lower O₂ affinity if infused into the blood stream. Another problem with measuring O₂ binding of whole blood is that RBCs are quite fragile and in the process of manipulating the erythrocyte into the instrument used to measure the O₂ binding it is inevitable that at least a small percentage of the RBCs will lyse. Lysed RBCs leak Hgb into the surrounding media away from 2,3-DPG; hence, since free Hgb has a higher affinity than intraerythrocyte Hgb, lysed RBCs will have a higher O₂ affinity and can cause a falsely low P50 value for whole blood P50 determinations. It is widely accepted that under physiologic conditions while blood has a P50 value of 26- 28 mm Hg. When Hgb is isolated from whole blood, however, the measured P50 is on the order of 1-10 mm Hg depending on the investigator's experimental conditions. For these reasons it is most accurate to measure Hgb-oxygen equilibria with purified Hgb molecules under strict conditions of buffer, pH and salt concentrations. Unfortunately, there are no accepted "standards" for all investigators to measure Hgb oxygen binding for in vitro systems.

Still, as many mutant hemoglobins are first identified in patient's whole blood, one would like to be able to compare the relative affinities of native and mutant Hgb for O2, between whole blood and purified Hgb preparations. An example of this is Hgb Chico (beta lys⁶⁶-thr). If one examined only the P₅₀ value of the purified mutant Hgb (10.1 mmHg) one would note that Hgb has a P₅₀ value less than that for normal whole blood (27.2 mmHg). Still, when that hemoglobin is measured in RBCs under physiologic conditions it is apparent that it does have a higher P₅₀ than normal whole blood (38mmHg). One cannot predict the degree that the P₅₀ value will change going from whole blood Chico to a purified Hgb Chico if it were infused into the bloodstream as a blood substitute. One can conclude however, that the P₅₀ will be higher than it is in pure form, and that by reacting the mutant Hgb with organic phosphates that P₅₀ will be even higher.

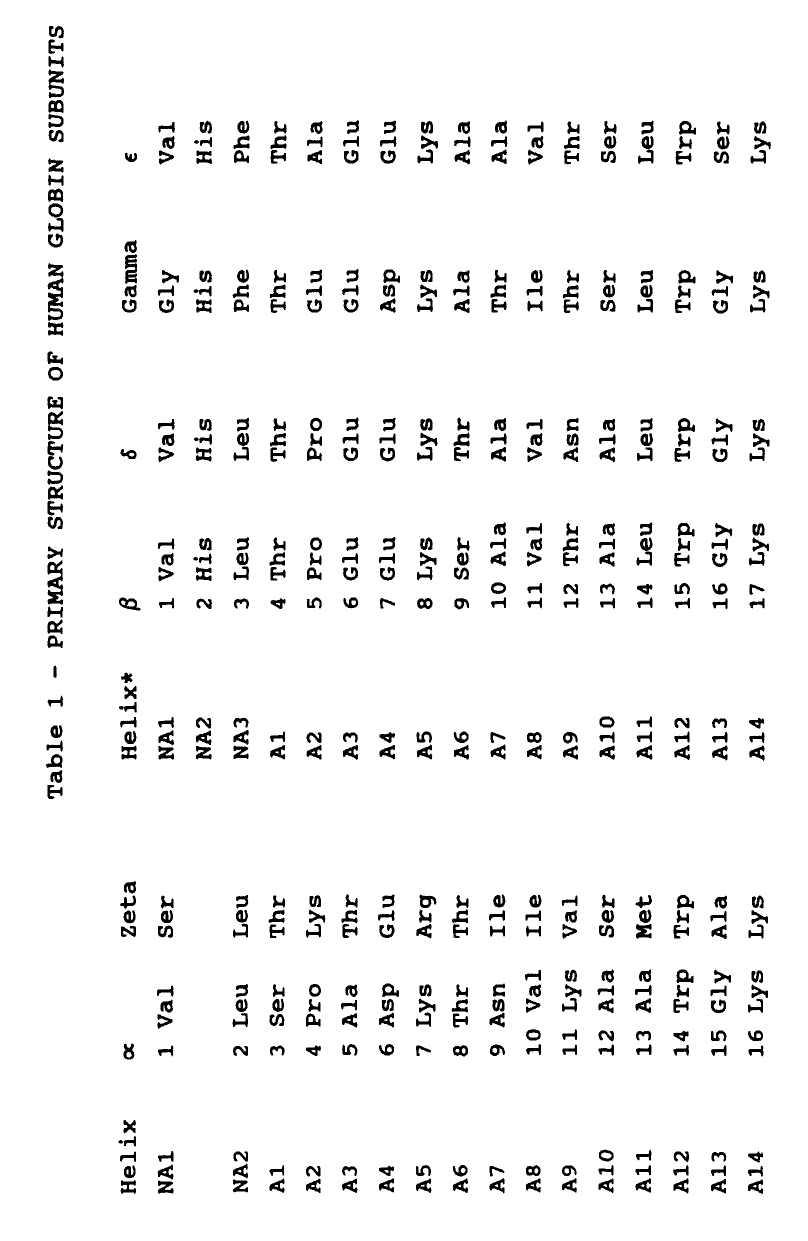

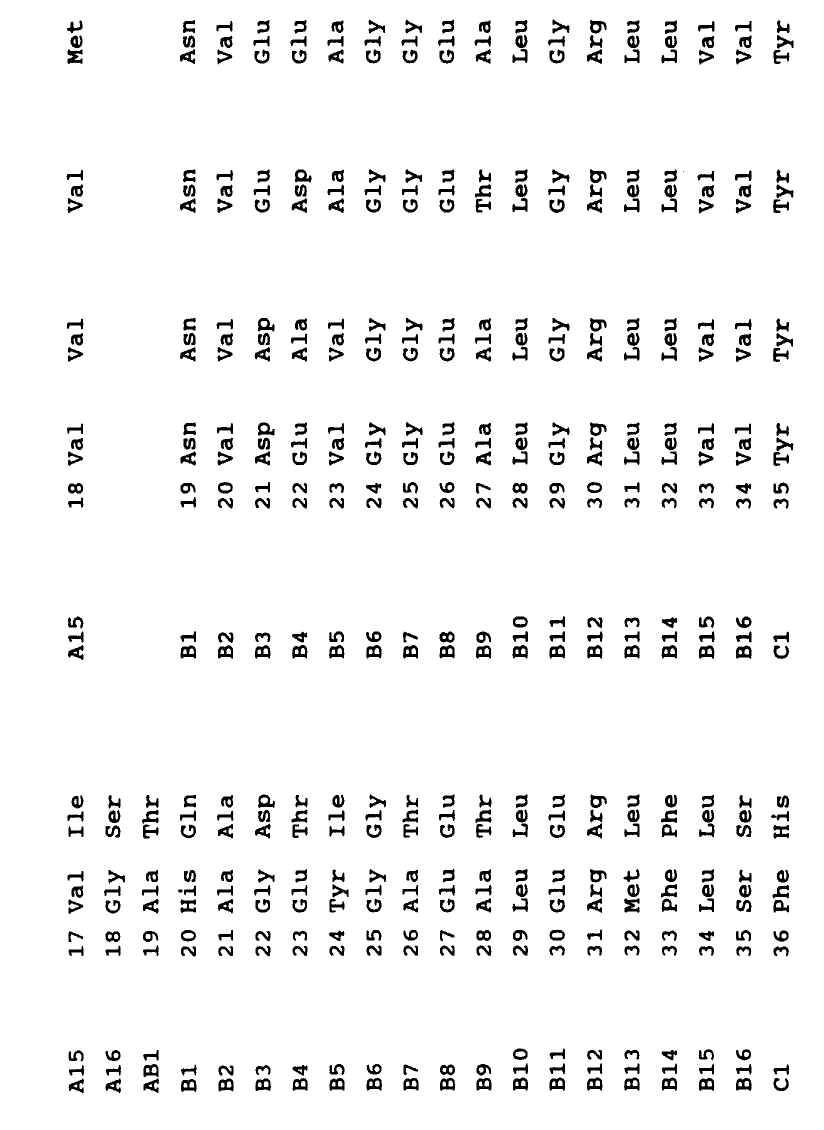

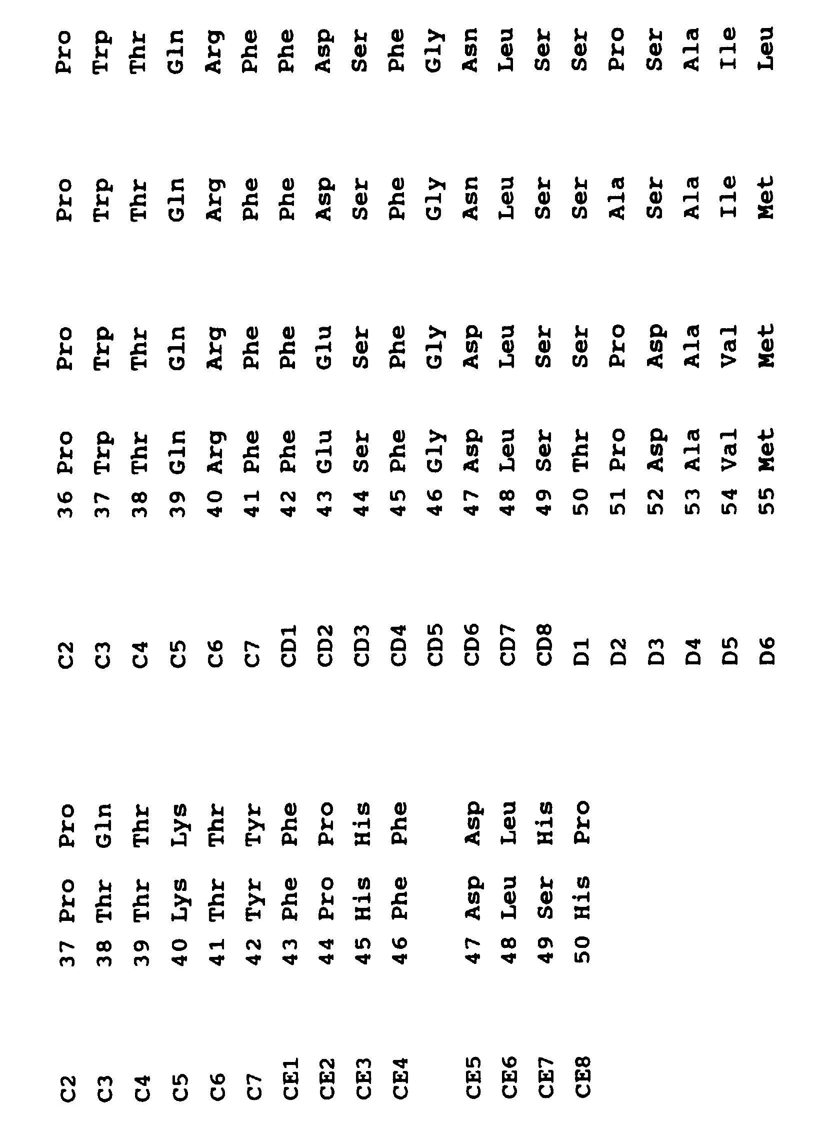

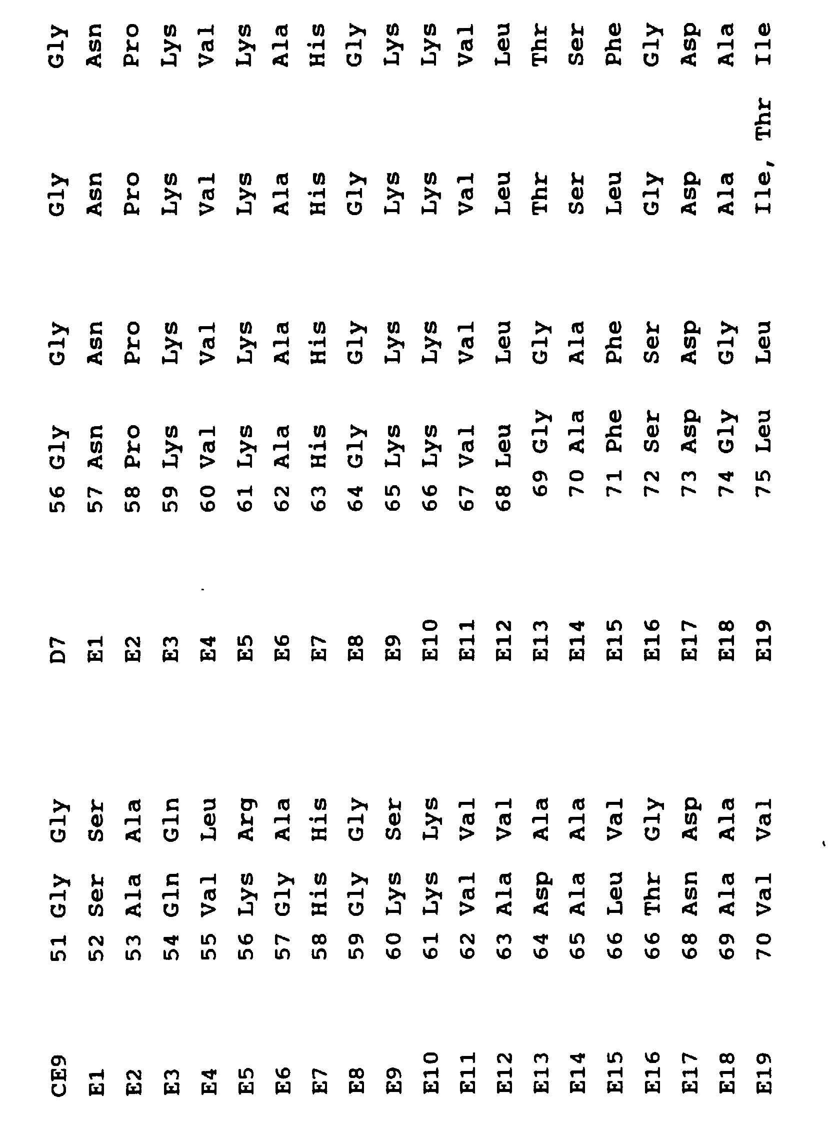

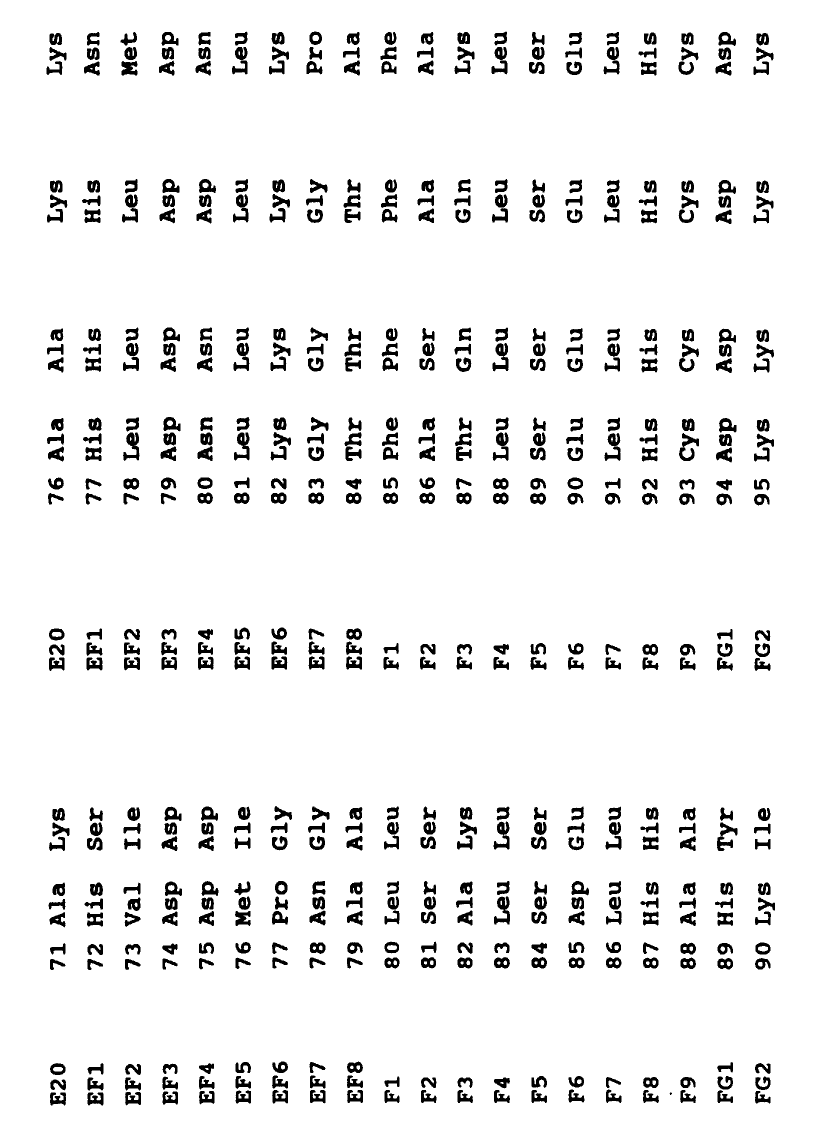

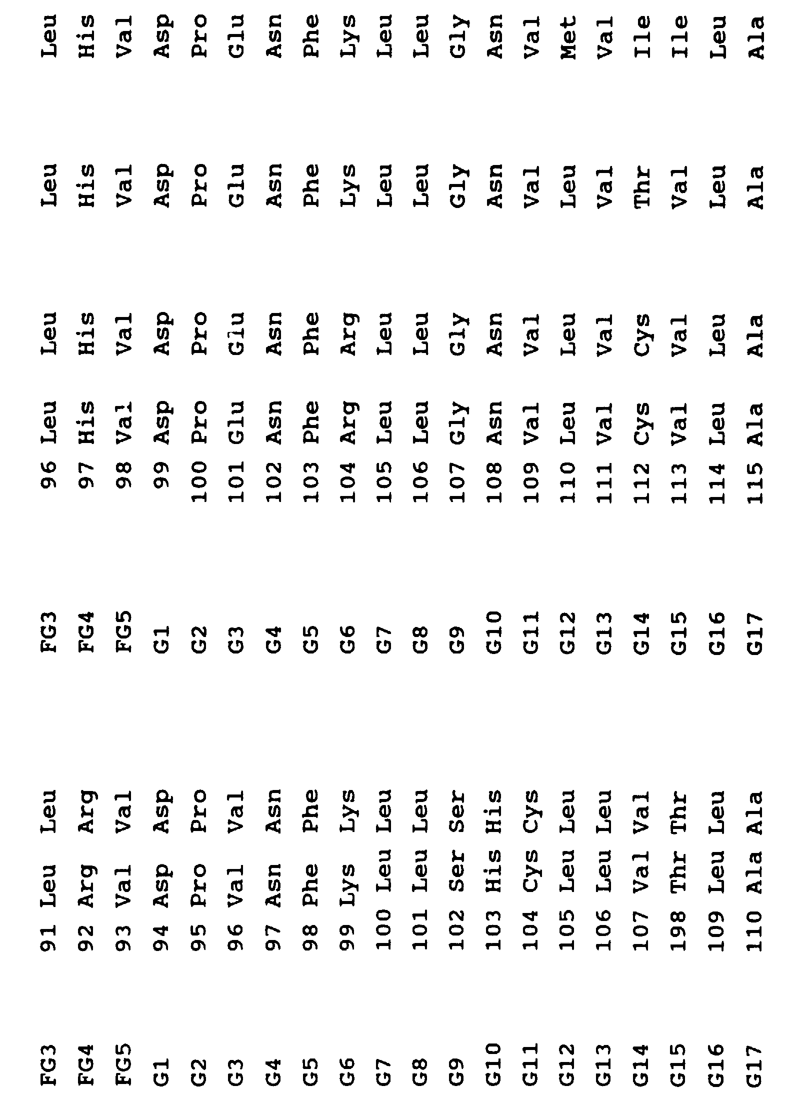

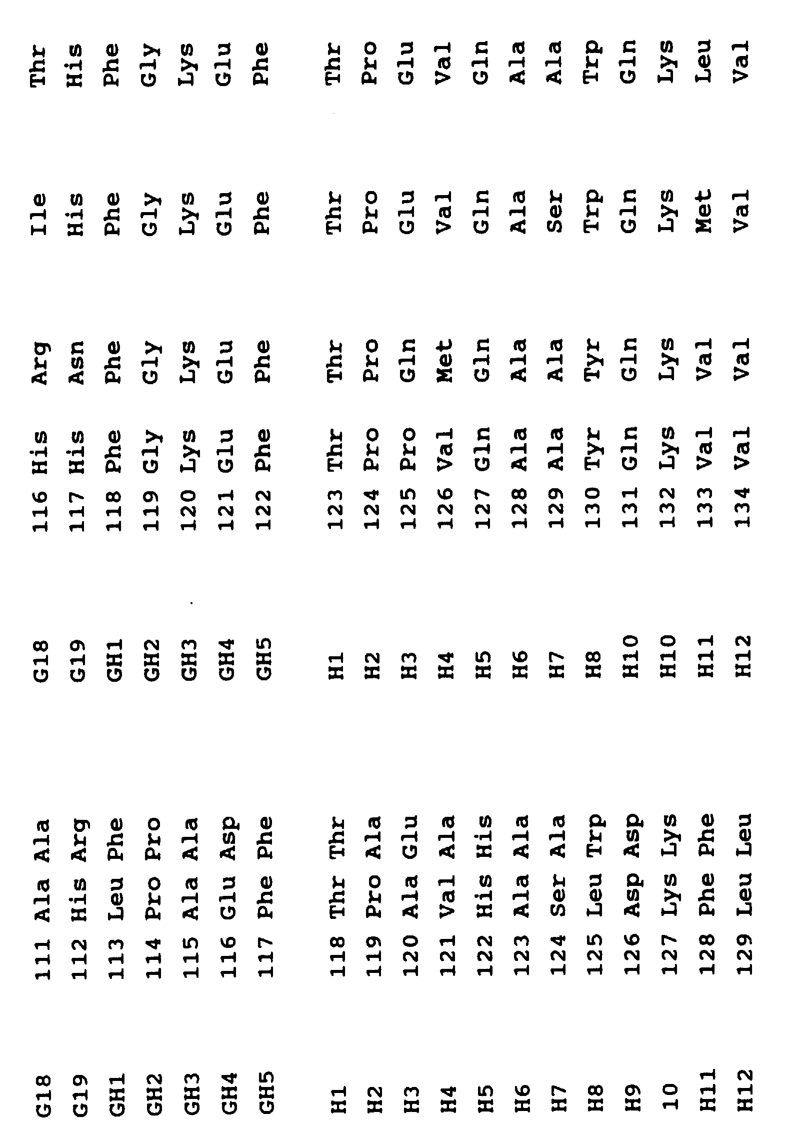

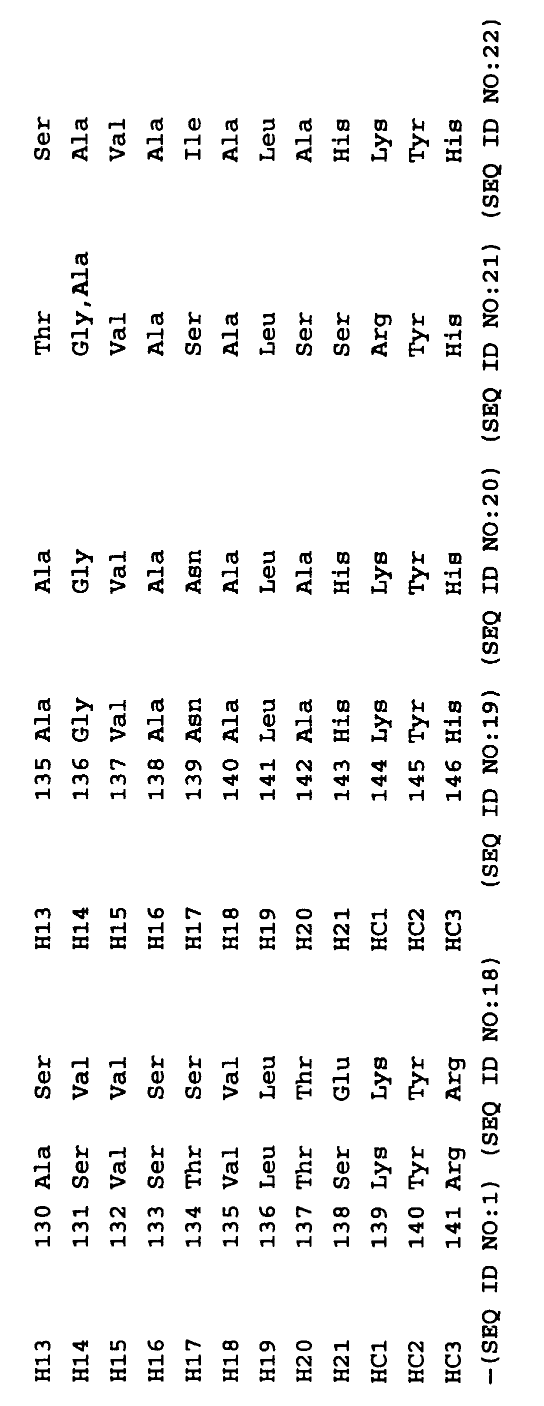

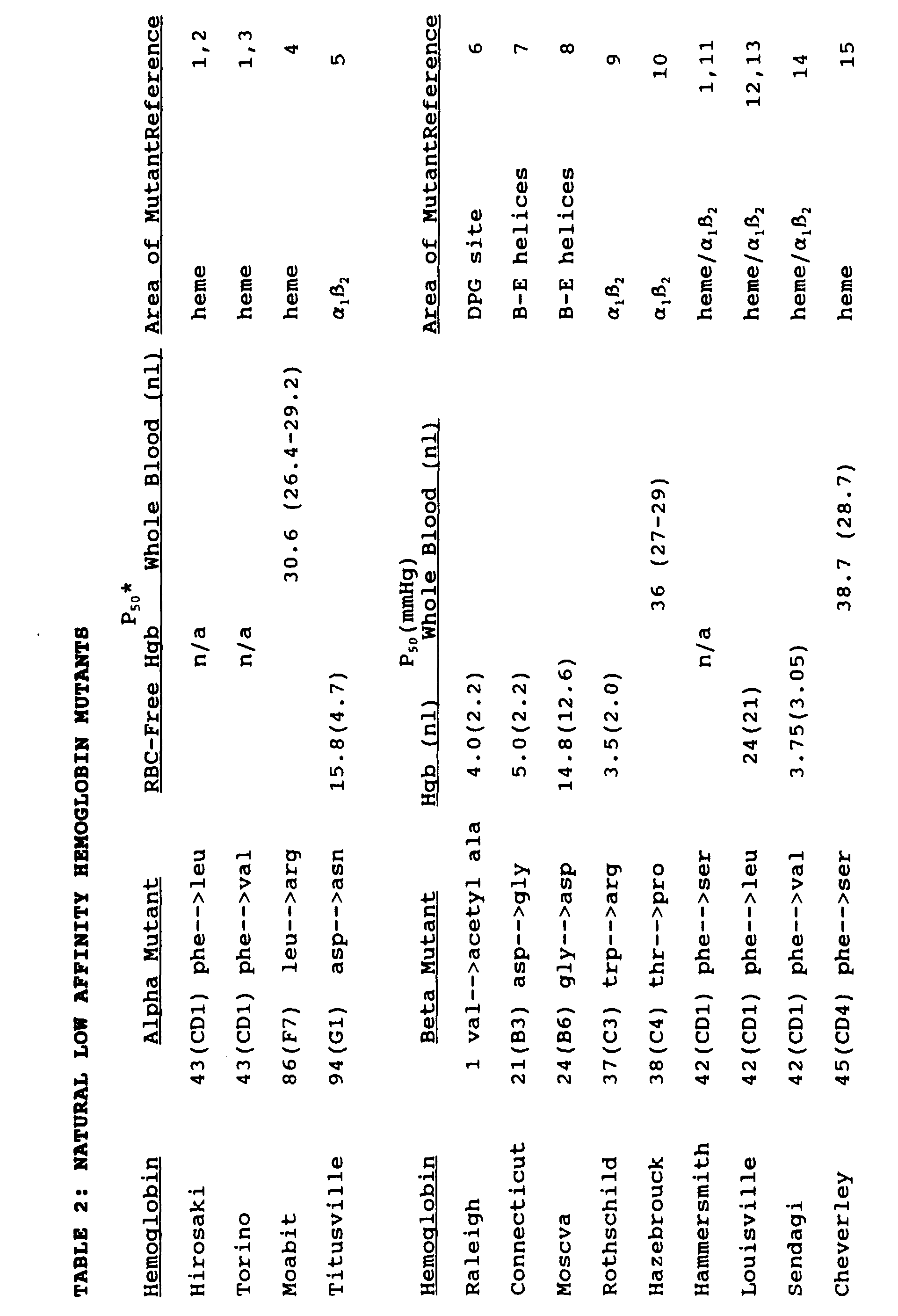

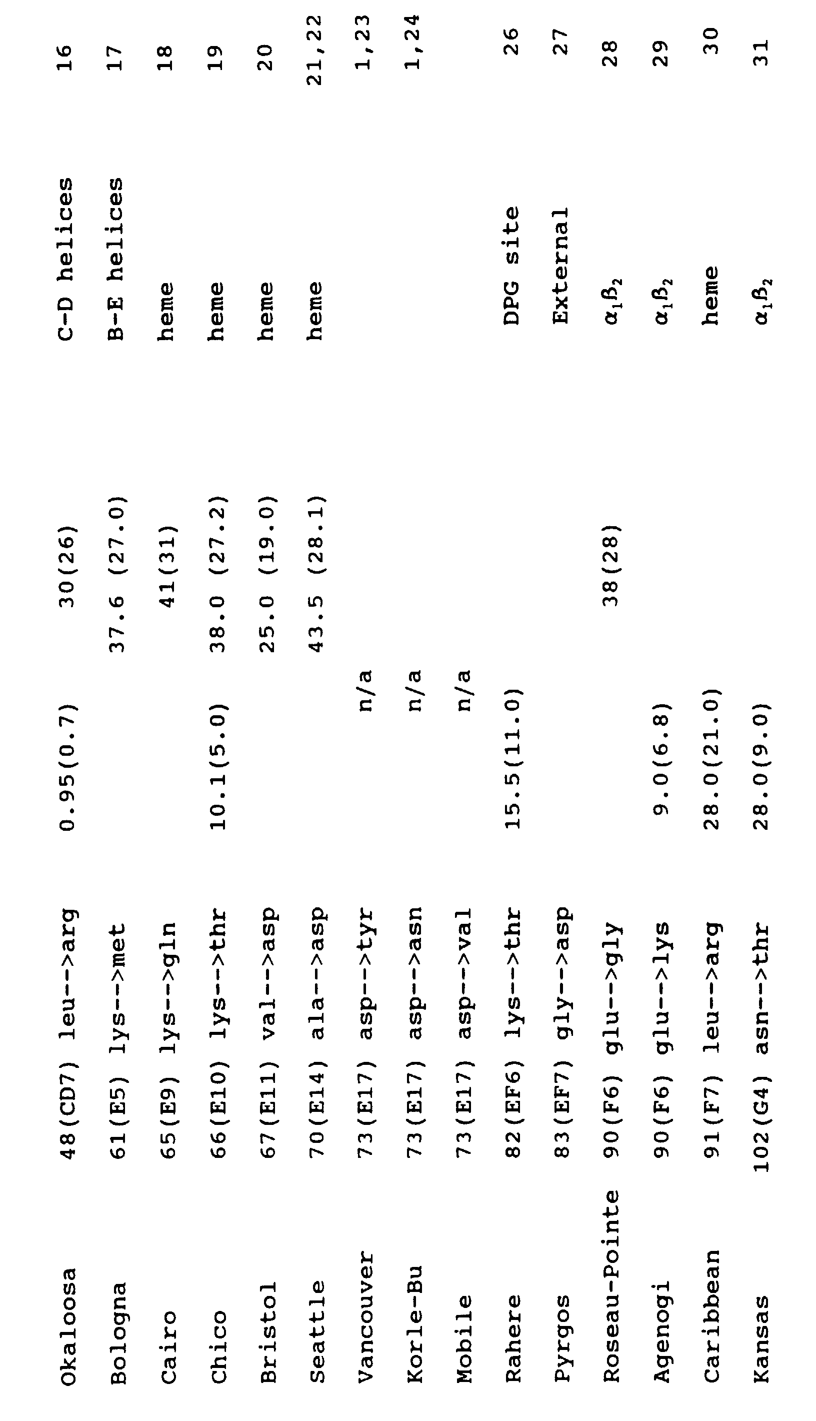

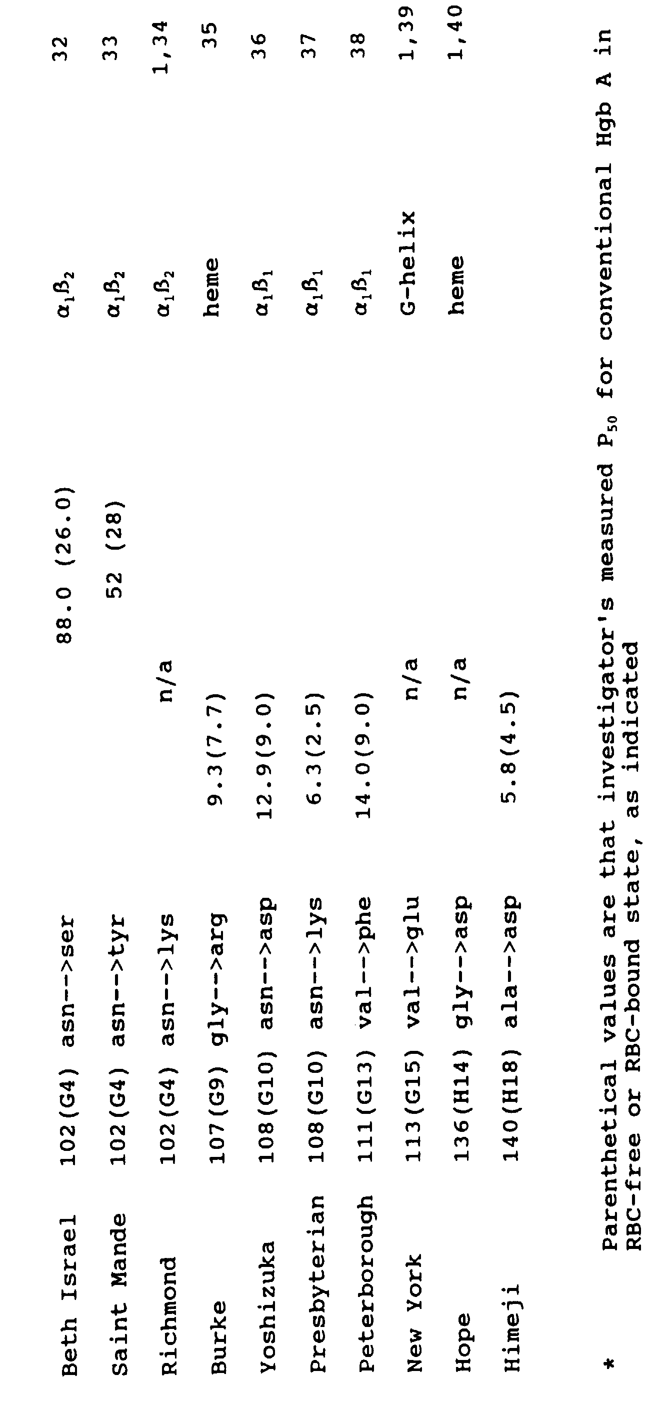

### References for Table 2

1) Hemoglobin 1987, 11, 241-308.
2) Ohba, Y.; Miyaji, T.; Matsuoka, M.; Yokoyama, M.; Numakura, H.; Nagata, K.; Takebe, Y.; Izumu, Y.; Shibata, S. Biochemi. Biophys. Acta 1975, 405, 155-160.
3) Beretta, A.; Prato, V.; Gallo, E.; Lehmann, H. Nature 1968, 217, 1016-1018.
4) Knuth, A.; Pribilla, W.; Marti, H.R.; Winterhalter, K.H. Acta Haematol 1979, 61, 121-124.
5) Schneider, R.G.; Atkins, R.J.; Hosty, T.S.; Tomlin, G.; Casey, R.; Lehmann, H.; Lorkin, P.A.; Nagei, K. Biochem Biophys. Acta 1975, 400, 365-373.
6) Moo-Penn, W.F.; Bechtel, K.C.; Schmidt, R.M.; Johnson, M.H.; Jue, D.L.; Schmidt, D.E.; Dunlap, W.M.; Opella, S.J.; Boneventura, J.; Boneventura, C. Biochemistry 1977, 16, 4872-4879.
7) Moo-Penn, W.F.; McPhedran, P.; Bobrow, S.; Johnson, M.H.; Jue, D.L.; Olsen, K.W. Amer. J. Hematol 1981, 11, 137-145.
8) Idelson, L.I.; Didkowsky, N.A.; Casey, R.; Lorkin, P.A.; Lehmann, H. Nature 1974, 249, 768-770.
9) Gacon, G.; Belkhodja, O.; Wajcman, H.; Labie, D. Febs Lett 1977, 82, 243-246.
10) Blouquit.; Delanoe,-Garin, J.; Lacombe, C.; Arous, N.; Cayre, Y.; Peduzzi, J.; Braconnier, F.; Galacteros, F.; Febs Lett. 1984, 172, 155-158.
11) Dacie, J.V.; Shinton, N.K.; Gaffney, P.J.; Carrell, R.W.; Lehmann, H. Nature 1967, 216, 663-665.
12) Keeling, M.M.; Ogden, L.L.; Wrightstone, R.N.; Wilson, J.B.; Reynolds, C.A.; Kitchens, J.L.; Huisman, T.H. J. Clin. Invest. 1971, 50, 2395-2402.
13) Bratu, V.; Larkin, P.A.; Lehmann, H.; Predescu, C. Biochem. Biophys. Acta. 1971, 251, 1-6.
14) Ogata, K.; Ho, T.; Okazaki, T.: Dan, K.; Nomura, T.; Nozawa, Y.; Kajita, A. Hemoglobin 1986, 10**,** 469-481.
15) Yeager, A.M.; Zinkham, W.H.; Jue, D.L.; Winslow, R.M.; Johnson, M.H.; McGuffey, J.E.; Moo-Penn, W.F. Ped. Res. 1983, 17, 503-507.
16) Charache, S.; Brimhall, B.; Milner, P.; Cobb, L. J. Clin. Invest. 1973, 52, 2858-2864.
17) Marinucci, M.; Giuliani, A.; Maffi, D.; Massa, A.; Giampolo, A.; Mavilio, F.; Zannotti, M.; Tantori, L. Biochem. Biophys. Acta. 1981, 668, 209-215.
18) Garel, M.C.; Hasson, W.; Coquelet, M.T.; Goosens, M.; Rosa, J.; Arous, N. Biochem. Biophys. Acta. 1976, 420, 97-104.
19) Shih, D.T.; Jones, R.T.; Shih, M.F.C.; Jones, M.B.; Koler, R.D.; Hemoglobin 1987, 11, 453-464.
20) Steadman, J.H.; Yates, A.; Huehns, E.R.; Brit., J. Haematol 1970, 18**,** 435-446.
21) Stamotoyannopoulos, G.; Parer, J.T.; Finch, C. New Eng. J. Med. 1969, 281, 915-919.
22) Anderson, N.L.; Perutz, M.F.; Stamatoyannopoulos, G. Nature New Biol. 1973, 243, 275-276.
23) Jones, R.T.; Brimhall, B.; Pootrakul, S.; Gray, G. J. Mol. Evol. 1976, 9, 37-44.
24) Konotey-Ahulu, F.I.D.; Gallo, E.; Lehmann, H.; Ringelhann, B. J. Med. Genet. 1968, 5, 107-111.
25) Schneider, R.G.; Hosty, T.S.; Tomlin, G.; Atkins, R.; Brimhall, B.; Jones, R.T. Biochem Genet. 1975, 13, 411-415.
26) Sugihara, J.; Imamura, T.; Nagafuchi, S.; Boneventura, J.; Boneventura, C.; Cashon, R. J. Clin. Invest. 1985, 76, 1169-1173.
27) Tatsis, B.; Sofroniadou, K.; Stergiopoulas, C.I. Blood 1976, 47, 827-832.
28) Merault, G.; Keclard, L.; Saint-Martin, C.; Jasmin, K.; Campier, A.; Delanoe Garin, J.; Arous, N.; Fortune, R.; Theodore, M.; Seytor, S.; Rosa, J.; Blouquit, Y.; Galacteros, F. Febs Lett. 1985, 184, 10-13.
29) Imar, K.; Morimoto, H.; Kotani, M.; Shibata, S.; Miyaji, T.1 Matsutomo, K. Biochem. Biophys. Acta. 1970, 200, 197-202.
30) Ahern, E.; Ahern, V.; Hilton, T.; Serjeant, G.D.; Serjeant, B.E.; Seakins, M.; Lang, A.; Middleton, A.; Lehmann, H. Febs Lett. 1976, 69, 99-102.
31) Boneventura, J.; Riggs, A.; J. Biol. Chem. 1968, 243, 980-991.
32) Nagel, R.L.; Lynfield, J.; Johnson, J.; Landeau, L.; Bookchin, R.M.; Harris, M.B. N. Ena. J. Med. 1976, 295, 125-130.
33) Arous, N.; Braconnier, F.; Thillet, J.; Blouquit, Y.; Galacteros, F.; Chevrier, M.; Bordahandy, C.; Rosa, J. Febs Lett. 1981, 126, 114-116.
34) Efremov, G.D.; Huisman, T.H.J.; Smith, L.L.; Wilson, J.B.; Kitchens, J.L.; Wrightston, R.N.; Adams, H.R.; J. Biol. Chem. 1969, 244, 6105-6116.
35) Turner, J.W.; Jones, R.T.; Brimhall, B.; DuVal, M.C.; Koler, R.D. Biochem. Genet. 1976, 14, 577-585.
36) Imamura, T.; Fujita, S.; Ohta, Y.; Hanada, M.; Yanase, T. J. Clin. Invest. 1969, 48, 2341-2348.
37) Moo-Penn, W.F.; Wolff, J.A.; Simon, G.; Vacek, M.; Jue, D.L.; Johnson, M.H. Febs Lett. 1978, 92, 53-56.
38) King, M.A.R.; Willshire, B.G.; Lehmann, H.; Marimoto, H. Br. J. Haem. 1972, 22, 125-134.
39) Ranney, H.M.; Jacobs, A.S.; Nagel, R.L. Nature 1967, 213, 876-878.
40) Minnich, V.; Hill, R.J.; Khuri, P.D.; Anderson M.E. Blood 1965, 25, 830-838.
41) Ohba, Y.; Miyaji, T.; Murakami, M.; Kadowaki, S.; Fujita, T.; Oimoni, M.; Hatanaka, H.; Ishikawa, K.; Baba, S.; Hitaka, K,; Imai, K. Hemoglobin 1986, 10, 109-126.

**Table 3:**

| **Candidate Non-Naturally Occurring Low Affinity Hemogblobin Mutants** | |
|---|---|
| alpha chain | |
| 46 | phe-->thr |
| 46 | phe-->ser |
| 46 | phe-->ala |
| 58 | his-->phe |
| 58 | his-->trp |
| 61 | lys-->thr |
| 61 | lys-->ser |
| 61 | lys-- >met |
| 61 | lys-->asn |
| 62 | val-->leu |
| 62 | val-->ile |
| 62 | val-->phe |
| 62 | val-->trp |
| 65 | ala-->asp |
| 94 | asp-->gln |
| 94 | asp-->thr |
| 94 | asp-->ser |
| 94 | asp-->lys |
| 94 | asp-->gly |
| 94 | asp-->arg |

| beta chain | |
|---|---|
| 21 | asp-->ala |
| 21 | asp-->ser |
| 45 | phe-->ala |
| 45 | phe-->thr |
| 45 | phe-->val |
| 63 | his-->phe |
| 63 | his-->trp |
| 66 | lys-->ser |
| | |
| 66 | lys-->asn |
| 67 | val-->phe |
| 67 | val-->trp |
| 67 | val-->ile |
| 70 | ala-->glu |
| 70 | ala-->ser |
| 70 | ala-->thr |
| 96 | leu-->phe |
| 96 | leu-->his |
| 96 | leu-->lys |
| 98 | val-->trp |
| 98 | val-->phe |
| 102 | asn-->asp |
| 102 | asn-->glu |
| 102 | asn-->arg |
| 102 | asn-->his |
| 102 | asn-->gly |
| 108 | asn-->arg |
| 108 | asn-->glu |

**TABLE 400**

| HIGH OXYGEN AFFINITY, NATURALLY OCCURRING HEMOGLOBIN MUTANTS | |
|---|---|
| Structure | Name |
| A. Alpha Chain Mutants | |
| 6 (A4) | Asp->AlaSawara |
| | Asp->AsnDunn |
| | Asp->ValFerndown |
| | Asp->TyrWoodville |
| | Lys->AsnAlbany-Suma |
| 40 (C5) | Lys->GluKariya |
| 44 (CE2) | Pro->LeuMilledgeville |
| | Pro->ArgKawachi |
| 45 (CE3) | His->ArgFort de France |
| 85 (F6) | Asp->AsnG-Norfolk |
| 92 (FG4) | Arg->GlnJ-Cape Town |
| | Arg->LeuChesapeake |
| 95 (G2) | Pro->LeuG-Georgia |
| | Pro->SerRampa |
| | Pro->AlaDenmark Hill |
| | Pro->ArgSt. Luke's |
| 97 (G4) | Asn->LysDallas |
| 126 (H9) | Asp->AsnTarrant |
| 141 (HC3) | Arg->HisSuresnes |
| | Arg->SerJ-Cubujuqui |
| | Arg->LeuLegnano |
| | |

| B. Beta Chain Mutants | |
|---|---|
| 2 (NA2) | His->ArgDeer Lodge |
| | His->GlnOkayama |
| 20 (B2) | Val->MetOlympia |
| 23 (B5) | Val->AspStrasbourg |
| | Val->PhePalmerston North |
| 34 (B16) | Val->PhePitie-Salpetriere |
| 36 (C2) | Pro->ThrLinkoping |
| 37 (C3) | Trp->SerHirose |
| 40 (C6) | Arg->LysAthens-Ga |
| | Arg->SerAustin |
| 51 (D2) | Pro->ArgWillamette |
| | Leu->HisBrisbane |
| 79 (EF3) | Asp->Gly G-Hsi-Tsou |
| | Lys->ThrRahere |
| | Lys->MetHelsinki |
| 89 (F5) | Ser->AsnCreteil |
| | Ser->ArgVanderbilt |
| | |
| 94 (FG1) | Asp->HisBarcelona |
| | Asp->AsnBunbury |
| 96 (FG3) | Leu->Val Regina |
| 97 (FG4) | His->GlnMalmo |
| | His->LeuWood |
| 99 (G1) | Asp->AsnKempsey |
| | Asp->HisYakima |
| | Asp->AlaRadcliffe |
| | Asp->Tyr Ypsilanti |
| | Asp->GlyHotel-Dieu |
| | Asp->Val Chemilly |
| 100 (G2) | Pro->LeuBrigham |
| 101 (G3) | Glu->LysBritish Columbia |
| | Glu->GlyAlberta |
| | Glu->AspPotomac |
| 103 (G5) | Phe->LeuHeathrow |
| 109 (G11) | Val->MetSan Diego |
| 121 (GH4) | Glu->GlnD-Los Angeles |
| | Pro->GlnTu Gard |
| | Ala->ProCrete |
| 140 (H18) | Ala->ThrSt.-Jacques |
| 142 (H20) | Ala->AspOhio |
| 143 (H21) | His->ArgAbruzzo |
| | His->GlnLittle Rock |
| | His->ProSyracuse |
| 144 (HC1) | Lys->AsnAndrew-Minneapolis |
| 145 (HC2) | Tyr->HisBethesda |
| | Tyr->CysRainier |
| | Tyr->AspFort Gordon |
| | Tyr->TermMcKees Rocks |
| 146 (HC3) | His->AspHiroshima |
| | His->ProYork |
| | His->LeuCowtown |

## Claims

1. A pseudotetrameric protein consisting of polypeptide chains and having at least the following characteristics:
(i) it is sufficiently soluble in blood to be clinically useful as a blood substitute, (ii) it binds oxygen reversibly under physiological conditions, (iii) each polypeptide chain has at least one globin-like domain which is substantially homologous with a globin subunit of a naturally occurring hemoglobin and (iv) each of the globin-like domains of the protein is capable of incorporating a heme prosthetic group,
said protein comprising four globin-like domains which are each substantially homologous with a globin subunit of a naturally occurring hemoglobin,
at least one polypeptide chain of the protein being a pseudodimeric polypeptide comprising two such globin-like domains, which are connected either directly by peptide bond or indirectly by a peptide bonded amino acid or peptide linker between the normal C terminus of one domain and the normal N terminus of the next domain so as to form a single polypeptide chain, one domain of which is mutated to provide an asymmetric crosslinkable cysteine residue, the corresponding residue in the other domain of said pseudodimeric polypeptide being an amino acid other than cysteine,
said pseudotetrameric protein further comprising at least one other polypeptide bearing a globin-like domain which is substantially homologous with a globin subunit of a naturally occurring hemoglobin.

2. A multimeric protein having at least the following characteristics:
(i) it is sufficiently soluble in blood to be clinically useful as a blood substitute, (ii) it binds oxygen reversibly under physiological conditions, (iii) each polypeptide chain has at least one globin-like domain which is substantially homologous with a globin subunit of a naturally occurring hemoglobin and (iv) each of the globin-like domains of the protein is capable of incorporating a heme prosthetic group,
said protein being composed of two or more pseudotetrameric proteins according to claim 1, the asymmetric crosslinkable cysteine residue of a first tetrameric protein being crosslinked to an asymmetric crosslinkable cysteine residue of a second tetrameric protein.

3. The protein of claim 2 wherein the crosslink comprises a disulfide bond.

4. The protein of claim 2 wherein the crosslink is formed by reaction of the tetrameric proteins with a multi-functional, thiol-specific crosslinking reagent.

5. The protein of claim 4 wherein the crosslinking reagent forms a crosslink which is substantially less reducible by reducing agents endogenous to plasma than would be a disulfide bond between the same residues.

6. The protein of claim 4 or 5 wherein the crosslink is a thioether or thio-maleiimide crosslink.

7. The protein of claims 4 or 5 in which the crosslinking reagent is a polyethylene glycol derivative with thiol-specific reactive moieties.

8. The protein of any of claims 2-7 in which the protein is composed of two pseudotetrameric proteins.

9. The protein of any of claims 4-7 in which the protein is composed of more than two pseudotetrameric proteins.

10. The protein of any of claims 4-9 in which the crosslinking agent comprises a peptide moiety.

11. The protein of claim 10 in which the crosslinking agent comprises one or more D-amino acids.

12. The protein of claim 10 in which the peptide moiety comprises a stable, extended, secondary structure.

13. The protein of claim 12 in which the peptide moiety comprises a polyproline helix.

14. The protein of claim 12 in which the crosslinking agent comprises a two-stranded coiled coil.

15. The protein of claim 14 in which the two strands of the coiled coil are disulfide cross-linked.

16. The protein of claim 12 in which the crosslinking agent comprises a 4-helical or a 4-stranded coiled coil.

17. The protein of claim 12 in which the peptide moiety comprises a branched chain.

18. The protein of any of claims 4-17 wherein the crosslink comprises a negatively charged moiety, whereby the isoelectric point of the protein is reduced.

19. The protein of claim 18 wherein the negatively charged moiety comprises a plurality of Asp and/or Glu residues.

20. The protein of any of claims 1-19 wherein the cysteine residue lies in an alpha globin-like domain which is substantially homologous with an alpha globin subunit of a naturally occurring hemoglobin.

21. The protein of any of claims 1-19 wherein the cysteine residue lies in a beta globin-like domain which is substantially homologous with a beta globin subunit of a naturally occurring hemoglobin.

22. The protein of any of claims 1-21, wherein the protein is mutated, relative to human hemoglobin, to inhibit haptoglobin binding.

23. The multimeric protein of any of claims 2-22 wherein said crosslinking inhibits haptoglobin binding.

24. The protein of any of claims 1-23 wherein the globin-like domains of the pseudodimeric polypeptide are joined by a linker moiety consisting essentially of one or more glycines.

25. The protein of claim 24 wherein the domains are alpha-globin-like domains which are substantially homologous with an alpha-globin subunit of a naturally occurring hemoglobin and the linker moiety is 1-3 glycerines.

26. The protein of claim 24 wherein the domains are alpha globin-like domains which are substantially homologous with a beta-globin subunit of a naturally occurring hemoglobin and the linker moiety is 2-9 glycerines.

27. The protein of any of claims 1-26, which protein has a P₅₀ at least 10% greater than does human hemoglobin Ao under the same conditions.

28. The protein of any of claims 1-26, which protein has a P₅₀ at least 10% lower than does human hemoglobin Ao under the same conditions.

29. The protein of any of claims 1-26, wherein said protein has a P₅₀ of 0.327-0.435 kg/m² (24-32 torr).

30. The protein of any of claims 1-29, wherein said protein has substantially longer intravascular retention than normal hemoglobin free in plasma.

31. The protein of any of claims 1-30 wherein each of the globin-like domains is a vertebrate globin-like domain which is substantially homologous with a globin subunit of a naturally occurring vertebrate hemoglobin.

32. The protein of any of claims 1-30 wherein each of the globin-like domains is a mammalian globin-like domain which is substantially homologous with a globin subunit of a naturally occurring mammalian hemoglobin.

33. The protein of any of claims 1-30 wherein each of the globin-like domains is a human globin-like domain which is substantially homologous with a globin subunit of a naturally occurring human hemoglobin.

34. Use of the protein of any of claims 1-33 in the manufacture of a blood substitute composition.

35. A protein according to any of claims 1-33 for use as a medicament.

36. A protein according to any of claims 1-33 for use as a blood substitute.

37. A blood substitute composition comprising the protein of any of claims 1-33.

38. A composition according to claim 37 which is at least 50% monodisperse.

39. A protein according to any of claims 1-33 which is in at least partially purified form.

## Patentansprüche

1. Pseudotetrameres Protein, das aus Polypeptidketten besteht und wenigstens die nachfolgenden Eigenschaften aufweist:
(i) es ist ausreichend löslich in Blut, um klinisch als Blutersatz brauchbar zu sein, (ii) es bindet unter physiologischen Bedingungen reversibel Sauerstoff, (iii) jede Polypeptidkette hat wenigstens eine globinartige Domäne, die im wesentlichen mit einer Globin-Untereinheit eines natürlich auftretenden Hämoglobins homolog ist, und (iv) jede der globinartigen Domänen des Proteins kann eine prosthetische Hämgruppe einbauen,
wobei das Protein vier globinartige Domänen umfasst, die jeweils im wesentlichen mit einer Globin-Untereinheit eines natürlich auftretenden Hämoglobins homolog sind,
wobei wenigstens eine Polypeptidkette des Proteins ein pseudodimeres Polypeptid ist, das zwei solche globinartigen Domänen umfasst, die entweder direkt durch Peptidbindung oder indirekt durch eine peptidgebundene Aminosäure oder einen Peptidlinker zwischen dem normalen C-Terminus einer Domäne und dem normalen N-Terminus der nächsten Domäne verbunden sind, um eine einzelne Polypeptidkette zu bilden, wobei eine der Domänen mutiert ist, um einen asymmetrischen, vernetzbaren Cysteinrest zu liefern, wobei der entsprechende Rest in der anderen Domäne des pseudodimeren Polypeptids eine von Cystein verschiedene Aminosäure ist,
wobei das pseudotetramere Protein zusätzlich wenigstens ein anderes Polypeptid umfasst, das eine globinartige Domäne trägt, die im wesentlichen mit einer Globin-Untereinheit eines natürlich auftretenden Hämoglobins homolog ist.

2. Multimeres Protein mit wenigstens den nachfolgenden Eigenschaften:
(i) es ist ausreichend löslich in Blut, um klinisch als Blutersatz brauchbar zu sein, (ii) es bindet unter physiologischen Bedingungen reversibel Sauerstoff, (iii) jede Polypeptidkette hat wenigstens eine globinartige Domäne, die im wesentlichen mit einer Globin-Untereinheit eines natürlich auftretenden Hämoglobins homolog ist, und (iv) jede der globinartigen Domänen des Proteins kann eine prosthetische Hämgruppe einbauen,
wobei das Protein aus zwei oder mehr pseudotetrameren Proteinen gemäss Anspruch 1 zusammengesetzt ist, wobei der asymmetrische, vernetzbare Cysteinrest eines ersten tetrameren Proteins mit einem asymmetrischen, vernetzbaren Cysteinrest eines zweiten tetrameren Proteins vernetzt ist.

3. Protein gemäss Anspruch 2, worin die Vernetzungsstelle eine Disulfidbindung umfasst.

4. Protein gemäss Anspruch 2, worin die Vernetzungsstelle durch Reaktion des tetrameren Proteins mit einem multifunktionellen thiolspezifischen Vernetzungsmittel gebildet wird.

5. Protein gemäss Anspruch 4, worin das Vernetzungsmittel eine Vernetzungsstelle bildet, die durch Plasma-endogene Reduktionsmittel im wesentlichen weniger reduzierbar ist, als eine Disulfidbindung zwischen den gleichen Resten sein würde.

6. Protein gemäss Anspruch 4 oder 5, worin die Vernetzungsstelle eine Thioether- oder Thiomaleimid-Vernetzungsstelle ist.

7. Protein gemäss Anspruch 4 oder 5, worin das Vernetzungsmittel ein Polyethylenglykolderivat mit thiolspezifischen, reaktiven Komponenten ist.

8. Protein gemäss einem der Ansprüche 2 bis 7, worin das Protein aus zwei pseudotetrameren Proteinen zusammengesetzt ist.

9. Protein gemäss einem der Ansprüche 4 bis 7, worin das Protein aus mehr als zwei pseudotetrameren Proteinen zusammengesetzt ist.

10. Protein gemäss einem der Ansprüche 4 bis 9, worin das Vernetzungsmittel eine Peptidkomponente umfasst.

11. Protein gemäss Anspruch 10, worin das Vernetzungsmittel ein oder mehr D-Aminosäuren umfasst.

12. Protein gemäss Anspruch 10, worin die Peptidkomponente eine stabile, ausgedehnte Sekundärstruktur umfasst.

13. Protein gemäss Anspruch 12, worin die Peptidkomponente eine Polyprolinhelix umfasst.

14. Protein gemäss Anspruch 12, worin das Vernetzungsmittel eine zweisträngige Superhelix umfasst.

15. Protein gemäss Anspruch 14, worin die zwei Stränge der Superhelix Disulfid-vernetzt sind.

16. Protein gemäss Anspruch 12, worin das Vernetzungsmittel eine 4-helikale oder viersträngige Superhelix umfasst.

17. Protein gemäss Anspruch 12, worin die Peptidkomponente eine verzweigte Kette umfasst.

18. Protein gemäss einem der Ansprüche 4 bis 17, worin die Vernetzungsstelle eine negativ geladene Komponente umfasst, wodurch der isoelektrische Punkt des Proteins reduziert wird.

19. Protein gemäss Anspruch 18, worin die negativ geladene Komponente eine Mehrzahl von Asp- und/oder Glu-Resten umfasst.

20. Protein gemäss einem der Ansprüche 1 bis 19, worin der Cysteinrest in einer α-globinartigen Domäne liegt, die im wesentlichen mit einer α-Globin-Untereinheit eines natürlich auftretenden Hämoglobins homolog ist.

21. Protein gemäss einem der Ansprüche 1 bis 19, worin der Cysteinrest in einer β-globinartigen Domäne liegt, die im wesentlichen mit einer β-Globin-Untereinheit eines natürlich auftretenden Hämoglobins homolog ist.

22. Protein gemäss einem der Ansprüche 1 bis 21, worin das Protein relativ zu menschlichem Hämoglobin mutiert ist, um die Haptoglobinbindung zu inhibieren.

23. Multimeres Protein gemäss einem der Ansprüche 2 bis 22, worin die Vernetzung die Haptoglobinbindung inhibiert.

24. Protein gemäss einem der Ansprüche 1 bis 23, worin die globinartigen Domänen des pseudodimeren Polypeptids durch eine Linkerkomponente verknüpft sind, die im wesentlichen aus ein oder mehr Glycinen besteht.

25. Protein gemäss Anspruch 24, worin die Domänen α-globinartige Domänen sind, die im wesentlichen mit einer α-Globin-Untereinheit eines natürlich auftretenden Hämoglobins homolog sind, und die Linkerkomponente 1 bis 3 Glycine ist.

26. Protein gemäss Anspruch 24, worin die Domänen α-globinartige Domänen sind, die im wesentlichen mit einer β-Globin-Untereinheit eines natürlich auftretenden Hämoglobins homolog sind, und die Linkerkomponente 2 bis 9 Glycine ist.

27. Protein gemäss einem der Ansprüche 1 bis 26, worin das Protein ein P₅₀ hat, das wenigstens 10 % grösser ist als menschliches Hämoglobin Ao unter den gleichen Bedingungen.

28. Protein gemäss einem der Ansprüche 1 bis 26, worin das Protein ein P₅₀ hat, das wenigstens 10 % niedriger ist als menschliches Hämoglobin Ao unter den gleichen Bedingungen.

29. Protein gemäss einem der Ansprüche 1 bis 26, worin das Protein ein P₅₀ von 0,372 bis 0,435 kg/m² (24 bis 32 Torr) hat.

30. Protein gemäss einem der Ansprüche 1 bis 29, worin das Protein eine im wesentlichen längere intravaskuläre Retention als normales Hämoglobin hat, das frei im Plasma ist.

31. Protein gemäss einem der Ansprüche 1 bis 30, worin jede der globinartigen Domänen eine Wirbeltierglobinartige Domäne ist, die im wesentlichen mit einer Globin-Untereinheit eines natürlich auftretenden Wirbeltierhämoglobins homolog ist.

32. Protein gemäss einem der Ansprüche 1 bis 30, worin jede der globinartigen Domänen eine Säugetierglobinartige Domäne ist, die im wesentlichen mit einer Globin-Untereinheit eines natürlich auftretenden Säugetierhämoglobins homolog ist.

33. Protein gemäss einem der Ansprüche 1 bis 30, worin jede der globinartigen Domänen eine humanglobinähnliche Domäne ist, die im wesentlichen mit einer Globin-Untereinheit eines natürlich auftretenden Humanhämoglobins homolog ist.

34. Verwendung des Proteins gemäss einem der Ansprüche 1 bis 33 zur Herstellung einer Blutersatzzusammensetzung.

35. Protein gemäss einem der Ansprüche 1 bis 33 zur Verwendung als Medikament.

36. Protein gemäss einem der Ansprüche 1 bis 33 zur Verwendung als Blutersatz.

37. Blutersatzzusammensetzung, umfassend das Protein gemäss einem der Ansprüche 1 bis 33.

38. Zusammensetzung gemäss Anspruch 37, die zu wenigstens 50 % monodispers ist.

39. Protein gemäss einem der Ansprüche 1 bis 33, das in wenigstens teilweise gereinigter Form ist.

## Revendications

1. Protéine pseudo-tétramérique constituée de chaînes polypeptidiques et ayant au moins les caractéristiques suivantes :
(i) elle est suffisamment soluble dans le sang pour être cliniquement utile comme substitut sanguin, (ii) elle capte l'oxygène de façon réversible dans les conditions physiologiques, (iii) chaque chaîne polypeptidique comporte au moins un domaine de type globine qui est sensiblement homologue à une sous-unité de globine d'une hémoglobine naturelle et (iv) chacun des domaines de type globine de la protéine est capable d'incorporer un groupe de hème prosthétique,
ladite protéine comprenant quatre domaines de type globine qui sont tous sensiblement homologues à une sous-unité de globine d'une hémoglobine naturelle,
au moins une chaîne polypeptidique de la protéine étant un polypeptide pseudodimérique comprenant deux domaines de type globine de ce genre, qui sont reliés soit directement par une liaison peptidique, soit indirectement par un lieur acide aminé ou peptide lié à un peptide entre l'extrémité C-terminale normale d'un domaine et l'extrémité N-terminale normale du domaine suivant de manière à former une seule chaîne polypeptidique, dont un domaine est muté pour fournir un résidu cystéine réticulable asymétrique, le résidu correspondant dans l'autre domaine dudit polypeptide pseudodimérique étant un acide aminé autre que la cystéine,
ladite protéine pseudotétramérique comprenant en outre au moins un autre polypeptide portant un domaine de type globine qui est sensiblement homologue à une sous-unité de type globine d'une hémoglobine naturelle.

2. Protéine multimérique ayant au moins les caractéristiques suivantes :
(i) elle est suffisamment soluble dans le sang pour être cliniquement utile comme substitut sanguin, (ii) elle capte l'oxygène de façon réversible dans les conditions physiologiques, (iii) chaque chaîne polypeptidique a au moins un domaine de type globine qui est sensiblement homologue à une sous-unité de globine d'une hémoglobine naturelle et (iv) chacun des domaines de type globine de la protéine est capable d'incorporer un groupe de hème prosthétique,
ladite protéine étant composée de deux ou plusieurs protéines pseudotétramériques selon la revendication 1, le résidu cystéine réticulable asymétrique d'une première protéine tétramérique étant réticulé à un résidu cystéine réticulable asymétrique d'une seconde protéine tétramérique.

3. Protéine de la revendication 2 dans laquelle la réticulation comprend une liaison disulfure.

4. Protéine de la revendication 2 dans laquelle la réticulation est formée par réaction des protéines tétramériques avec un agent réticulant thiolspécifique multifonctionnel.

5. Protéine de la revendication 4 dans laquelle l'agent réticulant forme une réticulation qui est sensiblement moins réductible par les agents réducteurs endogènes au plasma que ne le serait une liaison disulfure entre les mêmes résidus.

6. Protéine de la revendication 4 ou 5 dans laquelle la réticulation est une réticulation thioéther ou thio-maléimide.

7. Protéine des revendications 4 ou 5 dans laquelle le réactif réticulant est un dérivé de polyéthylène glycol présentant des parties réactives thiolspécifiques.

8. Protéine de l'une quelconque des revendications 2-7 où la protéine est composée de deux protéines pseudotétramériques.

9. Protéine de l'une quelconque des revendications 4-7 dans laquelle la protéine est composée de plus de deux protéines pseudotétramériques.

10. Protéine de l'une quelconque des revendications 4-9 dans laquelle l'agent réticulant comprend une partie peptide.

11. Protéine de la revendication 10 dans laquelle l'agent réticulant comprend un ou plusieurs acides aminés D.

12. Protéine de la revendication 10 dans laquelle la partie peptide comprend une structure secondaire étendue et stable.

13. Protéine de la revendication 12 dans laquelle la partie peptide comprend une hélice de polyproline.

14. Protéine de la revendication 12 dans laquelle l'agent réticulant comprend un serpentin enroulé à deux brins.

15. Protéine de la revendication 14 dans laquelle les deux brins du serpentin enroulé sont réticulés par des ponts disulfures.

16. Protéine de la revendication 12 dans laquelle l'agent réticulant comprend un serpentin enroulé à 4 hélices ou à 4 brins.

17. Protéine de la revendication 12 dans laquelle la partie peptide comprend une chaîne ramifiée.

18. Protéine de l'une quelconque des revendications 4-17 dans laquelle la réticulation comprend une partie négativement chargée, ce qui réduit le point isoélectrique de la protéine.

19. Protéine de la revendication 18 dans laquelle la partie négativement chargée comprend plusieurs résidus Asp et/ou Glu.

20. Protéine de l'une quelconque des revendications 1-19 dans laquelle le résidu cystéine se situe dans un domaine de type alpha-globine qui est sensiblement homologue à une sous-unité alpha-globine d'une hémoglobine naturelle.

21. Protéine de l'une quelconque des revendications 1-19 dans laquelle le résidu cystéine se situe dans un domaine de type bêta-globine qui est sensiblement homologue à une sous-unité bêta-globine d'une hémoglobine naturelle.

22. Protéine de l'une quelconque des revendications 1-21, dans laquelle la protéine a subi une mutation, par rapport à l'hémoglobine humaine, pour inhiber la liaison de l'haptoglobine.

23. Protéine multimérique de l'une quelconque des revendications 2-22 dans laquelle ladite réticulation inhibe la liaison de l'haptoglobine.

24. Protéine de l'une quelconque des revendications 1-23 dans laquelle les domaines de type globine du polypeptide pseudodimérique sont réunis par une partie lieuse constituée essentiellement d'une ou plusieurs glycines.

25. Protéine de la revendication 24 dans laquelle les domaines sont des domaines de type alpha-globine qui sont sensiblement homologues à une sous-unité alpha-globine d'une hémoglobine naturelle et la partie lieuse est constituée de une à trois glycérines.

26. Protéine de la revendication 24 dans laquelle les domaines sont des domaines de type alpha-globine qui sont sensiblement homologues à une sous-unité bêta-globine d'une hémoglobine naturelle et la partie lieuse est constituée de deux à neuf glycérines.

27. Protéine de l'une quelconque des revendications 1-26, qui a une P₅₀ au moins 10% supérieure à celle de l'hémoglobine Ao dans les mêmes conditions.

28. Protéine de l'une quelconque des revendications 1-26, qui a une P₅₀ au moins 10% inférieure à celle de l'hémoglobine Ao dans les mêmes conditions.

29. Protéine de l'une quelconque des revendications 1-26, ladite protéine ayant une P₅₀ de 0,327-0,435 kg/m2 (24-32 torr).

30. Protéine de l'une quelconque des revendications 1-29, ladite protéine ayant une rétention intravasculaire sensiblement plus longue que l'hémoglobine normale libre dans le plasma.

31. Protéine de l'une quelconque des revendications 1-30 dans laquelle chacun des domaines de type globine est un domaine de type globine vertébrée qui est sensiblement homologue à une sous-unité de globine d'une hémoglobine vertébrée naturelle.

32. Protéine de l'une quelconque des revendications 1-30 dans laquelle chacun des domaines de type globine est un domaine de type globine de mammifère qui est sensiblement homologue à une sous-unité de globine d'une hémoglobine de mammifère naturelle.

33. Protéine de l'une quelconque des revendications 1-30 dans laquelle chacun des domaines de type globine est un domaine de type globine humaine qui est sensiblement homologue à une sous-unité de globine d'une hémoglobine humaine naturelle.

34. Utilisation de la protéine de l'une quelconque des revendications 1-33 dans la préparation d'une composition de substitut sanguin.

35. Protéine selon l'une quelconque des revendications 1-33 pour utilisation comme médicament.

36. Protéine selon l'une quelconque des revendications 1-33 pour utilisation comme substitut sanguin.

37. Composition de substitut sanguin comprenant la protéine de l'une quelconque des revendications 1-33.

38. Composition selon la revendication 37 qui est à au moins 50% monodispersée.

39. Protéine selon l'une quelconque des revendications 1-33 qui est sous une forme au moins partiellement purifiée.


### SEQUENCE LISTING

(1) GENERAL INFORMATION:
   (i) APPLICANT:
      ANDERSON, DAVID C.
      MATHEWS, ANTONY JAMES
      STETLER, GARY L.
   (ii) TITLE OF INVENTION: PRODUCTION AND USE OF MULTIMERIC HEMOGLOBINS
   (iii) NUMBER OF SEQUENCES: 28
   (iv) CORRESPONDENCE ADDRESS:
      (A) ADDRESSEE: Browdy and Neimark
      (B) STREET: 419 Seventh Street, N.W., Suite 300
      (C) CITY: Washington
      (D) STATE: D.C.
      (E) COUNTRY: USA
      (F) ZIP: 20004
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: EP PCT/US92/09752
      (B) FILING DATE: 06-NOV-1992
      (C) CLASSIFICATION:
   (viii) ATTORNEY/AGENT INFORMATION:
      (A) NAME: COOPER, IVER P
      (B) REGISTRATION NUMBER: 28,005
      (C) REFERENCE/DOCKET NUMBER: ANDERSON-6-PCT
   (ix) TELECOMMUNICATION INFORMATION:
      (A) TELEPHONE: 202-628-5197
      (B) TELEFAX: 202-737-3528
      (C) TELEX: 248633
(2) INFORMATION FOR SEQ ID NO:1:
   (i) SEQUENCE CHARACTERISTICS:
      (A) LENGTH: 1464 base pairs
      (B) TYPE: nucleic acid
      (C) STRANDEDNESS: single
      (D) TOPOLOGY: linear
(2) INFORMATION FOR SEQ ID NO:2:
   (i) SEQUENCE CHACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Ile Glu Gly Arg
   1
(2) INFORMATION FOR SEQ ID NO:3:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Val His Leu Thr Pro
   1               5
(2) INFORMATION FOR SEQ ID NO:4:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Ser Lys Tyr Arg
   1
(2) INFORMATION FOR SEQ ID NO:5:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
   Val Leu Ser Pro
   1
(2) INFORMATION FOR SEQ ID NO:6:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   His Lys Tyr His
   1
(2) INFORMATION FOR SEQ ID NO:7:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:7:
   Val His Leu Thr
   1
(2) INFORMATION FOR SEQ ID NO:8:
   (i) SEQUENCE CHARACTERISTICS:
      (A) LENGTH: 37 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:8:
   Lys Cys Ala Glu Leu Glu Gly Arg Leu Glu Ala Leu Glu Gly Arg Leu
   1               5                   10                  15
   Glu Ala Leu Glu Gly Arg Leu Glu Ala Leu Glu Gly Arg Leu Glu Ala
               20                  25                  30
   Leu Glu Gly Lys Leu
           35
(2) INFORMATION FOR SEQ ID NO:9:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Gly Glu Leu Glu Glu Leu Leu Lys Lys Leu Lys Glu Leu Leu Lys Gly
   1               5                   10                  15
(2) INFORMATION FOR SEQ ID NO:10:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:10:
   GAAGCCAUGG                                                          10
(2) INFORMATION FOR SEQ ID NO:11:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:11:
   Ile Glu Gly Arg
   1
(2) INFORMATION FOR SEQ ID NO:12:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:12:
   GCGGCCGCGG AAGAGTCAAT TCAGGAGGGT G 31
(2) INFORMATION FOR SEQ ID NO:13:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:13:
   GCCGGCCGTC ACTGCCCGCT TTCCAGTCGG GAA 33
(2) INFORMATION FOR SEQ ID NO:14:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:14:
   CCGAACGCGT TGTGCGCTCT GTCTGAT 27
(2) INFORMATION FOR SEQ ID NO:15:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:15:
   GGTGCTCACG TTGATTGCAT GCCGAACGCG 30
(2) INFORMATION FOR SEQ ID NO:16:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:16:
   CTGACCAACG CTGTTTGCCA CGTTGATGAT 30
(2) INFORMATION FOR SEQ ID NO:17:
   (i) SEQUENCE CHARACTERISTICS:
      (A) LENGTH: 141 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:17:
   Val Leu Ser Pro Ala Asp Lys Thr Asn Val Lys Ala Ala Trp Gly Lys
   1               5                   10                  15
   Val Gly Ala His Ala Gly Glu Tyr Gly Ala Glu Ala Leu Glu Arg Met
               20                  25                  30
   Phe Leu Ser Phe Pro Thr Thr Lys Thr Tyr Phe Pro His Phe Asp Leu
           35                  40                  45
   Ser His Gly Ser Ala Gln Val Lys Gly His Gly Lys Lys Val Ala Asp
       50                  55                  60
   Ala Leu Thr Asn Ala Val Ala His Val Asp Asp Met Pro Asn Ala Leu
   65                  70                  75                  80
   Ser Ala Leu Ser Asp Leu His Ala His Lys Leu Arg Val Asp Pro Val
                   85                  90                  95
   Asn Phe Lys Leu Leu Ser His Cys Leu Leu Val Thr Leu Ala Ala His
               100                 105                 110
   Leu Pro Ala Glu Phe Thr Pro Ala Val His Ala Ser Leu Asp Lys Phe
           115                 120                 125
   Leu Ala Ser Val Ser Thr Val Leu Thr Ser Lys Tyr Arg
       130                 135                 140
(2) INFORMATION FOR SEQ ID NO:18:
   (i) SEQUENCE CHARACTERISTICS:
      (A) LENGTH: 141 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Ser Leu Thr Lys Thr Glu Arg Thr Ile Ile Val Ser Met Trp Ala Lys
   1               5                   10                  15
   Ile Ser Thr Gln Ala Asp Thr Ile Gly Thr Glu Thr Leu Glu Arg Leu
               20                  25                  30
   Phe Leu Ser His Pro Gln Thr Lys Thr Tyr Phe Pro His Phe Asp Leu
           35                  40                  45
   His Pro Gly Ser Ala Gln Leu Arg Ala His Gly Ser Lys Val Val Ala
       50                  55                  60
   Ala Val Gly Asp Ala Val Lys Ser Ile Asp Asp Ile Gly Gly Ala Leu
   65                  70                  75                  80
   Ser Lys Leu Ser Glu Leu His Ala Tyr Ile Leu Arg Val Asp Pro Val
                   85                  90                  95
   Asn Phe Lys Leu Leu Ser His Cys Leu Leu Val Thr Leu Ala Ala Arg
               100                 105                 110
   Phe Pro Ala Asp Phe Thr Ala Glu Ala His Ala Ala Trp Asp Lys Phe
           115                 120                 125
   Leu Ser Val Val Ser Ser Val Leu Thr Glu Lys Tyr Arg
       130                 135                 140
(2) INFORMATION FOR SEQ ID NO:19:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:19:
   Val His Leu Thr Pro Glu Glu Lys Ser Ala Val Thr Ala Leu Trp Gly
   1               5                   10                  15
   Lys Val Asn Val Asp Glu Val Gly Gly Glu Ala Leu Gly Arg Leu Leu
               20                  25                  30
   Val Val Tyr Pro Trp Thr Gln Arg Phe Phe Glu Ser Phe Gly Asp Leu
           35                  40                  45
   Ser Thr Pro Asp Ala Val Met Gly Asn Pro Lys Val Lys Ala His Gly
       50                  55                  60
   Lys Lys Val Leu Gly Ala Phe Ser Asp Gly Leu Ala His Leu Asp Asn
   65                  70                  75                  80
   Leu Lys Gly Thr Phe Ala Thr Leu Ser Glu Leu His Cys Asp Lys Leu
                   85                  90                  95
   His Val Asp Pro Glu Asn Phe Arg Leu Leu Gly Asn Val Leu Val Cys
               100                 105                 110
   Val Leu Ala His His Phe Gly Lys Glu Phe Thr Pro Pro Val Gln Ala
           115                 120                 125
   Ala Tyr Gln Lys Val Val Ala Gly Val Ala Asn Ala Leu Ala His Lys
       130                 135                 140
   Tyr His
   145
(2) INFORMATION FOR SEQ ID NO:20:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:20:
   Val His Leu Thr Pro Glu Glu Lys Thr Ala Val Asn Ala Leu Trp Gly
   1 5 10 15
   Lys Val Asn Val Asp Ala Val Gly Gly Glu Ala Leu Gly Arg Leu Leu
   20 25 30
   Val Val Tyr Pro Trp Thr Gln Arg Phe Phe Glu Ser Phe Gly Asp Leu
   35 40 45
   Ser Ser Pro Asp Ala Val Met Gly Asn Pro Lys Val Lys Ala His Gly
   50 55 60
   Lys Lys Val Leu Gly Ala Phe Ser Asp Gly Leu Ala His Leu Asp Asn
   65 70 75 80
   Leu Lys Gly Thr Phe Ser Gln Leu Ser Glu Leu His Cys Asp Lys Leu
   85 90 95
   His Val Asp Pro Glu Asn Phe Arg Leu Leu Gly Asn Val Leu Val Cys
   100 105 110
   Val Leu Ala Arg Asn Phe Gly Lys Glu Phe Thr Pro Gln Met Gln Ala
   115 120 125
   Ala Tyr Gln Lys Val Val Ala Gly Val Ala Asn Ala Leu Ala His Lys
   130 135 140
   Tyr His
   145
(2) INFORMATION FOR SEQ ID NO:21:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:21:
   Gly His Phe Thr Glu Glu Asp Lys Ala Thr Ile Thr Ser Leu Trp Gly
   1 5 10 15
   Lys Val Asn Val Glu Asp Ala Gly Gly Glu Thr Leu Gly Arg Leu Leu
   20 25 30
   Val Val Tyr Pro Trp Thr Gln Arg Phe Phe Asp Ser Phe Gly Asn Leu
   35 40 45
   Ser Ser Ala Ser Ala Ile Met Gly Asn Pro Lys Val Lys Ala His Gly
   50 55 60
   Lys Lys Val Leu Thr Ser Leu Gly Asp Ala Ile Lys His Leu Asp Asp
   65 70 75 80
   Leu Lys Gly Thr Phe Ala Gln Leu Ser Glu Leu His Cys Asp Lys Leu
   85 90 95
   His Val Asp Pro Glu Asn Phe Lys Leu Leu Gly Asn Val Leu Val Thr
   100 105 110
   Val Leu Ala Ile His Phe Gly Lys Glu Phe Thr Pro Glu Val Gln Ala
   115 120 125
   Ser Trp Gln Lys Met Val Thr Ala Val Ala Ser Ala Leu Ser Ser Arg
   130 135 140
   Tyr His
   145
(2) INFORMATION FOR SEQ ID NO:22:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:22:
   Gly His Phe Thr Glu Glu Asp Lys Ala Thr Ile Thr Ser Leu Trp Gly
   1               5                   10                  15
   Lys Val Asn Val Glu Asp Ala Gly Gly Glu Thr Leu Gly Arg Leu Leu
               20                  25                  30
   Val Val Tyr Pro Trp Thr Gln Arg Phe Phe Asp Ser Phe Gly Asn Leu
           35                  40                  45
   Ser Ser Ala Ser Ala Ile Met Gly Asn Pro Lys Val Lys Ala His Gly
       50                  55                  60
   Lys Lys Val Leu Thr Ser Leu Gly Asp Ala Ile Lys His Leu Asp Asp
   65                  70                  75                  80
   Leu Lys Gly Thr Phe Ala Gln Leu Ser Glu Leu His Cys Asp Lys Leu
                   85                  90                  95
   His Val Asp Pro Glu Asn Phe Lys Leu Leu Gly Asn Val Leu Val Thr
               100                 105                 110
   Val Leu Ala Ile His Phe Gly Lys Glu Phe Thr Pro Glu Val Gln Ala
           115                 120                 125
   Ser Trp Gln Lys Met Val Thr Gly Val Ala Ser Ala Leu Ser Ser Arg
       130                 135                 140
   Tyr His
   145
(2) INFORMATION FOR SEQ ID NO:23:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:23:
   Gly His Phe Thr Glu Glu Asp Lys Ala Thr Ile Thr Ser Leu Trp Gly
   1               5                   10                  15
   Lys Val Asn Val Glu Asp Ala Gly Gly Glu Thr Leu Gly Arg Leu Leu
               20                  25                  30
   Val Val Tyr Pro Trp Thr Gln Arg Phe Phe Asp Ser Phe Gly Asn Leu
           35                  40                  45
   Ser Ser Ala Ser Ala Ile Met Gly Asn Pro Lys Val Lys Ala His Gly
       50                  55                  60
   Lys Lys Val Leu Thr Ser Leu Gly Asp Ala Thr Lys His Leu Asp Asp
   65                  70                  75                      80
   Leu Lys Gly Thr Phe Ala Gln Leu Ser Glu Leu His Cys Asp Lys Leu
                   85                  90                  95
   His Val Asp Pro Glu Asn Phe Lys Leu Leu Gly Asn Val Leu Val Thr
               100                 105                 110
   Val Leu Ala Ile His Phe Gly Lys Glu Phe Thr Pro Glu Val Gln Ala
           115                 120                 125
   Ser Trp Gln Lys Met Val Thr Ala Val Ala Ser Ala Leu Ser Ser Arg
       130                 135                 140
   Tyr His
   145
(2) INFORMATION FOR SEQ ID NO:24:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:24:
   Val His Phe Thr Ala Glu Glu Lys Ala Ala Val Thr Ser Leu Trp Ser
   1 5 10 15
   Lys Met Asn Val Glu Glu Ala Gly Gly Glu Ala Leu Gly Arg Leu Leu
   20 25 30
   Val Val Tyr Pro Trp Thr Gln Arg Phe Phe Asp Ser Phe Gly Asn Leu
   35 40 45
   Ser Ser Pro Ser Ala Ile Leu Gly Asn Pro Lys Val Lys Ala His Gly
   50 55 60
   Lys Lys Val Leu Thr Ser Phe Gly Asp Ala Ile Lys Asn Met Asp Asn
   65 70 75 80
   Leu Lys Pro Ala Phe Ala Lys Leu Ser Glu Leu His Cys Asp Lys Leu
   85 90 95
   His Val Asp Pro Glu Asn Phe Lys Leu Leu Gly Asn Val Met Val Ile
   100 105 110
   Ile Leu Ala Thr His Phe Gly Lys Glu Phe Thr Pro Glu Val Gln Ala
   115 120 125
   Ala Trp Gln Lys Leu Val Ser Ala Val Ala Ile Ala Leu Ala His Lys
   130 135 140
   Tyr His
   145
(2) INFORMATION FOR SEQ ID NO:25:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:25:
   Met Tyr Arg Leu Asn Lys Glu Glu
   1 5
(2) INFORMATION FOR SEQ ID NO:26:
   (i) SEQUENCE CHARACTERISTICS:
      (A) LENGTH: 141 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:26:
   Met Leu Ser Pro Ala Asp Lys Thr Asn Val Lys Ala Ala Trp Gly Lys
   1 5 10 15
   Val Gly Ala His Ala Gly Glu Tyr Gly Ala Glu Ala Leu Glu Arg Met
   20 25 30
   Phe Leu Ser Phe Pro Thr Thr Lys Thr Tyr Phe Pro His Phe Asp Leu
   35 40 45
   Ser His Gly Ser Ala Gln Val Lys Gly His Gly Lys Lys Val Ala Asp
   50 55 60
   Ala Leu Thr Asn Ala Val Ala His Val Asp Asp Met Pro Asn Ala Leu
   65 70 75 80
   Ser Ala Leu Ser Asp Leu His Ala His Lys Leu Arg Val Asp Pro Val
   85 90 95
   Asn Phe Lys Leu Leu Ser His Cys Leu Leu Val Thr Leu Ala Ala His
   100 105 110
   Leu Pro Ala Glu Phe Thr Pro Ala Val His Ala Ser Leu Asp Lys Phe
   115 120 125
   Leu Ala Ser Val Ser Thr Val Leu Thr Ser Lys Tyr Arg
   130 135 140
(2) INFORMATION FOR SEQ ID NO:27:
   (i) SEQUENCE CHARACTERISTICS:
      (A) LENGTH: 141 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:27:
   Val Leu Ser Pro Ala Asp Lys Thr Asn Val Lys Ala Ala Trp Gly Lys
   1 5 10 15
   Val Gly Ala His Ala Gly Glu Tyr Gly Ala Glu Ala Leu Glu Arg Met
   20 25 30
   Phe Leu Ser Phe Pro Thr Thr Lys Thr Tyr Phe Pro His Phe Asp Leu
   35 40 45
   Ser His Gly Ser Ala Gln Val Lys Gly His Gly Lys Lys Val Ala Asp
   50 55 60
   Ala Leu Thr Asn Ala Val Ala His Val Asp Asp Met Pro Asn Ala Leu
   65 70 75 80
   Ser Ala Leu Ser Asp Leu His Ala His Lys Leu Arg Val Asp Pro Val
   85 90 95
   Asn Phe Lys Leu Leu Ser His Cys Leu Leu Val Thr Leu Ala Ala His
   100 105 110
   Leu Pro Ala Glu Phe Thr Pro Ala Val His Ala Ser Leu Asp Lys Phe
   115 120 125
   Leu Ala Ser Val Ser Thr Val Leu Thr Ser Lys Tyr Arg
   130 135 140
(2) INFORMATION FOR SEQ ID NO:28:
   (i) SEQUENCE CHARACTERISTICS:
      (A) LENGTH: 146 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:28:
   Met His Leu Thr Pro Glu Glu Lys Ser Ala Val Thr Ala Leu Trp Gly
   1 5 10 15
   Lys Val Asn Val Asp Glu Val Gly Gly Glu Ala Leu Gly Arg Leu Leu
   20 25 30
   Val Val Tyr Pro Trp Thr Gln Arg Phe Phe Glu Ser Phe Gly Asp Leu
   35 40 45
   Ser Thr Pro Asp Ala Val Met Gly Asn Pro Lys Val Lys Ala His Gly
   50 55 60
   Lys Lys Val Leu Gly Ala Phe Ser Asp Gly Leu Ala His Leu Asp Asn
   65 70 75 80
   Leu Lys Gly Thr Phe Ala Thr Leu Ser Glu Leu His Cys Asp Lys Leu
   85 90 95
   His Val Asp Pro Glu Asn Phe Arg Leu Leu Gly Lys Val Leu Val Cys
   100 105 110
   Val Leu Ala His His Phe Gly Lys Glu Phe Thr Pro Pro Val Gln Ala
   115 120 125
   Ala Tyr Gln Lys Val Val Ala Gly Val Ala Asn Ala Leu Ala His Lys
   130 135 140
   Tyr His
   145